(19) Europäisches Patentamt
European Patent Office
Office européen des brevets

(11) **EP 3 310 927 B1**

(12) **EUROPEAN PATENT SPECIFICATION**

(45) Date of publication and mention
of the grant of the patent:
**19.05.2021 Bulletin 2021/20**

(21) Application number: **16731299.0**

(22) Date of filing: **17.06.2016**

(51) Int Cl.:
**C12Q 1/6886** *(2018.01)*

(86) International application number:
**PCT/GB2016/051825**

(87) International publication number:
**WO 2016/203262 (22.12.2016 Gazette 2016/51)**

(54) **GENE SIGNATURES PREDICTIVE OF METASTATIC DISEASE**

FÜR METASTASIERUNG PRÄDIKTIVE GENSIGNATUREN

SIGNATURES GÉNIQUES PRÉDICTIVES D'UNE MALADIE MÉTASTATIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.06.2015 GB 201510684**

(43) Date of publication of application:
**25.04.2018 Bulletin 2018/17**

(73) Proprietor: **Almac Diagnostic Services Limited
Craigavon BT63 5QD (GB)**

(72) Inventors:
• **WALKER, Steven**
Craigavon BT63 5QD (GB)
• **HILL, Laura**
Craigavon BT63 5QD (GB)
• **MCCAVIGAN, Andrena**
Craigavon BT63 5QD (GB)
• **DONEGAN, Sinead**
Craigavon BT63 5QD (GB)
• **DAVISON, Timothy**
Craigavon BT63 5QD (GB)
• **KENNEDY, Richard**
Craigavon BT63 5QD (GB)
• **HARKIN, Denis Paul**
Craigavon BT63 5QD (GB)
• **PRICE, Bethanie**
Craigavon BT63 5QD (GB)

(74) Representative: **Boult Wade Tennant LLP
Salisbury Square House
8 Salisbury Square
London EC4Y 8AP (GB)**

(56) References cited:
EP-A2- 1 471 154        WO-A1-2011/068839
WO-A1-2014/012176       WO-A2-02/30268
WO-A2-2004/085621       US-A1- 2014 018 249

• RAHUL V. GOPALKRISHNAN ET AL: "Molecular markers and determinants of prostate cancer metastasis", JOURNAL OF CELLULAR PHYSIOLOGY, vol. 189, no. 3, 1 December 2001 (2001-12-01), pages 245-256, XP055297597, US ISSN: 0021-9541, DOI: 10.1002/jcp.10023

• AMRALLAH A. MOHAMMED: "Biomarkers in prostate cancer: new era and prospective", MEDICAL ONCOLOGY, vol. 31, no. 8, 1 August 2014 (2014-08-01) , XP055297604, GB ISSN: 1357-0560, DOI: 10.1007/s12032-014-0140-3

• GORLOV IVAN P ET AL: "Candidate pathways and genes for prostate cancer: a meta-analysis of gene expression data", BMC MEDICAL GENOMICS, BIOMED CENTRAL LTD, LONDON UK, vol. 2, no. 1, 4 August 2009 (2009-08-04), page 48, XP021060675, ISSN: 1755-8794, DOI: 10.1186/1755-8794-2-48

• MCCART REED AMY E ET AL: "Thrombospondin-4 expression is activated during the stromal response to invasive breast cancer", VIRCHOWS ARCHIV, SPRINGER INTERNATIONAL, BERLIN, DE, vol. 463, no. 4, 14 August 2013 (2013-08-14), pages 535-545, XP035373143, ISSN: 0945-6317, DOI: 10.1007/S00428-013-1468-3 [retrieved on 2013-08-14]

**(Cont. next page)**

Remarks:
    The complete document including Reference Tables and the Sequence Listing can be downloaded from the EPO website

**Description**

**FIELD OF THE INVENTION**

The present invention relates to cancer and in particular to prostate cancer and ER positive breast cancer. Provided are methods for characterising and prognosing cancer and in particular prostate cancer and ER positive breast cancer. The methods utilize various biomarkers, specifically in the form of one or more gene signatures. Primers, probes, antibodies, kits, devices and systems useful in the methods are also described.

**BACKGROUND OF THE INVENTION**

Prostate cancer is the most common malignancy in men with a lifetime incidence of 15.3% (Howlader 2012). Based upon data from 1999-2006 approximately 80% of prostate cancer patients present with early disease clinically confined to the prostate (Altekruse et al 2010) of which around 65% are cured by surgical resection or radiotherapy (Kattan et al 1999, Pound et al 1999). 35% will develop PSA recurrence of which approximately 35% will develop local or metastatic recurrence, which is non-curable. At present it is unclear which patients with early prostate cancer are likely to develop recurrence and may benefit from more intensive therapies. Current prognostic factors such as tumour grade as measured by Gleason score have prognostic value but a significant number of those considered lower grade (7 or less) still recur and a proportion of higher-grade tumours do not. Additionally there is significant heterogeneity in the prognosis of Gleason 7 tumours (Makarov et al 2002, Rasiah et al 2003). Furthermore it has become evident that the grading of Gleason score has changed leading to changes in the distribution of Gleason scores over time (Albertsen et al 2005, Smith et al 2002).

It is now clear that most solid tumours originating from the same anatomical site represent a number of distinct entities at a molecular level (Perou et al 2000). DNA microarray platforms allow the analysis of tens of thousands of transcripts simultaneously from archived paraffin embedded tissues and are ideally suited for the identification of molecular subgroups. This kind of approach has identified primary cancers with metastatic potential in solid tumours such as breast (van 't Veer et al 2002) and colon cancer (Bertucci et al 2004). Gorlov et al. (BMC Med Genomics. 2009 Aug 4;2:48) disclose candidate pathways and genes for prostate cancer.

**DESCRIPTION OF THE INVENTION**

The invention is defined in the appended claims. The present invention is based upon the identification and verification of cancer biomarkers, particularly prognostic biomarkers that identify potentially metastatic cancers (such as prostate and ER positive breast cancers).

The present inventors have identified a group of primary prostate cancers that are similar to metastatic disease at a molecular level. Primary tumour samples which clustered with metastatic samples define a group with poor (bad) prognosis. These tumours may be defined by down regulation of genes associated with cell adhesion, cell differentiation and cell development. These tumours may be defined by up regulation of androgen related processes and epithelial to mesenchymal transition (EMT). In contrast, benign and primary like benign tumours cluster to define a group with improved (good) prognosis. A series of biomarker/gene signatures that can be used to prospectively identify tumours within either subgroup (i.e. with metastatic or non-metastatic biology) have been generated and validated which have prognostic power. The signatures can thus be used to prospectively assess a tumour's progression, for example to determine whether a tumour is at increased likelihood of recurrence and/or metastatic development. The signatures also display excellent performance in heterogeneity studies as discussed further herein. In particular, a 70 gene signature is described herein. The gene signatures are also shown to be effective in other cancer types including ER positive breast cancer, thus suggesting that the underlying molecular biology may have applicability in defining potentially metastatic primary tumours.

Thus, in a first aspect the invention provides a method for characterising and/or prognosing cancer, such as prostate cancer or ER positive breast cancer, in a subject with a primary cancer but no known metastases comprising: determining the expression level of THBS4 and at least 11 genes selected from Table 1 in a sample comprising nucleic acids from the primary tumour from the subject wherein the determined expression level is used to provide a characterisation of and/or a prognosis for the cancer, wherein the characterisation of and/or prognosis for the cancer consists of predicting an increased likelihood of recurrence and/or predicting an increased likelihood of metastasis and/or a poor prognosis.

Described herein is a method for diagnosing (or identifying or characterizing) a cancer, such as prostate cancer or ER positive breast cancer, with an increased metastatic potential in a subject comprising: determining the expression level of at least one gene from Table 1 in a sample from the subject wherein the determined expression level is used to identify whether a subject has a cancer, such as prostate cancer or ER positive breast cancer, with increased metastatic potential.

Described herein is a method for characterising and/or prognosing a cancer, such as prostate cancer or ER positive breast cancer in a subject comprising:

determining the expression level of at least one gene from Table 1 in a sample from the subject in order to identify the presence or absence of cells characteristic of an increased likelihood of recurrence and/or metastasis wherein the determined presence or absence of the cells is used to provide a characterisation of and/or a prognosis for the cancer, such as prostate cancer or ER positive breast cancer.

Described herein is a method for characterising and/or prognosing a cancer, such as prostate cancer or ER positive breast cancer in a subject comprising:

a) obtaining a sample from the subject/ in a sample obtained from the subject
b) applying a nucleic acid probe that specifically hybridizes with the nucleotide sequence of at least one gene or full sequence or target sequence selected from Table 1 to the sample from the subject
c) applying a detection agent that detects the nucleic acid probe-gene complex
d) using the detection agent to determine the level of the at least one gene or full sequence or target sequence
d) wherein the determined level of the at least one gene (or full sequence or target sequence) is used to provide a characterisation of and/or a prognosis for the cancer, such as prostate cancer or ER positive breast cancer. Suitable probes and probesets are listed in Table 1 and further details are provided in Table 1A.

Described herein is a method for characterising and/or prognosing a cancer, such as prostate cancer or ER positive breast cancer in a subject comprising:

a) obtaining a sample from the subject/ in a sample obtained from the subject
b) applying a set of nucleic acid primers that specifically hybridize with the nucleotide sequence of at least one gene or full sequence or target sequence selected from Table 1 to the sample from the subject
c) specifically amplifying the nucleotide sequence using the set of nucleic acid primers
d) detecting the amplification products using a specific detection agent to determine the level of the at least one gene or full sequence or target sequence
e) wherein the determined level of the at least one gene (or full sequence or target sequence) is used to provide a characterisation of and/or a prognosis for the cancer, such as prostate cancer or ER positive breast cancer. Suitable primers and primer pairs are listed in Table 1B.

The detection agent may comprise a label, such as a fluorescence label or fluorophore/quencher system attached to the nucleic acid probe and/or primer (as appropriate). Suitable systems and methodologies are known in the art and described herein.

The characterization, prognosis or diagnosis of the cancer, such as prostate cancer or ER positive breast cancer can also be used to guide treatment.

Accordingly, in a further aspect, the present invention relates to a method for selecting a treatment for cancer, such as prostate cancer or ER positive breast cancer in a subject with a primary cancer but no known metastases comprising:

(a) determining the expression level of THBS4 and at least 11 genes selected from Table 1 in a sample comprising nucleic acids from the primary tumour from the subject wherein the determined expression level is used to provide a characterisation of and/or a prognosis for the cancer, such as prostate cancer or ER positive breast cancer and
(b) selecting a treatment appropriate to the characterisation of and/or prognosis for the cancer, such as prostate cancer or ER positive breast cancer,

wherein the characterisation of and/or prognosis for the cancer consists of predicting an increased likelihood of recurrence and/or predicting an increased likelihood of metastasis and/or a poor prognosis.

Described herein is a method for selecting a treatment for a cancer, such as prostate cancer or ER positive breast cancer in a subject comprising:

(a) determining the expression level of at least one gene selected from Table 1 in a sample from the subject wherein the determined expression level is used to provide a characterisation of and/or a prognosis for the cancer, such as prostate cancer or ER positive breast cancer
(b) selecting a treatment appropriate to the characterisation of and/or prognosis for the cancer, such as prostate cancer or ER positive breast cancer and
(c) treating the subject with the selected treatment.

Described herein is a method of treating cancer, such as prostate cancer or ER positive breast cancer comprising administering a chemotherapeutic agent or radiotherapy, optionally extended radiotherapy, preferably extended-field radiotherapy, to a subject or carrying out surgery on a subject wherein the subject is selected for treatment on the basis of a method as described herein.

In a further aspect, the present invention relates to a chemotherapeutic agent for use in treating a cancer, such as prostate cancer or ER positive breast cancer in a subject, wherein the subject is selected for treatment on the basis of a method as described herein.

Described herein is a method of treating a cancer, such as prostate cancer or ER positive breast cancer comprising administering a chemotherapeutic agent or radiotherapy, optionally extended radiotherapy, preferably extended-field radiotherapy to a subject or carrying out surgery on a subject wherein the subject has an increased expression level of at least one gene with a positive weight selected from Table 1 and/or wherein the subject has a decreased expression level of at least one gene with negative weight selected from Table 1.

The invention also relates to a chemotherapeutic agent for use in treating a cancer, such as prostate cancer or ER positive breast cancer in a subject, wherein the subject is selected for treatment on the basis of a method as described herein and wherein the subject has an increased expression level of at least one gene with a positive weight selected from Table 1 and/or wherein the subject has a decreased expression level of at least one gene with a negative weight selected from Table 1.

In certain embodiments according to all relevant aspects of the invention the chemotherapeutic agent comprises, consists essentially of or consists of

a) an anti-hormone treatment, preferably bicalutamide and/or abiraterone
b) a cytotoxic agent
c) a biologic, preferably an antibody and/or a vaccine, more preferably Sipuleucel-T and/or
d) a targeted therapeutic agent

Suitable therapies and therapeutic agents are discussed in further detail herein. The treatment may comprise or be adjuvant therapy in some embodiments.

According to all aspects of the invention the cancer may be a prostate cancer or ER positive breast cancer. Typically, the cancer is a primary tumor. In some embodiments, the prostate cancer may be a primary prostate cancer.

It is shown herein that the gene signatures may have particularly advantageous utility when combined with determination of other prognostic factors. Thus, all aspects of the invention may include other prognostic factors in the characterization, diagnosis or prognosis of the cancer. This may comprise generation of a combined risk score. This is particularly applicable in the context of prostate cancer. Other prognostic factors include prostate specific antigen (PSA) levels and/or Gleason score. MRI scan results may also be taken into account. Thus, according to all aspects of the invention, characterization, prognosis or diagnosis may take into account other prognostic factors such as PSA levels and/or Gleason score. PSA is a well-known serum biomarker and may be used according to the invention, in particular when measured pre-operatively. For example, a PSA value of 4-10 ng/ml may be considered "low risk". A PSA value of 10-20 ng/ml may be considered reflective of "medium risk". A PSA value of 20 ng/ml or more may be considered reflective of "high risk". High risk would correspond to poor prognosis and/or be indicative of aggressive disease. Levels of PSA may contribute towards a final characterization of the cancer in combination with the measured expression levels. Medium risk PSA levels when combined with a positive or high signature score may indicate poor prognosis.

The Gleason system is used to grade prostate tumours with a score from 2 to 10, where a Gleason score of 10 indicates the most abnormalities. Cancers with a higher Gleason score are more aggressive and have a worse prognosis. The system is based on how the prostate cancer tissue appears under a microscope and indicates how likely it is that a tumour will spread. A low Gleason score means the cancer tissue is similar to normal prostate tissue and the tumour is less likely to spread; a high Gleason score means the cancer tissue is very different from normal and the tumour is more likely to spread. Gleason scores are calculated by adding the score of the most common grade (primary grade pattern) and the second most common grade (secondary grade pattern) of the cancer cells. Where more than two grades are observed the primary grade is added to the worst observable grade to arrive at the Gleason score. Grades are assigned using the 2005 (amended in 2009) International Society of Urological Pathology (ISUP) Consensus Conference on Gleason Grading of Prostatic Carcinoma. Thus, in some embodiments, a Gleason score of 7 or more contributes to a characterization of poor prognosis. In such embodiments, a Gleason score of less than 7 may contribute to a characterization of good prognosis. In some embodiments, a Gleason score of 7 thus contributes less to a characterization of poor prognosis than does a Gleason score of 8 or more, but more than a Gleason score of 6 or less. A Gleason score of 7 when combined with a positive or high signature score may indicate poor prognosis.

Where both Gleason score and PSA levels contribute to the characterization of the cancer, they may be weighted relative to one another. Typically, Gleason score is given greater significance than PSA levels. Thus, for example a Gleason score indicative of poor prognosis in combination with PSA levels associated with low risk, or good prognosis, may still result in a conclusion of poor prognosis (depending upon the measured expression levels of the gene or genes from Table 1). Similar considerations may apply to MRI results, which may be given greater weight than PSA levels in making the final characterization of the cancer.

The genes which may be included in suitable gene signatures and their identifying information are described and defined in further detail in Table 1 below. The genes may also be referred to, interchangeably, as biomarkers. Full sequences, against which suitable expression level determination assays may be designed, are also indicated in the table. Similarly, target sequences, against which suitable expression level determination assays may be designed, are also indicated in the table. Probe sequences interrogating the target sequences are also provided. Each sequence type is useful in the performance of the invention.

Table 1

| Signature Weight | Signature Bias | Weight (absolute) | Rank by weight | Gene symbol | Probesets | SEQ ID NO of sequence | | |
|---|---|---|---|---|---|---|---|---|
| | | | | | | Full | Target | Probe |
| -0.01089888 | 4.440873234 | 0.01089888 | 1 | CAPN6 | 3Snip.7769-1124a at | 15 | 247 | 619-629 |
| -0.009631509 | 6.912586369 | 0.009631509 | 2 | THBS4 | PC3P.12363. C1_s_at | 28 | 260 | 750-760 |
| -0.008885735 | 4.383572327 | 0.008885735 | 3 | PLP1 | PC3P.17142. C1_s_at | 64 | 296 | 1143-1153 |
| | | | | | PCADA.1273 8_s_at | 168 | 400 | 2298-2308 |
| -0.008680747 | 6.747956978 | 0.008680747 | 4 | MT1A | 'PCRS3.3951 _at' | 231 | 463 | 2994-3003 |
| | | | | | 'PCRS3.3951 _x_at' | 232 | 464 | 3004-3014 |
| -0.008278545 | 7.215245389 | 0.008278545 | 5 | MIR205HG | 'PC3P.1643.C 1_s_at' | 54 | 286 | 1033-1043 |
| | | | | | 'PC3P.1643.C 4-370a_s_at' | 55 | 287 | 1044-1054 |
| | | | | | 'PC3P.1643.C 6-335a_s_at' | 56 | 288 | 1055-1065 |
| | | | | | 'PCRS2.3147 _x_at' | 227 | 459 | 2952-2962 |
| -0.007934619 | 4.230422622 | 0.007934619 | 6 | SEMG1 | '3Snip.972-5a_s_at' | 16 | 248 | 630-640 |
| -0.007295796 | 4.293172794 | 0.007295796 | 7 | RSPO3 | '3Snip.465-263a_s_at' | 8 | 240 | 552 |
| | | | | | 'PCRS2.4412 _s_at' | 228 | 460 | 2963-2971 |
| -0.007164357 | 6.522547774 | 0.007164357 | 8 | ANO7 | 'PC3P.1358.C 1_at' | 37 | 269 | 849-859 |
| | | | | | 'PC3P.1358.C 1-1172a_s_at' | 38 | 270 | 860-870 |
| | | | | | 'PC3SNG.174 2-20a_s_at' | 125 | 357 | 1825-1835 |
| | | | | | 'PCHP.560_s_ at' | 205 | 437 | 2715-2724 |
| | | | | | 'PCHP.564_s_ at' | 206 | 438 | 2725-2735 |
| -0.007138975 | 7.621758138 | 0.007138975 | 9 | PCP4 | 'PC3P.11557. C1_s_at' | 23 | 255 | 696-706 |
| -0.006922498 | 5.92831485 | 0.006922498 | 10 | ANKRD 1 | 'PC3SNG.154 9-27a_s_at' | 124 | 356 | 1814-1824 |

| Signature Weight | Signature Bias | Weight (absolute) | Rank by weight | Gene symbol | Probesets | SEQ ID NO of sequence | | |
|---|---|---|---|---|---|---|---|---|
| | | | | | | Full | Target | Probe |
| -0.006844539 | 4.57431880 7 | 0.00684453 9 | 11 | MYBPC 1 | 'PC3P.13654. C1_at' | 39 | 271 | 871-881 |
| | | | | | 'PC3P.13654. C1_x_at' | 40 | 272 | 882-892 |
| | | | | | 'PC3P.3003.C 1_s_at | 74 | 306 | 1253-1263 |
| | | | | | 'PC3P.3003.C 1_x_at' | 75 | 307 | 1264-1274 |
| | | | | | 'PC3P.7685.C 1_at' | 101 | 333 | 1550-1560 |
| | | | | | 'PC3P.7685.C 1_x_at' | 102 | 334 | 1561-1571 |
| | | | | | 'PC3P.7685.C 1-693a_s_at' | 103 | 335 | 1572-1582 |
| | | | | | 'PC3SNGnh.2 74_x_at' | 144 | 376 | 2034-2044 |
| -0.00683545 | 6.75672206 3 | 0.00683545 | 12 | MMP7 | 'PC3P.2763.C 1_s_at' | 71 | 303 | 1220-1230 |
| -0.006830879 | 5.74546175 2 | 0.00683087 9 | 13 | SERPIN A3 | 'PC3P.104.C B1_s_at' | 19 | 251 | 663-673 |
| -0.006809804 | 5.97768214 3 | 0.00680980 4 | 14 | SELE | 'PCHP.1458_ s_at' | 199 | 431 | 2639-2649 |
| -0.006402712 | 6.08049398 3 | 0.00640271 2 | 15 | KRT5 | 'PC3P.10239. C1_s_at' | 17 | 249 | 641-651 |
| -0.006400452 | 6.49725999 1 | 0.00640045 2 | 16 | LTF | 'PC3P.167.C1 _s_at' | 61 | 293 | 1110-1120 |
| | | | | | 'PC3P.9581.C 1_x_at' | 118 | 350 | 1737-1747 |
| | | | | | 'PC3SNG.146 7-30a_s_at' | 123 | 355 | 1803-1813 |
| -0.006380629 | 3.55996601 | 0.00638062 9 | 17 | KIAA12 10 | 'PC3P.12920. C1_x_at' | 34 | 266 | 816-826 |
| -0.006312212 | 8.06342124 9 | 0.00631221 2 | 18 | TMEM1 58 | 'PCADA.9364 _s_at' | 177 | 409 | 2397-2407 |
| -0.006271047 | 9.96082669 | 0.00627104 7 | 19 | ZFP36 | 'PCHP.1147_ s_at' | 196 | 428 | 2606-2407 |
| -0.006108115 | 6.95493601 5 | 0.00610811 5 | 20 | FOSB | 'PC3P.1906.C 1_s_at' | 65 | 297 | 1154-1164 |
| | | | | | 'PC3P.1906.C | 66 | 298 | 1165-1175 |
| | | | | | 1-568a_s_at' | | | |
| | | | | | 'PCEM.1525_ s_at' | 192 | 424 | 2562-2572 |
| | | | | | 'PCPD.3244. C1_s_at' | 217 | 449 | 2845-2853 |

(continued)

| Signature Weight | Signature Bias | Weight (absolute) | Rank by weight | Gene symbol | Probesets | SEQ ID NO of sequence | | |
|---|---|---|---|---|---|---|---|---|
| | | | | | | Full | Target | Probe |
| -0.006101922 | 5.262341585 | 0.006101922 2 | 21 | PCA3 | '3Snip.6683-12a_x_at' | 12 | 244 | 586-596 |
| | | | | | 'PC3P.11294. C1_s_at' | 22 | 254 | 685-695 |
| | | | | | 'PC3P.13143. C1_at' | 35 | 267 | 827-837 |
| | | | | | 'PC3P.13143. C1_x_at' | 36 | 268 | 838-848 |
| | | | | | 'PC3P.2274.C 1_s_at' | 67 | 299 | 1176-1186 |
| | | | | | 'PC3P.5053.C 1_s_at' | 88 | 320 | 1407-1417 |
| | | | | | 'PC3P.5053.C 1-490a_s_at' | 89 | 321 | 1418-1428 |
| | | | | | 'PC3SNGnh.9 32_x_at' | 163 | 395 | 2243-2253 |
| -0.006059944 | 4.865791397 | 0.006059944 | 22 | TRPM8 | 'PC3P.12013. C1_s_at' | 24 | 256 | 707-717 |
| | | | | | 'PC3P.12591. C1_x_at' | 29 | 261 | 761-771 |
| | | | | | 'PC3P.1261.C 1_s_at' | 30 | 262 | 772-782 |
| | | | | | 'PC3P.1507.C 1_at' | 45 | 277 | 934-944 |
| | | | | | 'PC3P.1507.C 1_x_at' | 46 | 278 | 945-955 |
| | | | | | 'PC3P.3670.C 1_s_at' | 78 | 310 | 1297-1307 |
| | | | | | 'PC3P.3670.C 1-625a_s_at' | 79 | 311 | 1308-1318 |
| | | | | | 'PC3P.3670.C 2_s_at' | 80 | 312 | 1319-1329 |
| | | | | | 'PC3SNGnh.1 467_at' | 137 | 369 | 1957-1967 |
| | | | | | 'PC3SNGnh.1 467_x_at' | 138 | 370 | 1968-1978 |
| | | | | | 'PC3SNGnh.2 659_at' | 143 | 375 | 2023-2033 |
| | | | | | 'PC3SNGnh.3 350_at' | 145 | 377 | 2045-2055 |
| | | | | | 'PC3SNGnh.3 350_x_at' | 146 | 378 | 2056-2066 |
| | | | | | 'PC3SNGnh.5 454_at' | 159 | 391 | 2199-2209 |
| 0.0060017344 | 4.712692803 | 0.0060017344 | 23 | PTTG1 | 'PC3P.16730. C1_x_at' | 62 | 294 | 1121-1131 |
| | | | | | 'PCHP.233_x_at' | 201 | 433 | 2661-2671 |

| Signature Weight | Signature Bias | Weight (absolute) | Rank by weight | Gene symbol | Probesets | SEQ ID NO of sequence | | |
|---|---|---|---|---|---|---|---|---|
| | | | | | | Full | Target | Probe |
| -0.005950381 | 4.98038094 1 | 0.00595038 1 | 24 | N/A | 'PC3P.12756. C1_x_at' | 32 | 264 | 794-804 |
| | | | | | 'PC3P.5784.C 1_at' | 96 | 328 | 1495-1505 |
| | | | | | 'PC3P.5784.C 1_x_at' | 97 | 329 | 1506-1516 |
| | | | | | 'PC3P.8725.C 1_at' | 112 | 344 | 1671-1681 |
| | | | | | 'PC3P.8725.C 1_x_at' | 113 | 345 | 1682-1692 |
| | | | | | 'PC3P.8968.C 1_s_at' | 114 | 346 | 1693-1703 |
| | | | | | PC3P.9903.C 1_at' | 120 | 352 | 1759-1769 |
| | | | | | 'PC3P.9903.C 1_x_at' | 121 | 353 | 1770-1780 |
| | | | | | 'PC3SNG.638 7-29a_x_at' | 132 | 364 | 1902-1912 |
| | | | | | 'PC3SNGnh.1 48_x_at' | 141 | 373 | 2001-2011 |
| | | | | | 'PC3SNGnh.3 957_at' | 149 | 381 | 2089-2099 |
| | | | | | 'PCADNP.36 40_at' | 185 | 417 | 2485-2495 |
| | | | | | 'PCADNP.36 40_x_at' | 186 | 418 | 2496-2506 |
| | | | | | 'PCPD.14169 .C1_at' | 210 | 442 | 2769-2779 |
| | | | | | 'PCPD.14169 .C1_x_at' | 211 | 443 | 2780-2790 |
| | | | | | 'PCPD.20005 .C1_at' | 213 | 445 | 2801-2811 |
| | | | | | 'PCPD.20005 .C1_x_at' | 214 | 446 | 2812-2822 |
| | | | | | 'PCPD.5961. C1_at' | 221 | 453 | 2887-2897 |
| -0.005837135 | 7.07390658 | 0.00583713 5 | 25 | PAGE4 | 'PCHP.651_s_ at' | 208 | 440 | 2747-2757 |
| -0.005684812 | 8.10529536 2 | 0.00568481 2 | 26 | STEAP 4 | '3Snip.1577-444a_s_at' | 1 | 233 | 465-475 |
| | | | | | 'PC3P.2452.C 1_s_at' | 68 | 300 | 1187-1197 |
| | | | | | 'PC3P.2452.C 1-520a_s_at' | 69 | 301 | 1198-1208 |
| | | | | | 'PC3SNG.367 0-154a_s_at' | 129 | 361 | 1869-1879 |
| -0.00564663 | 7.59452596 | 0.00564663 | 27 | TMEM1 78A | 'PC3P.2736.C 1_at' | 70 | 302 | 1209-1219 |

| Signature Weight | Signature Bias | Weight (absolute) | Rank by weight | Gene symbol | Probesets | SEQ ID NO of sequence | | |
|---|---|---|---|---|---|---|---|---|
| | | | | | | Full | Target | Probe |
| -0.005597719 | 8.92897751 4 | 0.00559771 9 | 28 | CXCL2 | 'PCHP.412_x _at' | 203 | 435 | 2693-2703 |
| -0.005593197 | 4.23278173 2 | 0.00559319 7 | 29 | HS3ST3 A1 | '3Snip.377-232a_s_at' | 6 | 238 | 520-530 |
| | | | | | 'PCADA.1220 9_at' | 166 | 398 | 2276-2286 |
| | | | | | 'PCADA.1220 9_x_at' | 167 | 399 | 2287-2297 |
| -0.005581031 | 5.50427620 4 | 0.00558103 1 | 30 | EYA1 | '3Snip.546-712a_s_at' | 10 | 242 | 564-574 |
| | | | | | 'PC3P.4095.C 1_at' | 82 | 314 | 1341-1351 |
| | | | | | 'PC3P.4095.C 1_x_at' | 83 | 315 | 1352-1362 |
| -0.005562783 | 3.92242079 4 | 0.00556278 3 | 31 | RSPO2 | 'PC3SNGnh.4 553_s_at' | 151 | 383 | 2111-2121 |
| | | | | | PCPD.3722.C 1_s_at' | 218 | 450 | 2854-2864 |
| | | | | | 'PC3P.16583. C1_at' | 59 | 291 | 1088-1098 |
| | | | | | 'PC3P.16583. C1_x_at' | 60 | 292 | 1099-1109 |
| -0.005553136 | 5.91218617 1 | 0.00555313 6 | 32 | PKP1 | '3Snip.4433-2675a_s_at' | 7 | 239 | 531-541 |
| | | | | | 'PC3P.6847.C 1_s_at' | 98 | 330 | 1517-1527 |
| -0.005522157 | 6.64003727 4 | 0.00552215 7 | 33 | MUC6 | 'PC3P.15628. C1_s_at' | 50 | 282 | 989-999 |
| -0.005505761 | 4.51485504 9 | 0.00550576 1 | 34 | PENK | 'PCADNP.90 49_s_at' | 190 | 422 | 2540-2550 |
| | | | | | 'PCRS2.6477 _s_at' | 229 | 461 | 2972-2982 |
| -0.005399899 | 6.82549092 4 | 0.00539989 9 | 35 | DEFB1 | '3Snip.1845-41a_x_at' | 2 | 234 | 476-486 |
| | | | | | '3Snip.5724-41a_s_at' | 11 | 243 | 575-585 |
| -0.005389518 | 4.64900363 | 0.00538951 8 | 36 | SLC7A3 | 'PCADA.1045 9_at' | 164 | 396 | 2254-2264 |
| -0.00535523 | 5.08738932 | 0.00535523 | 37 | MIR57 8 | 'PC3SNGnh.4 158_at' | 150 | 382 | 2100-2110 |

| Signature Weight | Signature Bias | Weight (absolute) | Rank by weight | Gene symbol | Probesets | SEQ ID NO of sequence | | |
|---|---|---|---|---|---|---|---|---|
| | | | | | | Full | Target | Probe |
| -0.005263663 | 4.85871624 3 | 0.00526366 3 | 38 | PI15 | '3Snip.2873-1277a_at' | 4 | 236 | 498-508 |
| | | | | | PC3P.7245.C 1_at' | 99 | 331 | 1528-1538 |
| | | | | | PC3P.7245.C 1_x_at' | 100 | 332 | 1539-1549 |
| | | | | | 'PC3P.8311.C 1_x_at' | 110 | 342 | 1649-1659 |
| | | | | | 'PC3P.8311.C 1-482a_s_at' | 111 | 343 | 1660-1670 |
| | | | | | 'PCADNP.17 332_s_at' | 182 | 414 | 2452-2462 |
| -0.005259309 | 6.06587761 | 0.00525930 | 39 | UBXN1 | 'PCPD.39829 | 219 | 451 | 2865-2875 |
| | 5 | 9 | | 0-AS1 | .C1_s_at' | | | |
| -0.00524875 | 4.17409431 2 | 0.00524875 | 40 | PDK4 | 'PC3P.16300. C1_at' | 52 | 284 | 1011-1021 |
| | | | | | 'PC3P.16300. C1_x_at' | 53 | 285 | 1022-1032 |
| | | | | | 'PC3P.16894. C1_x_at' | 63 | 295 | 1132-1142 |
| | | | | | 'PC3P.8159.C 1_s_at' | 108 | 340 | 1627-1637 |
| | | | | | 'PC3P.8159.C 1-773a_s_at' | 109 | 341 | 1638-1648 |
| | | | | | 'PC3SNGnh.4 912_at' | 152 | 384 | 2122-2132 |
| | | | | | 'PC3SNGnh.4 912_x_at' | 153 | 385 | 2133-2143 |
| | | | | | 'PC3SNGnh.5 369_at' | 157 | 389 | 2177-2187 |
| | | | | | 'PC3SNGnh.5 369_x_at' | 158 | 390 | 2188-2198 |
| | | | | | 'PCADNP.18 913_s_at' | 184 | 416 | 2474-2484 |
| | | | | | 'PCEM.2221_ at' | 194 | 426 | 2584-2594 |
| | | | | | 'PCPD.29484 .C1_at' | 216 | 448 | 2834-2844 |
| -0.0052075 | 5.18357114 3 | 0.0052075 | 41 | PHGR1 | '3Snip.3288-5a_x_at' | 5 | 237 | 509-519 |

(continued)

| Signature Weight | Signature Bias | Weight (absolute) | Rank by weight | Gene symbol | Probesets | SEQ ID NO of sequence | | |
|---|---|---|---|---|---|---|---|---|
| | | | | | | Full | Target | Probe |
| -0.005194886 | 6.69186628 4 | 0.00519488 6 | 42 | SERPIN E1 | '3Snip.7067-10a_s_at | 13 | 245 | 597-607 |
| | | | | | '3Snip.7068-570a_s_at' | 14 | 246 | 608-618 |
| | | | | | 'PC3P.3933.C 1_s_at' | 81 | 313 | 1330-1340 |
| | | | | | 'PC3P.9147.C 1_s_at' | 115 | 347 | 1704-1714 |
| | | | | | 'PCADNP.43 00_x_at' | 187 | 419 | 2507-2517 |
| | | | | | 'PCHP.1474_ s_at' | 200 | 432 | 2650-2660 |
| -0.005146623 | 4.75232765 2 | 0.00514662 3 | 43 | PDZRN 4 | 'PC3P.15181. C1_at' | 47 | 279 | 956-966 |
| | | | | | 'PC3P.15181. C1_s_at' | 48 | 280 | 967-977 |
| | | | | | 'PC3P.15181. C1_x_at' | 49 | 281 | 978-988 |
| | | | | | 'PC3P.16541. C1_at' | 50 | 290 | 1077-1087 |
| -0.005105327 | 6.90054422 | 0.00510532 7 | 44 | ZNF18 5 | 'PCHP.120_s_ at' | 198 | 430 | 2628-2638 |
| -0.005054713 | 7.07837686 4 | 0.00505471 3 | 45 | ADRA2 C | 'PCADA.8850 _s_at' | 176 | 408 | 2385-2396 |
| -0.0050184 | 8.19117750 1 | 0.0050184 | 46 | AZGP1 | 'PC3P.122.C B1_x_at' | 26 | 258 | 729-739 |
| | | | | | 'PC3P.122.C B2_at' | 27 | 259 | 740-749 |
| | | | | | 'PC3SNG.105 5-28a_x_at' | 122 | 354 | 1792-1802 |
| 0.004965887 | 5.58133457 | 0.00496588 7 | 47 | TK1 | 'PCHP.1153_ s_at' | 197 | 429 | 2617-2627 |
| -0.004961473 | 4.82497632 5 | 0.00496147 3 | 48 | POTEH | 'PC3SNGnh.3 389_at' | 147 | 379 | 2067-2077 |
| | | | | | 'PC3SNGnh.3 389_x_at' | 148 | 380 | 2078-2088 |
| | | | | | 'PCPD.5859. C2_at' | 220 | 452 | 2876-2886 |
| | | | | | 'PCRS.626_x_ at' | 224 | 456 | 2920-2930 |
| 0.004928774 | 3.91766850 1 | 0.00492877 4 | 49 | KIF11 | 'PCADNP.16 534_at' | 180 | 412 | 2430-2440 |
| | | | | | 'PCADNP.16 534_x_at' | 181 | 413 | 2441-2451 |

EP 3 310 927 B1

13

| Signature Weight | Signature Bias | Weight (absolute) | Rank by weight | Gene symbol | Probesets | SEQ ID NO of sequence | | |
|---|---|---|---|---|---|---|---|---|
| | | | | | | Full | Target | Probe |
| -0.004924383 | 4.96028271 3 | 0.00492438 3 | 50 | CLDN1 | 'PC3P.2825.C 1_at' | 72 | 304 | 1231-1241 |
| | | | | | 'PC3P.2825.C 1_x_at' | 73 | 305 | 1242-1252 |
| | | | | | 'PC3SNGnh.7 327_x_at' | 162 | 394 | 2232-2242 |
| | | | | | 'PCADA.1207 2_at' | 165 | 397 | 2265-2275 |
| | | | | | 'PCADA.7259 _at' | 172 | 404 | 2342-2352 |
| | | | | | 'PCADA.7259 _x_at' | 173 | 405 | 2353-2363 |
| -0.004907676 | 10.5364522 3 | 0.00490767 6 | 51 | MIR45 30 | 'PCPD.1539. C1_s_at' | 212 | 444 | 2791-2800 |
| -0.004901224 | 8.49794525 1 | 0.00490122 4 | 52 | MAFF | 'PC3P.12787. C1_x_at' | 33 | 265 | 805-815 |
| | | | | | 'PCADA.1334 8_at' | 169 | 401 | 2309-2319 |
| | | | | | 'PCADA.1334 8_x_at' | 170 | 402 | 2320-2330 |
| -0.004861949 | 3.97633303 4 | 0.00486194 9 | 53 | ZNF76 5 | 'PC3P.3163.C 1_s_at' | 76 | 308 | 1275-1285 |
| | | | | | 'PCRS.812_s_ at' | 225 | 457 | 2931-2941 |
| 0.00485589 | 6.50398071 5 | 0.00485589 | 54 | CKS2 | 'PCHP.43_s_a t' | 204 | 436 | 2704-2714 |
| -0.004855875 | 4.81932798 3 | 0.00485587 5 | 55 | TCEAL 7 | 'PCADA.8842 _at' | 174 | 406 | 2364-2373 |
| 0.004830634 | 4.62939179 3 | 0.00483063 4 | 56 | PLIN1 | 'PC3P.12706. C1_s_at' | 31 | 263 | 783-793 |
| 0.004772601 | 5.50375238 3 | 0.00477260 1 | 57 | SIGLEC 1 | 'PC3SNG.521 5-18a_s_at' | 131 | 363 | 1891-1901 |
| -0.004772585 | 6.66459522 | 0.00477258 | 58 | FAM15 | 'PCRS2.7477 | 230 | 462 | 2983-2993 |
| | 4 | 5 | | 0B | _s_at' | | | |
| -0.004771653 | 4.12917654 6 | 0.00477165 3 | 59 | MFAP5 | '3Snip.4760-1950a_s_at' | 9 | 241 | 553-563 |
| | | | | | 'PC3SNG.440 7-18a_s_at' | 130 | 362 | 1880-1890 |
| -0.004761531 | 7.90126194 4 | 0.00476153 1 | 60 | SFRP1 | 'PC3P.9317.C 1_s_at' | 116 | 348 | 1715-1725 |
| | | | | | 'PC3SNG.195 8-2386a_s_at' | 126 | 358 | 1836-1846 |

| Signature Weight | Signature Bias | Weight (absolute) | Rank by weight | Gene symbol | Probesets | SEQ ID NO of sequence | | |
|---|---|---|---|---|---|---|---|---|
| | | | | | | Full | Target | Probe |
| -0.00471806 | 5.76267783 4 | 0.00471806 | 61 | DUSP5 | 'PC3P.1626.C 1_s_at' | 51 | 283 | 1000-1010 |
| | | | | | 'PCPD.2281. C1_at' | 215 | 447 | 2823-2833 |
| | | | | | 'PCRS2.2880 _s_at' | 226 | 458 | 2942-2951 |
| 0.004675188 | 5.22345519 2 | 0.00467518 8 | 62 | VARS2 | 'PC3P.4347.C 1_s_at' | 84 | 316 | 1363-1373 |
| -0.004664227 | 5.23037674 7 | 0.00466422 7 | 63 | ABCC4 | 'PC3P.3552.C 1_s_at' | 77 | 309 | 1286-1296 |
| | | | | | 'PC3P.4471.C 1_s_at' | 85 | 317 | 1374-1384 |
| | | | | | 'PC3P.4471.C 1-536a_s_at' | 86 | 318 | 1385-1395 |
| | | | | | 'PC3P.5711.C 1_at' | 92 | 324 | 1451-1461 |
| | | | | | 'PC3P.5711.C 1_s_at' | 93 | 325 | 1462-1472 |
| | | | | | 'PC3P.5711.C 2_at' | 94 | 326 | 1473-1483 |
| | | | | | 'PC3P.5711.C 2_x_at' | 95 | 327 | 1484-1494 |
| | | | | | 'PC3P.777.C1 _at' | 104 | 336 | 1583-1593 |
| | | | | | 'PC3P.777.C1 _x_at' | 105 | 337 | 1594-1564 |
| | | | | | 'PC3P.9828.C 1_s_at' | 119 | 351 | 1748-1758 |
| | | | | | 'PC3SNG.704 -22a_s_at' | 134 | 366 | 1924-1934 |
| | | | | | 'PC3SNGnh.1 41_x_at' | 136 | 368 | 1946-1946 |
| | | | | | 'PC3SNGnh.1 473_at' | 139 | 371 | 1979-1989 |
| | | | | | 'PC3SNGnh.1 473_x_at' | 140 | 372 | 1990-2000 |
| | | | | | 'PC3SNGnh.6 624_x_at' | 160 | 392 | 2210-2220 |
| | | | | | 'PC3SNGnh.6 679_s_at' | 161 | 393 | 2221-2231 |
| | | | | | 'PCADA.445_ s_at' | 171 | 403 | 2331-2341 |
| | | | | | 'PCADNP.11 46_s_at' | 178 | 410 | 2408-2418 |
| | | | | | 'PCADNP.12 255_at' | 179 | 411 | 2419-2429 |
| | | | | | PCPD.7116.C 1_at' | 222 | 454 | 2898-2908 |

| Signature Weight | Signature Bias | Weight (absolute) | Rank by weight | Gene symbol | Probesets | SEQ ID NO of sequence | | |
|---|---|---|---|---|---|---|---|---|
| | | | | | | Full | Target | Probe |
| | | | | | 'PCPD.7116. C1_x_at' | 223 | 455 | 2909-2919 |
| -0.004622969 | 4.88270806 7 | 0.00462296 9 | 64 | SH3BP 4 | 'PC3P.12104. C1_at' | 25 | 257 | 718-728 |
| | | | | | 'PC3P.14133. C1_at' | 41 | 273 | 893-903 |
| | | | | | 'PC3P.14133. C1_x_at' | 42 | 274 | 904-914 |
| | | | | | 'PC3SNGnh.1 032_x_at' | 135 | 367 | 1935-1945 |
| | | | | | 'PC3SNGnh.1 675_x_at' | 142 | 374 | 2012-2022 |
| | | | | | 'PC3SNGnh.4 946_at' | 154 | 386 | 2144-2154 |
| | | | | | 'PC3SNGnh.4 946_x_at' | 155 | 387 | 2155-2165 |
| | | | | | 'PC3SNGnh.5 | 156 | 388 | 2166-2993 |
| -0.004573155 | 8.95841106 9 | 0.00457315 5 | 65 | SORD | 297_x_at' | | | 2176 |
| | | | | | 'PCADNP.61 93_s_at' | 189 | 421 | 2529-2539 |
| | | | | | 'PC3P.14629. C1_s_at' | 44 | 276 | 926-933 |
| | | | | | 'PC3P.525.C B1_s_at' | 90 | 322 | 1429-1439 |
| | | | | | 'PC3P.525.C B1-789a_s_at' | 91 | 323 | 1440-1450 |
| | | | | | 'PC3P.9417.C 1_s_at' | 117 | 349 | 1726-1736 |
| 0.004522466 | 5.33419878 3 | 0.00452246 6 | 66 | MTERF D1 | 'PC3P.14465. C1_s_at' | 43 | 275 | 915-925 |
| -0.004505906 | 4.65974831 | 0.00450590 6 | 67 | DPP4 | '3Snip.2321-634a_s_at' | 3 | 235 | 487-497 |
| | | | | | 'PC3P.11025. C1_s_at' | 21 | 253 | 674-684 |
| | | | | | 'PC3P.4974.C 1_s_at' | 87 | 319 | 1396-1406 |
| | | | | | 'PCADNP.91 81_at' | 191 | 423 | 2551-2661 |
| | | | | | 'PCEM.2151_ at' | 193 | 425 | 2573-2583 |
| | | | | | 'PCHP.235_s_ at' | 202 | 434 | 2672-2682 |
| 0.004502134 | 4.90531269 2 | 0.00450213 4 | 68 | N/A | 'PC3SNG.662 6-95a_s_at' | 133 | 365 | 1913-1923 |

| Signature Weight | Signature Bias | Weight (absolute) | Rank by weight | Gene symbol | Probesets | SEQ ID NO of sequence | | |
|---|---|---|---|---|---|---|---|---|
| | | | | | | Full | Target | Probe |
| -0.0044434 | 7.38807128 1 | 0.0044434 | 69 | FAM3B | 'PC3P.8122.C 1_s_at' | 106 | 338 | 1605-1615 |
| | | | | | 'PC3P.8122.C 2_s_at' | 107 | 339 | 1616-1626 |
| | | | | | 'PCADNP.52 63_s_at' | 188 | 420 | 2518-2528 |
| -0.00442472 | 10.2264412 9 | 0.00442472 | 70 | KLK3 | 'PC3P.1038.C 2_s_at' | 18 | 250 | 652-662 |
| | | | | | 'PCADNP.18 829_x_at' | 183 | 415 | 2463-2473 |
| | | | | | 'PCEM.799_x _at' | 195 | 427 | 2595-2605 |
| | | | | | 'PCHP.604_x _at' | 207 | 439 | 2736-2746 |
| | | | | | 'PCHP.785_s_ at' | 209 | 441 | 2758-2768 |

Further details of the probesets can be found in Table 1A, including orientation information:

Table 1A - Probeset Information

| Probeset ID | Orientatio n | NoPA | ENSEMBL gene no. | Gene Symbol | Entrez Gene ID | HGNC symbol acc no | Strand | Csome no |
|---|---|---|---|---|---|---|---|---|
| 3Snip.1577-444a_s_at | Fully Exonic | 11 | ENSG00000127954 | STEAP4 | 79689 | 21923 | Reverse | 7 |
| 3Snip.1845-41a_x_at | Fully Exonic | 11 | ENSG00000164825 | DEFB1 | 1672 | 2766 | Reverse | 8 |
| 3Snip.2321-634a_s_at | Fully Exonic | 11 | ENSG00000197635 | DPP4 | 1803 | 3009 | Reverse | 2 |
| 3Snip.2873-1277a_at | Fully Exonic | 11 | ENSG00000137558 | PI15 | 51050 | 8946 | Forward | 8 |
| 3Snip.3288-5a_x_at | Fully Exonic | 11 | ENSG00000233041 | PHGR1 | 64484 4 | 37226 | Forward | 15 |
| 3Snip.377-232a_s_at | Fully Exonic | 11 | ENSG00000153976 | HS3ST3 A1 | 9955 | 5196 | Reverse | 17 |
| 3Snip.4433-2675a_s_at | Fully Exonic | 10 | ENSG00000081277 | PKP1 | 5317 | 9023 | Forward | 1 |
| 3Snip.465-263a_s_at | Fully Exonic | 11 | ENSG00000146374 | RSPO3 | 84870 | 20866 | Forward | 6 |
| 3Snip.4760-1950a_s_at | Fully Exonic | 11 | ENSG00000197614 | MFAP5 | 8076 | 29673 | Reverse | 12 |
| 3Snip.546-712a_s_at | Fully Exonic | 11 | ENSG00000104313 | EYA1 | 2138 | 3519 | Reverse | 8 |
| 3Snip.5724-41a_s_at | Fully Exonic | 10 | ENSG00000164825 | DEFB1 | 1672 | 2766 | Reverse | 8 |
| 3Snip.6683-12a_x_at | Fully Exonic | 11 | ENSG00000225937 | PCA3 | 50652 | 8637 | Forward | 9 |
| 3Snip.7067-10a_s_at | Fully Exonic | 11 | ENSG00000106366 | SERPIN E1 | 5054 | 8583 | Forward | 7 |
| 3Snip.7068-570a_s_at | Fully Exonic | 11 | ENSG00000106366 | SERPIN E1 | 5054 | 8583 | Forward | 7 |
| 3Snip.7769-1124a_at | Fully Exonic | 11 | ENSG00000077274 | CAPN6 | 827 | 1483 | Reverse | X |
| 3Snip.972-5a_s_at | Fully Exonic | 11 | ENSG00000124233 | SEMG1 | 6406 | 10742 | Forward | 20 |
| PC3P.10239 .C1_s_at | Fully Exonic | 11 | ENSG00000186081 | KRT5 | 3852 | 6442 | Reverse | 12 |
| PC3P.1038. C2_s_at | Fully Exonic | 11 | ENSG00000142515 | KLK3 | 354 | 6364 | Forward | 19 |
| PC3P.104.C B1_s_at | Fully Exonic | 11 | ENSG00000196136 | SERPIN A3 | 12 | 16 | Forward | 14 |
| B1_s_at | PC3P.104.C Fully Exonic | 11 | ENSG00000273259 | N/A | 12 | NOVEL pc | Forward | 14 |
| PC3P.11025 .C1_s_at | Fully Exonic | 9 | ENSG00000197635 | DPP4 | 1803 | 3009 | Reverse | 2 |
| PC3P.11294 .C1_s_at | Fully Exonic | 11 | ENSG00000225937 | PCA3 | 50652 | 8637 | Forward | 9 |
| PC3P.11557 .C1_s_at | Fully Exonic | 11 | ENSG00000183036 | PCP4 | 5121 | 8742 | Forward | 21 |
| PC3P.12013 .C1_s_at | Fully Exonic | 11 | ENSG00000144481 | TRPM8 | 79054 | 17961 | Forward | 2 |
| PC3P.12104 .C1_at | Fully Exonic | 11 | ENSG00000130147 | SH3BP4 | 23677 | 10826 | Forward | 2 |

EP 3 310 927 B1

19

| Probeset ID | Orientatio n | NoPA | ENSEMBL gene no. | Gene Symbol | Entrez Gene ID | HGNC symbol acc no | Strand | Csome no |
|---|---|---|---|---|---|---|---|---|
| PC3P.122.C B1_x_at | Fully Exonic | 7 | ENSG00000160862 | AZGP1 | 563 | 910 | Reverse | 7 |
| PC3P.122.C B2_at | Fully Exonic | 10 | ENSG00000160862 | AZGP1 | 563 | 910 | Reverse | 7 |
| PC3P.12363 .C1_s_at | Fully Exonic | 11 | ENSG00000113296 | THBS4 | 7060 | 11788 | Forward | 5 |
| PC3P.12591 .C1_x_at | Includes Intronic | 11 | ENSG00000144481 | TRPM8 | 79054 | 17961 | Forward | 2 |
| PC3P.1261. C1_s_at | Fully Exonic | 11 | ENSG00000144481 | TRPM8 | 79054 | 17961 | Forward | 2 |
| PC3P.12706 .C1_s_at | Fully Exonic | 11 | ENSG00000166819 | PLIN1 | 5346 | 9076 | Reverse | 15 |
| PC3P.12756 .C1_x_at | Includes Intronic | 9 | ENSG00000255240 | N/A | 28319 4 | NOVEL as | Reverse | 11 |
| PC3P.12787 .C1_x_at | Fully Exonic | 11 | ENSG00000185022 | MAFF | 23764 | 6780 | Forward | 22 |
| PC3P.12920 .C1_x_at | Fully Exonic | 11 | ENSG00000250423 | KIAA12 10 | 57481 | 29218 | Reverse | X |
| PC3P.13143 .C1_at | Includes Intronic | 9 | ENSG00000225937 | PCA3 | 50652 | 8637 | Forward | 9 |
| PC3P.13143 .C1_x_at | Includes Intronic | 10 | ENSG00000225937 | PCA3 | 50652 | 8637 | Forward | 9 |
| PC3P.1358. C1_at | Fully Exonic | 11 | ENSG00000146205 | ANO7 | 50636 | 31677 | Forward | 2 |
| PC3P.1358. C1-1172a_s_at | Fully Exonic | 11 | ENSG00000146205 | ANO7 | 50636 | 31677 | Forward | 2 |
| PC3P.13654 .C1_at | Includes Intronic | 10 | ENSG00000196091 | MYBPC1 | 4604 | 7549 | Forward | 12 |
| PC3P.13654 .C1_x_at | Includes Intronic | 9 | ENSG00000196091 | MYBPC1 | 4604 | 7549 | Forward | 12 |
| PC3P.14133 .C1_at | Fully Exonic | 11 | ENSG00000130147 | SH3BP4 | 23677 | 10826 | Forward | 2 |
| PC3P.14133 .C1_x_at | Fully Exonic | 10 | ENSG00000130147 | SH3BP4 | 23677 | 10826 | Forward | 2 |
| PC3P.14465 .C1_s_at | Fully Exonic | 10 | ENSG00000156469 | MTERFD 1 | 51001 | 24258 | Reverse | 8 |
| PC3P.14629 .C1_s_at | Fully Exonic | 8 | ENSG00000140263 | SORD | 6652 | 11184 | Forward | 15 |
| PC3P.1507. C1_at | Fully Exonic | 11 | ENSG00000144481 | TRPM8 | 79054 | 17961 | Forward | 2 |
| PC3P.1507. C1_x_at | Fully Exonic | 11 | ENSG00000144481 | TRPM8 | 79054 | 17961 | Forward | 2 |
| PC3P.15181 .C1_at | Fully Exonic | 11 | ENSG00000165966 | PDZRN4 | 29951 | 30552 | Forward | 12 |
| PC3P.15181 .C1_s_at | Fully Exonic | 11 | ENSG00000165966 | PDZRN4 | 29951 | 30552 | Forward | 12 |
| PC3P.15181 .C1_x_at | Fully Exonic | 11 | ENSG00000165966 | PDZRN4 | 29951 | 30552 | Forward | 12 |
| PC3P.15628 .C1_s_at | Fully Exonic | 11 | ENSG00000184956 | MUC6 | 4588 | 7517 | Reverse | 11 |

| Probeset ID | Orientatio n | NoPA | ENSEMBL gene no. | Gene Symbol | Entrez Gene ID | HGNC symbol acc no | Strand | Csome no |
|---|---|---|---|---|---|---|---|---|
| PC3P.1626. C1_s_at | Fully Exonic | 11 | ENSG00000138166 | DUSP5 | 1847 | 3071 | Forward | 10 |
| PC3P.16300 .C1_at | Includes Intronic | 10 | ENSG00000004799 | PDK4 | 5166 | 8812 | Reverse | 7 |
| PC3P.16300 .C1_x_at | Includes Intronic | 10 | ENSG00000004799 | PDK4 | 5166 | 8812 | Reverse | 7 |
| PC3P.1643. C1_s_at | Fully Exonic | 11 | ENSG00000230937 | MIR205 HG | 40698 8 | 43562 | Forward | 1 |
| PC3P.1643. C4-370a_s_at | Fully Exonic | 11 | ENSG00000230937 | MIR205 HG | 40698 8 | 43562 | Forward | 1 |
| PC3P.1643. C6-335a_s_at | Fully Exonic | 9 | ENSG00000230937 | MIR205 HG | 40698 8 | 43562 | Forward | 1 |
| PC3P.16431 .C1_at | Fully Exonic | 9 | ENSG00000196136 | SERPIN A3 | 12 | 16 | Forward | 14 |
| PC3P.16541 .C1_at | Includes Intronic | 11 | ENSG00000165966 | PDZRN4 | 29951 | 30552 | Forward | 12 |
| PC3P.16583 .C1_at | Fully Exonic | 11 | ENSG00000147655 | RSPO2 | 34041 9 | 28583 | Reverse | 8 |
| PC3P.16583 .C1_x_at | Fully Exonic | 11 | ENSG00000147655 | RSPO2 | 34041 9 | 28583 | Reverse | 8 |
| PC3P.167.C 1_s_at | Fully Exonic | 11 | ENSG00000012223 | LTF | 4057 | 6720 | Reverse | 3 |
| PC3P.16730 .C1_x_at | Fully Exonic | 8 | ENSG00000164611 | PTTG1 | 9232 | 9690 | Forward | 5 |
| PC3P.16894 .C1_x_at | Fully Exonic | 11 | ENSG00000004799 | PDK4 | 5166 | 8812 | Reverse | 7 |
| PC3P.17142 .C1_s_at | Fully Exonic | 11 | ENSG00000123560 | PLP1 | 5354 | 9086 | Forward | X |
| PC3P.1906. C1_s_at | Fully Exonic | 11 | ENSG00000125740 | FOSB | 2354 | 3797 | Forward | 19 |
| PC3P.1906. C1-568a_s_at | Fully Exonic | 11 | ENSG00000125740 | FOSB | 2354 | 3797 | Forward | 19 |
| PC3P.2274. C1_s_at | Fully Exonic | 11 | ENSG00000225937 | PCA3 | 50652 | 8637 | Forward | 9 |
| PC3P.2452. C1_s_at | Fully Exonic | 11 | ENSG00000127954 | STEAP4 | 79689 | 21923 | Reverse | 7 |
| PC3P.2452. C1-520a_s_at | Fully Exonic | 11 | ENSG00000127954 | STEAP4 | 79689 | 21923 | Reverse | 7 |
| PC3P.2736. C1_at | Fully Exonic | 9 | ENSG00000152154 | TMEM1 78A | 13073 3 | 28517 | Forward | 2 |
| PC3P.2763. C1_s_at | Fully Exonic | 11 | ENSG00000137673 | MMP7 | 4316 | 7174 | Reverse | 11 |
| PC3P.2825. C1_at | Fully Exonic | 10 | ENSG00000163347 | CLDN1 | 9076 | 2032 | Reverse | 3 |
| PC3P.2825. C1_x_at | Fully Exonic | 10 | ENSG00000163347 | CLDN1 | 9076 | 2032 | Reverse | 3 |
| PC3P.3003. C1_s_at | Fully Exonic | 11 | ENSG00000196091 | MYBPC1 | 4604 | 7549 | Forward | 12 |
| PC3P.3003. C1_x_at | Includes Intronic | 11 | ENSG00000196091 | MYBPC1 | 4604 | 7549 | Forward | 12 |

(continued)

| Probeset ID | Orientatio n | NoPA | ENSEMBL gene no. | Gene Symbol | Entrez Gene ID | HGNC symbol acc no | Strand | Csome no |
|---|---|---|---|---|---|---|---|---|
| PC3P.3163. C1_s_at | Fully Exonic | 11 | ENSG00000196417 | ZNF765 | 91661 | 25092 | Forward | 19 |
| PC3P.3552. C1_s_at | Includes Intronic | 11 | ENSG00000125257 | ABCC4 | 10257 | 55 | Reverse | 13 |
| PC3P.3670. C1_s_at | Fully Exonic | 11 | ENSG00000144481 | TRPM8 | 79054 | 17961 | Forward | 2 |
| PC3P.3670. C1-625a_s_at | Fully Exonic | 11 | ENSG00000144481 | TRPM8 | 79054 | 17961 | Forward | 2 |
| PC3P.3670. C2_s_at | Fully Exonic | 11 | ENSG00000144481 | TRPM8 | 79054 | 17961 | Forward | 2 |
| PC3P.3933. C1_s_at | Fully Exonic | 11 | ENSG00000106366 | SERPIN E1 | 5054 | 8583 | Forward | 7 |
| PC3P.4095. C1_at | Fully Exonic | 11 | ENSG00000104313 | EYA1 | 2138 | 3519 | Reverse | 8 |
| PC3P.4095. C1_x_at | Fully Exonic | 11 | ENSG00000104313 | EYA1 | 2138 | 3519 | Reverse | 8 |
| PC3P.4347. C1_s_at | Fully Exonic | 11 | ENSG00000137411 | VARS2 | 57176 | 21642 | Forward | 6 |
| PC3P.4471. C1_s_at | Fully Exonic | 11 | ENSG00000125257 | ABCC4 | 10257 | 55 | Reverse | 13 |
| PC3P.4471. C1-536a_s_at | Fully Exonic | 11 | ENSG00000125257 | ABCC4 | 10257 | 55 | Reverse | 13 |
| PC3P.4974. C1_s_at | Fully Exonic | 11 | ENSG00000197635 | DPP4 | 1803 | 3009 | Reverse | 2 |
| PC3P.5053. C1_s_at | Fully Exonic | 11 | ENSG00000225937 | PCA3 | 50652 | 8637 | Forward | 9 |
| PC3P.5053. C1-490a_s_at | Fully Exonic | 11 | ENSG00000225937 | PCA3 | 50652 | 8637 | Forward | 9 |
| PC3P.525.C B1_s_at | Fully Exonic | 11 | ENSG00000140263 | SORD | 6652 | 11184 | Forward | 15 |
| PC3P.525.C B1-789a_s_at | Fully Exonic | 11 | ENSG00000140263 | SORD | 6652 | 11184 | Forward | 15 |
| PC3P.5711. C1_at | Includes Intronic | 11 | ENSG00000125257 | ABCC4 | 10257 | 55 | Reverse | 13 |
| PC3P.5711. C1_s_at | Fully Exonic | 10 | ENSG00000125257 | ABCC4 | 10257 | 55 | Reverse | 13 |
| PC3P.5711. C2_at | Fully Exonic | 11 | ENSG00000125257 | ABCC4 | 10257 | 55 | Reverse | 13 |
| PC3P.5711. C2_x_at | Fully Exonic | 11 | ENSG00000125257 | ABCC4 | 10257 | 55 | Reverse | 13 |
| PC3P.5784. C1_at | Includes Intronic | 8 | ENSG00000255240 | N/A | 28319 4 | NOVEL as | Reverse | 11 |
| PC3P.5784. C1_x_at | Includes Intronic | 10 | ENSG00000255240 | N/A | 28319 4 | NOVEL as | Reverse | 11 |
| PC3P.6847. C1_s_at | Fully Exonic | 11 | ENSG00000081277 | PKP1 | 5317 | 9023 | Forward | 1 |
| PC3P.7245. C1_at | Fully Exonic | 11 | ENSG00000137558 | PI15 | 51050 | 8946 | Forward | 8 |
| PC3P.7245. C1_x_at | Fully Exonic | 11 | ENSG00000137558 | PI15 | 51050 | 8946 | Forward | 8 |

| Probeset ID | Orientation | NoPA | ENSEMBL gene no. | Gene Symbol | Entrez Gene ID | HGNC symbol acc no | Strand | Csome no |
|---|---|---|---|---|---|---|---|---|
| PC3P.7685. C1_at | Fully Exonic | 11 | ENSG00000196091 | MYBPC1 | 4604 | 7549 | Forward | 12 |
| PC3P.7685. C1_x_at | Fully Exonic | 11 | ENSG00000196091 | MYBPC1 | 4604 | 7549 | Forward | 12 |
| PC3P.7685. C1-693a_s_at | Fully Exonic | 11 | ENSG00000196091 | MYBPC1 | 4604 | 7549 | Forward | 12 |
| PC3P.777.C 1_at | Includes Intronic | 11 | ENSG00000125257 | ABCC4 | 10257 | 55 | Reverse | 13 |
| PC3P.777.C 1_x_at | Includes Intronic | 11 | ENSG00000125257 | ABCC4 | 10257 | 55 | Reverse | 13 |
| PC3P.8122. C1_s_at | Fully Exonic | 11 | ENSG00000183844 | FAM3B | 54097 | 1253 | Forward | 21 |
| PC3P.8122. C2_s_at | Fully Exonic | 11 | ENSG00000183844 | FAM3B | 54097 | 1253 | Forward | 21 |
| PC3P.8159. C1_s_at | Fully Exonic | 11 | ENSG00000004799 | PDK4 | 5166 | 8812 | Reverse | 7 |
| PC3P.8159. C1-773a_s_at | Fully Exonic | 11 | ENSG00000004799 | PDK4 | 5166 | 8812 | Reverse | 7 |
| PC3P.8311. C1_x_at | Fully Exonic | 6 | ENSG00000137558 | PI15 | 51050 | 8946 | Forward | 8 |
| PC3P.8311. C1-482a_s_at | Fully Exonic | 11 | ENSG00000137558 | PI15 | 51050 | 8946 | Forward | 8 |
| PC3P.8725. C1_at | Includes Intronic | 9 | ENSG00000255240 | N/A | 28319 4 | NOVEL as | Reverse | 11 |
| PC3P.8725. C1_x_at | Includes Intronic | 7 | ENSG00000255240 | N/A | 28319 4 | NOVEL as | Reverse | 11 |
| PC3P.8968. C1_s_at | Includes Intronic | 11 | ENSG00000255240 | N/A | 28319 4 | NOVEL as | Reverse | 11 |
| PC3P.9147. C1_s_at | Fully Exonic | 11 | ENSG00000106366 | SERPIN E1 | 5054 | 8583 | Forward | 7 |
| PC3P.9317. C1_s_at | Fully Exonic | 11 | ENSG00000104332 | SFRP1 | 6422 | 10776 | Reverse | 8 |
| PC3P.9417. C1_s_at | Fully Exonic | 11 | ENSG00000140263 | SORD | 6652 | 11184 | Forward | 15 |
| PC3P.9581. C1_x_at | Fully Exonic | 9 | ENSG00000012223 | LTF | 4057 | 6720 | Reverse | 3 |
| PC3P.9828. C1_s_at | Fully Exonic | 11 | ENSG00000125257 | ABCC4 | 10257 | 55 | Reverse | 13 |
| PC3P.9903. C1_at | Fully Exonic | 11 | ENSG00000255240 | N/A | 28319 4 | NOVEL as | Reverse | 11 |
| PC3P.9903. C1_x_at | Fully Exonic | 11 | ENSG00000255240 | N/A | 28319 4 | NOVEL as | Reverse | 11 |
| PC3SNG.105 5-28a_x_at | Fully Exonic | 11 | ENSG00000160862 | AZGP1 | 563 | 910 | Reverse | 7 |
| PC3SNG.146 7-30a_s_at | Fully Exonic | 11 | ENSG00000012223 | LTF | 4057 | 6720 | Reverse | 3 |
| PC3SNG.154 9-27a_s_at | Fully Exonic | 11 | ENSG00000148677 | ANKRD1 | 27063 | 15819 | Reverse | 10 |
| PC3SNG.174 2-20a_s_at | Fully Exonic | 11 | ENSG00000146205 | ANO7 | 50636 | 31677 | Forward | 2 |

| Probeset ID | Orientatio n | NoPA | ENSEMBL gene no. | Gene Symbol | Entrez Gene ID | HGNC symbol acc no | Strand | Csome no |
|---|---|---|---|---|---|---|---|---|
| PC3SNG.195 8-2386a_s_at | Fully Exonic | 11 | ENSG00000104332 | SFRP1 | 6422 | 10776 | Reverse | 8 |
| PC3SNG.366 9-40a_s_at | Fully Exonic | 11 | ENSG00000196136 | SERPIN A3 | 12 | 16 | Forward | 14 |
| PC3SNG.366 9-40a_s_at | Fully Exonic | 11 | ENSG00000273259 | N/A | 12 | NOVEL pc | Forward | 14 |
| PC3SNG.367 0-154a_s_at | Fully Exonic | 11 | ENSG00000127954 | STEAP4 | 79689 | 21923 | Reverse | 7 |
| PC3SNG.440 7-18a_s_at | Fully Exonic | 11 | ENSG00000197614 | MFAP5 | 8076 | 29673 | Reverse | 12 |
| PC3SNG.521 5-18a_s_at | Fully Exonic | 11 | ENSG00000088827 | SIGLEC1 | 6614 | 11127 | Reverse | 20 |
| PC3SNG.638 7-29a_x_at | Includes Intronic | 11 | ENSG00000255240 | N/A | 28319 4 | NOVEL as | Reverse | 11 |
| PC3SNG.662 6-95a_s_at | Fully Exonic | 11 | ENSG00000215458 | N/A | 28483 7 | NOVEL as | Reverse | 21 |
| PC3SNG.704 -22a_s_at | Fully Exonic | 11 | ENSG00000125257 | ABCC4 | 10257 | 55 | Reverse | 13 |
| PC3SNGnh.1 032_x_at | Fully Exonic | 6 | ENSG00000130147 | SH3BP4 | 23677 | 10826 | Forward | 2 |
| PC3SNGnh.1 41_x_at | Includes Intronic | 11 | ENSG00000125257 | ABCC4 | 10257 | 55 | Reverse | 13 |
| PC3SNGnh.1 467_at | Includes Intronic | 11 | ENSG00000144481 | TRPM8 | 79054 | 17961 | Forward | 2 |
| PC3SNGnh.1 467_x_at | Includes Intronic | 10 | ENSG00000144481 | TRPM8 | 79054 | 17961 | Forward | 2 |
| PC3SNGnh.1 473_at | Includes Intronic | 7 | ENSG00000125257 | ABCC4 | 10257 | 55 | Reverse | 13 |
| PC3SNGnh.1 473_x_at | Includes Intronic | 6 | ENSG00000125257 | ABCC4 | 10257 | 55 | Reverse | 13 |
| PC3SNGnh.1 48_x_at | Includes Intronic | 9 | ENSG00000255240 | N/A | 28319 4 | NOVEL as | Reverse | 11 |
| PC3SNGnh.1 675_x_at | Fully Exonic | 11 | ENSG00000130147 | SH3BP4 | 23677 | 10826 | Forward | 2 |
| PC3SNGnh.2 659_at | Includes Intronic | 8 | ENSG00000144481 | TRPM8 | 79054 | 17961 | Forward | 2 |
| PC3SNGnh.2 74_x_at | Includes Intronic | 11 | ENSG00000196091 | MYBPC1 | 4604 | 7549 | Forward | 12 |
| PC3SNGnh.3 350_at | Includes Intronic | 11 | ENSG00000144481 | TRPM8 | 79054 | 17961 | Forward | 2 |
| PC3SNGnh.3 350_x_at | Includes Intronic | 11 | ENSG00000144481 | TRPM8 | 79054 | 17961 | Forward | 2 |
| PC3SNGnh.3 389_at | Includes Intronic | 11 | ENSG00000198062 | POTEH | 23784 | 133 | Reverse | 22 |
| PC3SNGnh.3 389_x_at | Includes Intronic | 11 | ENSG00000198062 | POTEH | 23784 | 133 | Reverse | 22 |
| PC3SNGnh.3 957_at | Includes Intronic | 11 | ENSG00000255240 | N/A | 28319 4 | NOVEL as | Reverse | 11 |
| PC3SNGnh.4 158_at | Fully Exonic | 10 | ENSG00000207559 | MIR578 | 69316 3 | 32834 | Forward | 4 |

| Probeset ID | Orientation | NoPA | ENSEMBL gene no. | Gene Symbol | Entrez Gene ID | HGNC symbol acc no | Strand | Csome no |
|---|---|---|---|---|---|---|---|---|
| PC3SNGnh.4 553_s_at | Includes Intronic | 11 | ENSG00000104313 | EYA1 | 2138 | 3519 | Reverse | 8 |
| PC3SNGnh.4 912_at | Includes Intronic | 11 | ENSG00000004799 | PDK4 | 5166 | 8812 | Reverse | 7 |
| PC3SNGnh.4 912_x_at | Includes Intronic | 11 | ENSG00000004799 | PDK4 | 5166 | 8812 | Reverse | 7 |
| PC3SNGnh.4 946_at | Includes Intronic | 9 | ENSG00000130147 | SH3BP4 | 23677 | 10826 | Forward | 2 |
| PC3SNGnh.4 946_x_at | Includes Intronic | 10 | ENSG00000130147 | SH3BP4 | 23677 | 10826 | Forward | 2 |
| PC3SNGnh.5 297_x_at | Fully Exonic | 6 | ENSG00000130147 | SH3BP4 | 23677 | 10826 | Forward | 2 |
| PC3SNGnh.5 369_at | Includes Intronic | 11 | ENSG00000004799 | PDK4 | 5166 | 8812 | Reverse | 7 |
| PC3SNGnh.5 369_x_at | Includes Intronic | 8 | ENSG00000004799 | PDK4 | 5166 | 8812 | Reverse | 7 |
| PC3SNGnh.5 454_at | Includes Intronic | 11 | ENSG00000144481 | TRPM8 | 79054 | 17961 | Forward | 2 |
| PC3SNGnh.6 624_x_at | Includes Intronic | 10 | ENSG00000125257 | ABCC4 | 10257 | 55 | Reverse | 13 |
| PC3SNGnh.6 679_s_at | Includes Intronic | 11 | ENSG00000125257 | ABCC4 | 10257 | 55 | Reverse | 13 |
| PC3SNGnh.7 327_x_at | Includes Intronic | 11 | ENSG00000163347 | CLDN1 | 9076 | 2032 | Reverse | 3 |
| PC3SNGnh.9 32_x_at | Includes Intronic | 11 | ENSG00000225937 | PCA3 | 50652 | 8637 | Forward | 9 |
| PCADA.104 59_at | Fully Exonic | 11 | ENSG00000165349 | SLC7A3 | 84889 | 11061 | Reverse | X |
| PCADA.120 72_at | Fully Exonic | 10 | ENSG00000163347 | CLDN1 | 9076 | 2032 | Reverse | 3 |
| PCADA.122 09_at | Fully Exonic | 11 | ENSG00000153976 | HS3ST3 A1 | 9955 | 5196 | Reverse | 17 |
| PCADA.122 09_x_at | Fully Exonic | 11 | ENSG00000153976 | HS3ST3 A1 | 9955 | 5196 | Reverse | 17 |
| PCADA.127 38_s_at | Fully Exonic | 11 | ENSG00000123560 | PLP1 | 5354 | 9086 | Forward | X |
| PCADA.133 48_at | Fully Exonic | 11 | ENSG00000185022 | MAFF | 23764 | 6780 | Forward | 22 |
| PCADA.133 48_x_at | Fully Exonic | 11 | ENSG00000185022 | MAFF | 23764 | 6780 | Forward | 22 |
| PCADA.445_ s_at | Fully Exonic | 11 | ENSG00000125257 | ABCC4 | 10257 | 55 | Reverse | 13 |
| PCADA.72 5 9_at | Includes Intronic | 11 | ENSG00000163347 | CLDN1 | 9076 | 2032 | Reverse | 3 |
| PCADA.72 5 9_x_at | Includes Intronic | 11 | ENSG00000163347 | CLDN1 | 9076 | 2032 | Reverse | 3 |
| PCADA.884 2_at | Fully Exonic | 11 | ENSG00000182916 | TCEAL7 | 56849 | 28336 | Forward | X |
| PCADA.884 2_x_at | Fully Exonic | 11 | ENSG00000182916 | TCEAL7 | 56849 | 28336 | Forward | X |

| Probeset ID | Orientatio n | NoPA | ENSEMBL gene no. | Gene Symbol | Entrez Gene ID | HGNC symbol acc no | Strand | Csome no |
|---|---|---|---|---|---|---|---|---|
| PCADA.885 0_s_at | Fully Exonic | 11 | ENSG00000184160 | ADRA2 C | 152 | 283 | Forward | 4 |
| PCADA.936 4_s_at | Fully Exonic | 11 | ENSG00000249992 | TMEM1 58 | 25907 | 30293 | Reverse | 3 |
| PCADNP.11 46_s_at | Fully Exonic | 9 | ENSG00000125257 | ABCC4 | 10257 | 55 | Reverse | 13 |
| PCADNP.12 255_at | Includes Intronic | 11 | ENSG00000125257 | ABCC4 | 10257 | 55 | Reverse | 13 |
| PCADNP.16 534_at | Fully Exonic | 11 | ENSG00000138160 | KIF11 | 3832 | 6388 | Forward | 10 |
| PCADNP.16 534_x_at | Fully Exonic | 11 | ENSG00000138160 | KIF11 | 3832 | 6388 | Forward | 10 |
| PCADNP.17 332_s_at | Fully Exonic | 11 | ENSG00000137558 | PI15 | 51050 | 8946 | Forward | 8 |
| PCADNP.18 829_x_at | Fully Exonic | 11 | ENSG00000142515 | KLK3 | 354 | 6364 | Forward | 19 |
| PCADNP.18 913_s_at | Fully Exonic | 11 | ENSG00000004799 | PDK4 | 5166 | 8812 | Reverse | 7 |
| PCADNP.36 40_at | Fully Exonic | 11 | ENSG00000255240 | N/A | 28319 4 | NOVEL as | Reverse | 11 |
| PCADNP.36 40_x_at | Fully Exonic | 11 | ENSG00000255240 | N/A | 28319 4 | NOVEL as | Reverse | 11 |
| PCADNP.43 00_x_at | Includes Intronic | 11 | ENSG00000106366 | SERPIN E1 | 5054 | 8583 | Forward | 7 |
| PCADNP.52 63_s_at | Fully Exonic | 11 | ENSG00000183844 | FAM3B | 54097 | 1253 | Forward | 21 |
| PCADNP.61 93_s_at | Fully Exonic | 11 | ENSG00000130147 | SH3BP4 | 23677 | 10826 | Forward | 2 |
| PCADNP.90 49_s_at | Fully Exonic | 11 | ENSG00000181195 | PENK | 5179 | 8831 | Reverse | 8 |
| PCADNP.91 81_at | Includes Intronic | 10 | ENSG00000197635 | DPP4 | 1803 | 3009 | Reverse | 2 |
| PCEM.1525_ s_at | Fully Exonic | 11 | ENSG00000125740 | FOSB | 2354 | 3797 | Forward | 19 |
| PCEM.2151_ at | Includes Intronic | 11 | ENSG00000197635 | DPP4 | 1803 | 3009 | Reverse | 2 |
| PCEM.2221_ at | Fully Exonic | 11 | ENSG00000004799 | PDK4 | 5166 | 8812 | Reverse | 7 |
| PCEM.799_x _at | Fully Exonic | 6 | ENSG00000142515 | KLK3 | 354 | 6364 | Forward | 19 |
| PCHP.1147_ s_at | Fully Exonic | 11 | ENSG00000128016 | ZFP36 | 7538 | 12862 | Forward | 19 |
| PCHP.1153_ s_at | Fully Exonic | 11 | ENSG00000167900 | TK1 | 7083 | 11830 | Reverse | 17 |
| PCHP.120_s_at | Fully Exonic | 11 | ENSG00000147394 | ZNF185 | 7739 | 12976 | Forward | X |
| PCHP.1458_ s_at | Fully Exonic | 11 | ENSG00000007908 | SELE | 6401 | 10718 | Reverse | 1 |
| PCHP.1474_ s_at | Fully Exonic | 11 | ENSG00000106366 | SERPIN E1 | 5054 | 8583 | Forward | 7 |

| Probeset ID | Orientatio n | NoPA | ENSEMBL gene no. | Gene Symbol | Entrez Gene ID | HGNC symbol acc no | Strand | Csome no |
|---|---|---|---|---|---|---|---|---|
| PCHP.233_x _at | Fully Exonic | 7 | ENSG00000164611 | PTTG1 | 9232 | 9690 | Forward | 5 |
| PCHP.235_s_at | Fully Exonic | 11 | ENSG00000197635 | DPP4 | 1803 | 3009 | Reverse | 2 |
| PCHP.412_x _at | Fully Exonic | 11 | ENSG00000081041 | CXCL2 | 2920 | 4603 | Reverse | 4 |
| PCHP.43_s_a t | Fully Exonic | 11 | ENSG00000123975 | CKS2 | 1164 | 2000 | Forward | 9 |
| PCHP.560_s_at | Fully Exonic | 10 | ENSG00000146205 | ANO7 | 50636 | 31677 | Forward | 2 |
| PCHP.564_s_at | Fully Exonic | 11 | ENSG00000146205 | ANO7 | 50636 | 31677 | Forward | 2 |
| PCHP.604_x _at | Fully Exonic | 11 | ENSG00000142515 | KLK3 | 354 | 6364 | Forward | 19 |
| PCHP.651_s_at | Fully Exonic | 11 | ENSG00000101951 | PAGE4 | 9506 | 4108 | Forward | X |
| PCHP.785_s_at | Fully Exonic | 11 | ENSG00000142515 | KLK3 | 354 | 6364 | Forward | 19 |
| PCPD.14169 .C1_at | Includes Intronic | 11 | ENSG00000255240 | N/A | 28319 4 | NOVEL as | Reverse | 11 |
| PCPD.14169 .C1_x_at | Includes Intronic | 11 | ENSG00000255240 | N/A | 28319 4 | NOVEL as | Reverse | 11 |
| PCPD.1539. C1_s_at | Fully Exonic | 11 | ENSG00000266559 | MIR453 0 | 10061 6163 | 41764 | Reverse | 19 |
| PCPD.20005 .C1_at | Includes Intronic | 11 | ENSG00000255240 | N/A | 28319 4 | NOVEL as | Reverse | 11 |
| PCPD.20005 .C1_x_at | Includes Intronic | 9 | ENSG00000255240 | N/A | 28319 4 | NOVEL as | Reverse | 11 |
| PCPD.2281. C1_at | Includes Intronic | 6 | ENSG00000138166 | DUSP5 | 1847 | 3071 | Forward | 10 |
| PCPD.29484 .C1_at | Fully Exonic | 11 | ENSG00000004799 | PDK4 | 5166 | 8812 | Reverse | 7 |
| PCPD.3244. C1_s_at | Fully Exonic | 11 | ENSG00000125740 | FOSB | 2354 | 3797 | Forward | 19 |
| PCPD.3722. C1_s_at | Fully Exonic | 10 | ENSG00000104313 | EYA1 | 2138 | 3519 | Reverse | 8 |
| PCPD.39829 .C1_s_at | Fully Exonic | 11 | ENSG00000225986 | UBXN10 -AS1 | 10192 8017 | 41141 | Reverse | 1 |
| PCPD.5859. C2_at | Includes Intronic | 11 | ENSG00000198062 | POTEH | 23784 | 133 | Reverse | 22 |
| PCPD.5961. C1_at | Includes Intronic | 9 | ENSG00000255240 | N/A | 28319 4 | NOVEL as | Reverse | 11 |
| PCPD.7116. C1_at | Includes Intronic | 11 | ENSG00000125257 | ABCC4 | 10257 | 55 | Reverse | 13 |
| PCPD.7116. C1_x_at | Includes Intronic | 10 | ENSG00000125257 | ABCC4 | 10257 | 55 | Reverse | 13 |
| PCRS.626_x_at | Fully Exonic | 11 | ENSG00000198062 | POTEH | 23784 | 133 | Reverse | 22 |
| PCRS.812_s_at | Fully Exonic | 11 | ENSG00000196417 | ZNF765 | 91661 | 25092 | Forward | 19 |

(continued)

| Probeset ID | Orientatio n | NoPA | ENSEMBL gene no. | Gene Symbol | Entrez Gene ID | HGNC symbol acc no | Strand | Csome no |
|---|---|---|---|---|---|---|---|---|
| PCRS2.2880 _s_at | Fully Exonic | 10 | ENSG00000138166 | DUSP5 | 1847 | 3071 | Forward | 10 |
| PCRS2.3147 _x_at | Fully Exonic | 8 | ENSG00000230937 | MIR205 HG | 40698 8 | 43562 | Forward | 1 |
| PCRS2.4412 _s_at | Fully Exonic | 11 | ENSG00000146374 | RSPO3 | 84870 | 20866 | Forward | 6 |
| PCRS2.6477 _s_at | Fully Exonic | 11 | ENSG00000181195 | PENK | 5179 | 8831 | Reverse | 8 |
| PCRS2.7477 _s_at | Fully Exonic | 11 | ENSG00000189292 | FAM150 B | 28501 6 | 27683 | Reverse | 2 |
| PCRS3.3951 _at | Fully Exonic | 8 | ENSG00000205362 | MT1A | 4489 | 7393 | Forward | 16 |

NoPA - Number of probes aligned
Csome no - Chromosome number
NOVEL pc - novel protein coding (clone based vega gene)
NOVEL as - novel antisense (clone based vega gene)

Table 1 lists the sequence identifiers for the full sequences against which gene expression assays may be targeted, more specific target sequences and probes/probesets which hybridize to those target sequences. Suitable primers and/or probes may be designed using known methods to determine gene expression based on the deposited gene sequences, the full sequences and target sequences specified herein. Furthermore, specific nucleic acid amplification assays (e.g. PCR, such as qPCR) have also been designed that permit reliable determination of gene expression levels for the genes in table 1. These assays are summarized in Table 1B. The assay target sequence and primers and primer pairs form separate aspects of the invention. For two of the targets, MIR578 and MIR4530, due to the short length of the target sequences, the approach taken by the inventors was not applicable to generate an amplification assay. For those targets, commercial assays are available and the sequences of the primers are provided below. For MIR578, the Life Technologies 4426961 Origene HP300490 assay may be employed. The forward and reverse primers are as follows:

CTTCTTGTGCTCTAGGAT (SEQ ID NO: 3151)
GAACATGTCTGCGTATCTC (SEQ ID NO: 3152)

For MIR4530, the Life Technologies 4427012 Origene HP301022 assay may be employed. The forward and reverse primers are as follows:

CCCAGCAGGACGGGAGC (SEQ ID NO: 3153)
GAACATGTCTGCGTATCTC (SEQ ID NO: 3154) seems to be same as above

These specific primers, while useful in performing the methods of the invention, are thus not specifically claimed *per se* as forming part of the invention.

Table 1B - PCR assays designed for each of 70 genes in the signature

| Design Template used (Entrez Gene ID) | GeneBank ID | Gene Symbol | Assay ID | Exon spanning | Forward Primer ID | Forward primer SEQ ID NO | Forward Primer ABI TM | Reverse Primer ID | Reverse primer SEQ ID NO | Reverse Primer ABI TM |
|---|---|---|---|---|---|---|---|---|---|---|
| 827 | NM_014289.3 | CAPN6 | CAPN6_A1 | Yes | CAPN6_F1 | 3015 | 62.30 | CAPN6_R1 | 3083 | 60.78 |
| 7060 | NM_001306212.1 | THBS4 | THBS4_A1 | Yes | THL64_F1 | 3016 | 63.34 | THBS4_R1 | 3084 | 67.66 |
| 5354 | NM_000533.4 | PLP1 | PLP_A1 | Yes | PLP1_F1 | 3017 | 59.72 | PLP1_R1 | 3085 | 63.75 |
| 4489 | NM_005946.2 | MT1A | MT1A_A1 | Yes | MT1A_F1 | 3018 | 65.41 | MT1A_R1 | 3086 | 63.59 |
| 406988 | NR_029622.1 | MIR205HG | MIR205HG_A1 | Yes | MIR205HG_F1 | 3019 | 63.02 | MIR205HG_R1 | 3087 | 61.98 |
| 6406 | NM_003007.3 | SEMG1 | SEMG1_A1 | Yes | SEMG1_F1 | 3020 | 63.49 | SEMG1_R1 | 3088 | 63.59 |
| 84870 | NM_032784.4 | RSPO3 | RSP03_A1 | Yes | RSP03_F1 | 3021 | 61.24 | RSP03_R1 | 3089 | 63.13 |
| 50636 | NM_001001666.3 | ANO7 | AN07_A1 | Yes | ANO7_F1 | 3022 | 62.34 | ANO7_R1 | 3090 | 60.93 |
| 5121 | NM_006198.2 | PCP4 | PCP4_A1 | Yes | PCP4_F1 | 3023 | 60.53 | PCP4_R1 | 3091 | 61.70 |
| 27063 | NM_014391.2 | ANKRD1 | ANKRD1_A1 | Yes | ANKRD1_F1 | 3024 | 64.90 | ANKRD1_R1 | 3092 | 65.11 |
| 4604 | NM_001254718.1 | MYBPC1 | MYBPC1_A1 | Yes | MYBPC1_F1 | 3025 | 62.31 | MYBPC1_R1 | 3093 | 62.59 |
| 4316 | NM_002423.3 | MMP7 | MMP7_A1 | Yes | MMP7_F1 | 3026 | 53.80 | MMP7_R1 | 3094 | 48.86 |
| 12 | NM_001085.4 | SERPINA3 | SERPINA3_A1 | Yes | SERPINA3_F1 | 3027 | 60.39 | SERPINA3_R1 | 3095 | 62.07 |
| 6401 | NM_000450.2 | SELE | SELE_A1 | Yes | SELE_F1 | 3028 | 63.62 | SELE_R1 | 3096 | 62.56 |
| 3852 | NM_000424.3 | KRT5 | KRT5_A1 | Yes | KRT5_F1 | 3029 | 63.40 | KRT5_R1 | 3097 | 62.30 |
| 4057 | NM_001199149.1 | LTF | LTF_A1 | Yes | LTF_F1 | 3030 | 62.75 | LTF_R1 | 3098 | 64.08 |
| 57481 | NM_020721.1 | KIAA1210 | KIAA1210_A1 | Yes | KIAA1210_F1 | 3031 | 60.98 | KIAA1210_R1 | 3099 | 62.19 |
| 25907 | NM_015444.2 | TMEM158 | TMEM158_A1 | Yes | TMEM158_F1 | 3032 | 58.44 | TMEM158_R1 | 3100 | 62.20 |
| 7538 | NM_003407.3 | ZFP36 | ZFP36_A1 | Yes | ZFP36_F1 | 3033 | 63.26 | ZFP36_R1 | 3101 | 35.37 |
| 2354 | NM_001114171.1 | FOSB | FOSB_A1 | Yes | FOSB_F1 | 3034 | 61.04 | FOSB_R1 | 3102 | 62.16 |

| Design Template used (Entrez Gene ID) | GeneBank ID | Gene Symbol | Assay ID | Exon spanning | Forward Primer ID | Forward primer SEQ ID NO | Forward Primer ABI TM | Reverse Primer ID | Reverse primer SEQ ID NO | Reverse Primer ABI TM |
|---|---|---|---|---|---|---|---|---|---|---|
| 50652 | NR_015342.1 | PCA3 | PCA3_A1 | Yes | PCA3_F1 | 3035 | 62.83 | PCS3_R1 | 3103 | 61.36 |
| 79054 | NM_024080.4 | TRPM8 | TRPM8_A1 | Yes | TRPM8_F1 | 3036 | 61.89 | TRPM8_R1 | 3104 | 63.81 |
| 9232 | NM_001282382.1 | PTTG1 | PTTG1_A1 | No | PTTG1_F1 | 3037 | 60.97 | PTTG1_R1 | 3105 | 62.25 |
| 283194 | NR_033853.2 | LOC283194 | LOC283194_A1 | Yes | LOC288194_F1 | 3038 | 62.83 | LOC283194_R1 | 3106 | 61.36 |
| 9506 | NM_007003.3 | PAGE4 | PAGE4_A1 | Yes | PAGE4_F1 | 3039 | 61.09 | PAGE4_R1 | 3107 | 61.89 |
| 79689 | NM_001205315.1 | STEAP4 | STEAP4_A1 | Yes | STEAP4_F1 | 3040 | 64.22 | STEAP4_R1 | 3108 | 59.86 |
| 130733 | NM_001167959.1 | TMEM178A | TMEM178A_A1 | No | TMEM178A_F1 | 3041 | 70.52 | TMEM178A_R1 | 3109 | 59.86 |
| 2920 | NM_002089.3 | CXCL2 | CXCL2_A1 | Yes | CXCL2_F1 | 3042 | 62.60 | CXCL2_R1 | 3110 | 64.83 |
| 9955 | NM_006042.2 | HS3ST3A1 | HS3ST3A1_A1 | Yes | HS3ST3A1_F1 | 3043 | 61.52 | HS3ST3A1_R1 | 3111 | 62.80 |
| 2138 | NM_000503.5 | EYA1 | EYA1_A1 | Yes | EYA1_F1 | 3044 | 32.20 | EYA1_R1 | 3112 | 60.78 |
| 340419 | NM_001282863.1 | RSPO2 | RSPO2_A1 | Yes | RSPO2_F1 | 3045 | 64.91 | RSPO2_R1 | 3113 | 63.38 |
| 5317 | NM_000299.3 | PKP1 | PKP1_A1 | Yes | PKP1_F1 | 3046 | 60.55 | PKP1_R1 | 3114 | 63.39 |
| 4588 | NM_005961.2 | MUC6 | MUC6_A1 | Yes | MUC6_F1 | 3047 | 58.46 | MUC6_R1 | 3115 | 62.58 |
| 5179 | NM_001135690.1 | PENK | PENK_A1 | Yes | PENK_F1 | 3048 | 59 | PENK_R1 | 3116 | 58 |
| 1672 | NM_005218.3 | DEFB1 | DEFB1 | Yes | DEFB1_F1 | 3049 | 62.3 | DEFB1_R1 | 3117 | 62.1 |
| 84889 | NM_001048164.2 | SLC7A3 | SLC7A3_A1 | YES | SLC7A3 | 3050 | 60 | SLC7A3_R1 | 3118 | 59 |
| 693163 | NR_030304.1 | MIR578 | MIR578_A1 | No | MIR578_F1 | N/A | N/A | MIR578_R1 | N/A | N/A |
| 51050 | NM_015886.3 | PI15 | PI15_A1 | Yes | PI15_F1 | 3051 | 61.9 | PI15_R1 | 3119 | 62.1 |
| 101928017 | NR_110078.1 | UBXN10-AS1 | UBXB10-AS1_A1 | Yes | UBXB10-AS1_F1 | 3052 | 61.55 | UBXB10-AS1_R1 | 3120 | 61.42 |

| Design Tem-plate used (Entrez Gene ID) | GeneBank ID | Gene Symbol | Assay ID | Exon spanning | Forward Primer ID | Forward primer SEQ ID NO | Forward Primer ABI TM | Reverse Primer ID | Reverse primer SEQ ID NO | Reverse Primer ABI TM |
|---|---|---|---|---|---|---|---|---|---|---|
| 5166 | NM_002612.3 | PDK4 | PDK4_A1 | Yes | PDK4_F1 | 3053 | 62.00 | PDK4_R1 | 3121 | 61.90 |
| 644844 | NM_001145643.1 | PHGR1 | PHGR1_A1 | Yes | PHGR1_F1 | 3054 | 60.00 | PHGR1_R1 | 3122 | 59.00 |
| 5054 | NM_000602.4 | SERPINE1 | SERPINE1_A1 | Yes | SERPINE1_F1 | 3055 | 59.00 | SERPINE1_R1 | 3123 | 59.00 |
| 29951 | NM_001164595.1 | PDZRN4 | PDZRN4_A1 | Yes | PDZRN4_F1 | 3056 | 62 | PDZRN4_R1 | 3124 | 62.6 |
| 7739 | NM_001178106.1 | ZNF185 | ZNF185_A1 | Yes | ZNF185_F1 | 3057 | 63.92 | ZNF185_R1 | 3125 | 65.09 |
| 152 | NM_000683.3 | ADRA2C | ADRA2C_A1 | No | ADRA2C_F1 | 3058 | 61.8 | ADRA2C_R1 | 3126 | 61.4 |
| 563 | NM_001185.3 | AZGP1 | AZGP1_A1 | Yes | AZGP1_F1 | 3059 | 59.00 | AZGP1_R1 | 3127 | 59.00 |
| 7083 | NM_003258.4 | TK1 | TK1_A1 | Yes | TK1_F1 | 3060 | 61.8 | TK1_R1 | 3128 | 61.9 |
| 23784 | NM_001136213.1 | POTEH | POTEH_A1 | Yes | POTEH_F1 | 3061 | 62.4 | POTEH_R1 | 3129 | 62 |
| 3832 | NM_004523.3 | KIF11 | KIF11_A1 | Yes | KIF11 _F1 | 3062 | 60.00 | KIF11 _R1 | 3130 | 60.00 |
| 9076 | NM_021101.4 | CLDN1 | CLDN1_A1 | Yes | CLDN1_F1 | 3063 | 60.00 | CLDN1_R1 | 3131 | 59.00 |
| 100616163 | NR_039755.1 | MIR4530 | MIR4530_A1 | No | MIR4530_F1 | N/A | N/A | MIR4530_R1 | N/A | N/A |
| 23764 | NM_001161572.1 | MAFF | MAFF_A1 | Yes | MAFF_F1 | 3064 | 61.7 | MAFF_R1 | 3132 | 62.3 |
| 91661 | NM_001040185.1 | ZNF765 | ZNF765_A1 | Yes | ZNF765_F1 | 3065 | 62.1 | ZNF765_R1 | 3133 | 61.9 |
| 1164 | NM_001827.2 | CKS2 | CKS2_A1 | Yes | CKS2_F1 | 3066 | 59.00 | CKS2_R1 | 3134 | 59.00 |
| 56849 | NM_152278.3 | TCEAL7 | TCEAL7_A1 | Yes | TCEAL7 _F1 | 3067 | 59.00 | TCEAL7 _R1 | 3135 | 60.00 |
| 5346 | NM_001145311.1 | PLIN1 | PLIN1_A1 | Yes | PLIN1_F1 | 3068 | 62.2 | PLIN1_R1 | 3136 | 62.4 |
| 6614 | NM_023068.3 | SIGLEC1 | SIGLEC1_A1 | Yes | SIGLEC1_F1 | 3069 | 59.00 | SIGLEC1_R1 | 3137 | 60.00 |

| Design Template used (Entrez Gene ID) | GeneBank ID | Gene Symbol | Assay ID | Exon spanning | Forward Primer ID | Forward primer SEQ ID NO | Forward Primer ABI TM | Reverse Primer ID | Reverse primer SEQ ID NO | Reverse Primer ABI TM |
|---|---|---|---|---|---|---|---|---|---|---|
| 285016 | NM_001002919.2 | FAM150B | FAM150B_A1 | Yes | FAM150B_F1 | 3070 | 60.00 | FAM150B_R1 | 3138 | 59.00 |
| 8076 | NM_001297709.1 | MFAP5 | MFAP5_A1 | Yes | MFAP5_F1 | 3071 | 61.7 | MFAP5_R1 | 3139 | 62.2 |
| 6422 | NM_003012.4 | SFRP1 | SFRP1_A1 | **Yes** | SFRP1_F1 | 3072 | 62 | SFRP1_R1 | 3140 | 62.1 |
| 1847 | NM_004419.3 | DUSP5 | DUSP5_A1 | **Yes** | DUSP5_F1 | 3073 | 61.9 | DUSP5_R1 | 3141 | 61.7 |
| 57176 | NM_001167733.2 | VARS2 | VARS2_A1 | **Yes** | VARS2_F1 | 3074 | 62.1 | VARS2_R1 | 3142 | 61.8 |
| 10257 | NM_001105515.2 | ABCC4 | ABCC4_A1 | Yes | ABCC4_F1 | 3075 | 60.00 | ABCC4_R1 | 3143 | 60.00 |
| 23677 | NM_014521.2 | SH3BP4 | SH3BP4_A1 | Yes | SH3BP4_F1 | 3076 | 58.00 | SH3BP4_R1 | 3144 | 60.00 |
| 6652 | NM_003104.5 | SORD | SORD_A1 | Yes | SORD_F1 | 3077 | 60.00 | SORD_R1 | 3145 | 59.00 |
| 51001 | NM_001286643.1 | MTERFD1 | MTERFD1_A1 | Yes | MTERFD_F1 | 3078 | 59.00 | MTERFD1_R1 | 3146 | 60.00 |
| 1803 | XM_005246371.2 | DPP4 | DPP4_A1 | Yes | DPP4_F1 | 3079 | 60.00 | DPP4_R1 | 3147 | 59.00 |
| 284837 | NR_026961.1 | AATBC | AATBC_A1 | Yes | AATBC_F1 | 3080 | 61.99 | AATBC_R1 | 3148 | 62.42 |
| 54097 | NM_058186.3 | FAM3B | FAM3B_A1 | Yes | FAM3B_F1 | 3081 | 61.8 | FAM3B_R1 | 3149 | 62.2 |
| 354 | NM_001030047.1 | KLK3 | KLK3_A1 | Yes | KLK_F1 | 3082 | 59.00 | KLK3_R1 | 3150 | 59.00 |

It should be noted that the complement of each sequence described herein may be employed as appropriate (e.g. for designing hybridizing probes and/or primers, including primer pairs).

In certain embodiments the expression level of THBS4 and at least 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68 or 69 of the genes in table 1 is determined. Some analysis reported herein indicates that applying a signature comprising the measured expression levels of 7 or 12 genes can provide acceptable performance. Thus, in the invention, the minimum number of genes in the gene signature is 12. They comprise THBS4 and any 11 genes from the 70 genes.

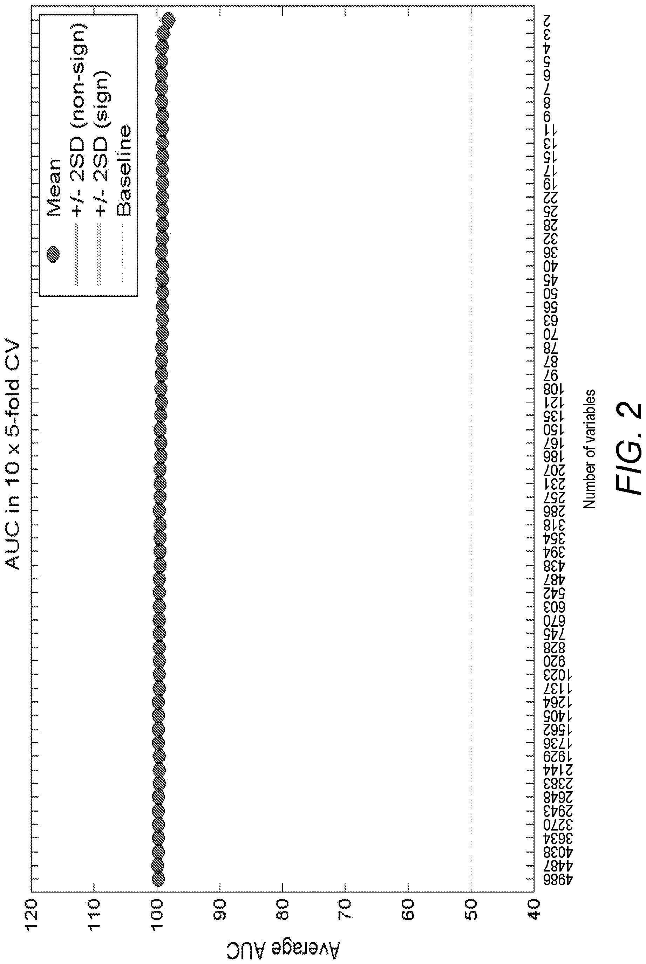

For the avoidance of doubt, additional genes (outside of the 70 genes) can be included in the signatures as would be readily appreciated by one skilled in the art. As is shown in figures 2 to 4, larger gene signatures are also potentially suitable.

In some embodiments, a signature score is derived from the measured expression levels of THBS4 and 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68 or 69 genes in table 1. Generation of such signature scores is described herein. The signature score may rely upon the weightings attributed to each gene as listed in Table 1, for the 70 gene signature. The weightings would, of course, need to be recalculated where a signature of different composition was utilized, for example including fewer than the total 70 gene signature. Similar considerations apply to the bias and constant offset values, as discussed below.

Gene signatures may be formulated in rank order, for example a 10 gene signature could be formed from the first 10 ranked genes listed in Table 1. However, the rankings are based on performance in the context of the 70 gene signature. Accordingly, formulation of sub-signatures of the 70 gene signature are not restricted to the same hierarchy and may be formulated using any combination of the 70 genes to form the suitably sized signature.

Core gene analysis was performed to determine a ranking for the genes based upon their impact on performance when removed from the signature. This analysis involved 10,000 random samplings of 10 signature genes from the original 70 signature gene set. For each iteration, 10 randomly selected signature genes were removed and the performance of the remaining 65 genes was evaluated using the endpoint to determine the impact on HR (Hazard Ratio) performance when these 10 genes were removed.

When this was performed using the FASTMAN Biopsy Validation Cohort of 248 samples, evaluation utilised the biochemical recurrence (BCR) endpoint.

The signature genes were weighted based upon the change in HR performance (Delta HR) based upon their inclusion or exclusion. The gene ranked '1' has the most negative impact on performance when removed and the gene ranked '70' has the least impact on performance when removed. The results are shown in Table 35 below.

Thus, in some embodiments, gene signatures are formulated in rank order. For example a 10 gene signature could comprise the first 10 ranked genes listed in Table 35. Accordingly, in some instances, the expression level of at least 1, 2, 3, 4, 5, 6, 7, 8, 9 or 10 of the 10 highest ranked genes in Table 35 is determined.

When this was performed using the Internal Resection Validation Cohort of 322 samples, evaluation utilised the metastatic recurrence (MET) endpoint.

The signature genes were weighted based upon the change in HR performance (Delta HR) based upon their inclusion or exclusion. The gene ranked '1' has the most negative impact on performance when removed and the gene ranked '70' has the least impact on performance when removed. The results are shown in Table 36 below.

Thus, in some embodiments, gene signatures are formulated in rank order. For example a 10 gene signature could comprise the first 10 ranked genes listed in Table 36. Accordingly, in some instances, the expression level of at least 1, 2, 3, 4, 5, 6, 7, 8, 9 or 10 of the 10 highest ranked genes in Table 36 is determined.

The results for combined rankings are shown in Table 38. In some embodiments, gene signatures are formulated in rank order. For example a 10 gene signature could comprise from the first 10 ranked genes listed in Table 38. Accordingly, in some instances, the expression level of at least 1, 2, 3, 4, 5, 6, 7, 8, 9 or 10 of the 10 highest ranked genes in Table 38 is determined.

Additional gene signatures representing selections from the genes of Table 1 are described herein and are applicable to all aspects of the invention. These signatures may also provide the basis for larger signatures. The additional signatures are set forth in Tables 2 to 24, together with suitable weight and bias scores that may be adopted when calculating the final signature score (as further described herein). The k value for each signature can be set once the threshold for defining a positive signature score has been determined, as would be readily appreciated by the skilled person. Similarly, the rankings for each gene in the signature can readily be determined by reviewing the weightings attributed to each gene (where a larger weight indicates a higher ranking in the signature - see Table 1 for the rank order in respect of the 70 gene signature).

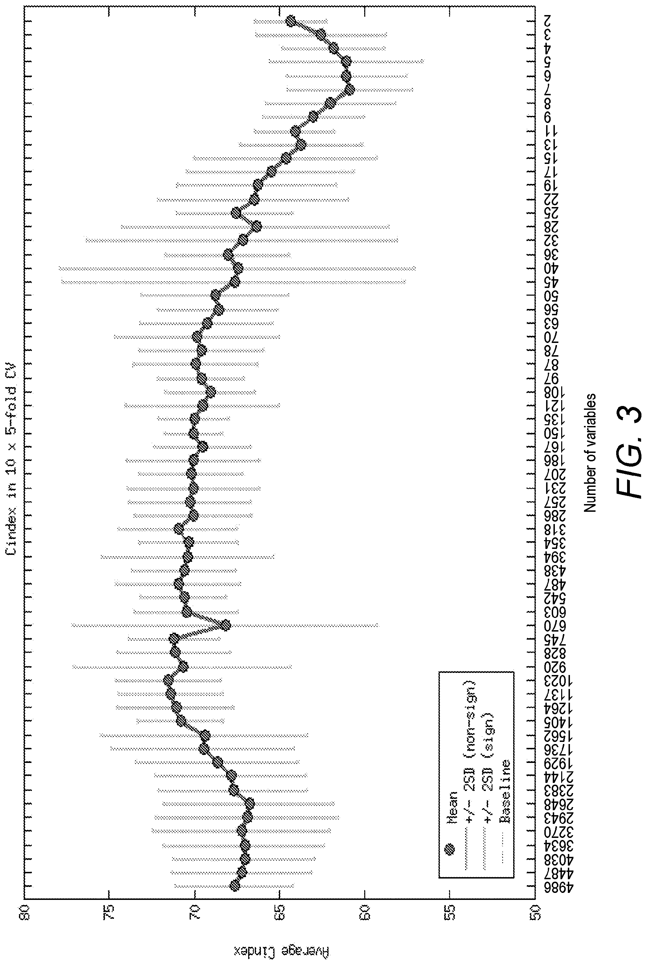

Thus, in some instances, the methods of the invention involve determining expression levels of at least MT1A and PCP4 (two gene signature shown in Table 2). As shown in Figures 2 and 3, signatures as small as the two gene signatures are capable of identifying the relevant biology and predicting metastatic recurrence. Larger signatures can be developed based upon these two genes, examples of which are given in tables 3 to 24, and in Table 1. Suitable probes and probsets to investigate expression of these genes are provided in Table 1 and 1A and primers useful to determine expression are listed in Table 1B.

Table 2 - Two gene signature

| Entrez Gene ID | Weight | Bias |
|---|---|---|
| 4489 | -0.0854336 | 6.74796 |
| 5121 | -0.0849287 | 7.62176 |

Table 3 - Three gene signature

| Entrez Gene ID | Weight | Bias |
|---|---|---|
| 406988 | -0.0584449 | 7.21525 |
| 4489 | -0.0594146 | 6.74796 |
| 5121 | -0.0590634 | 7.62176 |

Table 4 - Four gene signature

| Entrez Gene ID | Weight | Bias |
|---|---|---|
| 406988 | -0.0484829 | 7.21525 |
| 4489 | -0.0492874 | 6.74796 |
| 5121 | -0.0489961 | 7.62176 |
| 827 | -0.0438564 | 4.44087 |

Table 5 - Five gene signature

| Entrez Gene ID | Weight | Bias |
|---|---|---|
| 406988 | -0.0409374 | 7.21525 |
| 4489 | -0.0416166 | 6.74796 |
| 5121 | -0.0413707 | 7.62176 |
| 6401 | -0.0364515 | 5.97768 |
| 827 | -0.0370309 | 4.44087 |

Table 6 - Six gene signature

| Entrez Gene ID | Weight | Bias |
|---|---|---|
| 406988 | -0.0355221 | 7.21525 |
| 4489 | -0.0361114 | 6.74796 |
| 5121 | -0.035898 | 7.62176 |
| 5354 | -0.0309227 | 4.38357 |
| 6401 | -0.0316296 | 5.97768 |
| 827 | -0.0321323 | 4.44087 |

Table 7 - Seven gene signature

| Entrez Gene ID | Weight | Bias |
|---|---|---|
| 3852 | -0.026477 | 6.08049 |
| 406988 | -0.0314283 | 7.21525 |
| 4489 | -0.0319498 | 6.74796 |
| 5121 | -0.0317609 | 7.62176 |
| 5354 | -0.027359 | 4.38357 |
| 6401 | -0.0279844 | 5.97768 |
| 827 | -0.0284292 | 4.44087 |

Table 8 - Eight gene signature

| Entrez Gene ID | Weight | Bias |
|---|---|---|
| 3852 | -0.0240174 | 6.08049 |
| 406988 | -0.0285088 | 7.21525 |
| 4489 | -0.0289818 | 6.74796 |
| 5121 | -0.0288105 | 7.62176 |
| 5354 | -0.0248175 | 4.38357 |
| 57481 | -0.0223493 | 3.55997 |
| 6401 | -0.0253848 | 5.97768 |
| 827 | -0.0257883 | 4.44087 |

| Table 9 - Nine gene signature | | |
|---|---|---|
| Entrez Gene ID | Weight | Bias |
| 27063 | -0.0189187 | 5.92831 |
| 3852 | -0.022443 | 6.08049 |
| 406988 | -0.0266399 | 7.21525 |
| 4489 | -0.0270819 | 6.74796 |
| 5121 | -0.0269218 | 7.62176 |
| 5354 | -0.0231906 | 4.38357 |
| 57481 | -0.0208842 | 3.55997 |
| 6401 | -0.0237207 | 5.97768 |
| 827 | -0.0240977 | 4.44087 |

Table 10 - Eleven gene signature

| Entrez Gene ID | Weight | Bias |
|---|---|---|
| 25907 | -0.016386 | 8.06342 |
| 27063 | -0.0169106 | 5.92831 |
| 3852 | -0.0200608 | 6.08049 |

36

(continued)

| Entrez Gene ID | Weight | Bias |
|---|---|---|
| 406988 | -0.0238123 | 7.21525 |
| 4489 | -0.0242073 | 6.74796 |
| 5121 | -0.0240643 | 7.62176 |
| 5354 | -0.0207291 | 4.38357 |
| 57481 | -0.0186675 | 3.55997 |
| 6401 | -0.0212029 | 5.97768 |
| 827 | -0.0215399 | 4.44087 |
| 84870 | -0.0157681 | 4.29317 |

Table 11 - Thirteen gene signature

| Entrez Gene ID | Weight | Bias |
|---|---|---|
| 25907 | -0.0150652 | 8.06342 |
| 27063 | -0.0155475 | 5.92831 |
| 3852 | -0.0184438 | 6.08049 |
| 406988 | -0.0218928 | 7.21525 |
| 4489 | -0.0222561 | 6.74796 |
| 5121 | -0.0221245 | 7.62176 |
| 5354 | -0.0190581 | 4.38357 |
| 57481 | -0.0171628 | 3.55997 |
| 6401 | -0.0194938 | 5.97768 |
| 6406 | -0.0144896 | 4.23042 |
| 7060 | -0.0144516 | 6.91259 |
| 827 | -0.0198036 | 4.44087 |
| 84870 | -0.0144971 | 4.29317 |

Table 12 - Fifteen gene signature

| Entrez Gene ID | Weight | Bias |
|---|---|---|
| 2138 | -0.013038 | 5.50428 |
| 25907 | -0.0137554 | 8.06342 |
| 27063 | -0.0141957 | 5.92831 |
| 340419 | -0.0131822 | 3.92242 |
| 3852 | -0.0168402 | 6.08049 |
| 406988 | -0.0199894 | 7.21525 |
| 4489 | -0.020321 | 6.74796 |
| 5121 | -0.0202009 | 7.62176 |
| 5354 | -0.0174011 | 4.38357 |
| 57481 | -0.0156705 | 3.55997 |

(continued)

| Entrez Gene ID | Weight | Bias |
|---|---|---|
| 6401 | -0.0177989 | 5.97768 |
| 6406 | -0.0132298 | 4.23042 |
| 7060 | -0.0131951 | 6.91259 |
| 827 | -0.0180818 | 4.44087 |
| 84870 | -0.0132366 | 4.29317 |

Table 13 - Seventeen gene signature

| Entrez Gene ID | Weight | Bias |
|---|---|---|
| 2138 | -0.0122396 | 5.50428 |
| 2354 | -0.0114061 | 6.95494 |
| 25907 | -0.0129131 | 8.06342 |
| 27063 | -0.0133265 | 5.92831 |
| 340419 | -0.012375 | 3.92242 |
| 3852 | -0.015809 | 6.08049 |
| 4057 | -0.0113308 | 6.49726 |
| 406988 | -0.0187653 | 7.21525 |
| 4489 | -0.0190767 | 6.74796 |
| 5121 | -0.0189639 | 7.62176 |
| 5354 | -0.0163356 | 4.38357 |
| 57481 | -0.014711 | 3.55997 |
| 6401 | -0.0167091 | 5.97768 |
| 6406 | -0.0124197 | 4.23042 |
| 7060 | -0.0123871 | 6.91259 |
| 827 | -0.0169746 | 4.44087 |
| 84870 | -0.0124261 | 4.29317 |

Table 14 - Nineteen gene signature

| Entrez Gene ID | Weight | Bias |
|---|---|---|
| 12 | -0.0105382 | 5.74546 |
| 2138 | -0.011593 | 5.50428 |
| 2354 | -0.0108034 | 6.95494 |
| 25907 | -0.0122308 | 8.06342 |
| 27063 | -0.0126224 | 5.92831 |
| 340419 | -0.0117212 | 3.92242 |
| 3852 | -0.0149737 | 6.08049 |
| 4057 | -0.0107322 | 6.49726 |
| 406988 | -0.0177739 | 7.21525 |

(continued)

| Entrez Gene ID | Weight | Bias |
|---|---|---|
| 4489 | -0.0180688 | 6.74796 |
| 5121 | -0.017962 | 7.62176 |
| 5354 | -0.0154725 | 4.38357 |
| 57481 | -0.0139337 | 3.55997 |
| 6401 | -0.0158262 | 5.97768 |
| 6406 | -0.0117635 | 4.23042 |
| 7060 | -0.0117327 | 6.91259 |
| 7538 | -0.0101011 | 9.96083 |
| 827 | -0.0160778 | 4.44087 |
| 84870 | -0.0117696 | 4.29317 |

Table 15 - Twenty two gene signature

| Entrez Gene ID | Weight | Bias |
|---|---|---|
| 12 | -0.0102163 | 5.74546 |
| 2138 | -0.0112388 | 5.50428 |
| 2354 | -0.0104734 | 6.95494 |
| 25907 | -0.0118571 | 8.06342 |
| 27063 | -0.0122367 | 5.92831 |
| 340419 | -0.0113631 | 3.92242 |
| 3852 | -0.0145163 | 6.08049 |
| 4057 | -0.0104043 | 6.49726 |
| 406988 | -0.0172309 | 7.21525 |
| 4489 | -0.0175167 | 6.74796 |
| 4604 | -0.0069325 | 4.57432 |
| 50636 | -0.0064135 | 6.52255 |
| 5121 | -0.0174132 | 7.62176 |
| 5354 | -0.0149998 | 4.38357 |
| 57481 | -0.013508 | 3.55997 |
| 6401 | -0.0153427 | 5.97768 |
| 6406 | -0.0114041 | 4.23042 |
| 7060 | -0.0113742 | 6.91259 |
| 7538 | -0.0097925 | 9.96083 |
| 827 | -0.0155866 | 4.44087 |
| 84870 | -0.01141 | 4.29317 |
| 9232 | 0.00804755 | 4.71269 |

Table 16 - Twenty five gene signature

| Entrez Gene ID | Weight | Bias |
|---|---|---|
| 12 | -0.0101819 | 5.74546 |
| 2138 | -0.011201 | 5.50428 |
| 2354 | -0.0104381 | 6.95494 |
| 25907 | -0.0118172 | 8.06342 |
| 27063 | -0.0121956 | 5.92831 |
| 340419 | -0.0113249 | 3.92242 |
| 3852 | -0.0144674 | 6.08049 |
| 4057 | -0.0103693 | 6.49726 |
| 406988 | -0.0171729 | 7.21525 |
| 4489 | -0.0174578 | 6.74796 |
| 4604 | -0.0069091 | 4.57432 |
| 50636 | -0.0063919 | 6.52255 |
| 50652 | -0.0035123 | 5.26234 |
| 5121 | -0.0173546 | 7.62176 |
| 5354 | -0.0149493 | 4.38357 |
| 57481 | -0.0134626 | 3.55997 |
| 6401 | -0.0152911 | 5.97768 |
| 6406 | -0.0113657 | 4.23042 |
| 7060 | -0.0113359 | 6.91259 |
| 7538 | -0.0097595 | 9.96083 |
| 79054 | -0.0029055 | 4.86579 |
| 79689 | -0.0041936 | 8.1053 |
| 827 | -0.0155341 | 4.44087 |
| 84870 | -0.0113716 | 4.29317 |
| 9232 | 0.00802047 | 4.71269 |

Table 17 - Twenty eight gene signature

| Entrez Gene ID | Weight | Bias |
|---|---|---|
| 12 | -0.0113703 | 5.74546 |
| 2138 | -0.0102938 | 5.50428 |
| 2354 | -0.0091518 | 6.95494 |
| 25907 | -0.0112273 | 8.06342 |
| 27063 | -0.0109933 | 5.92831 |
| 2920 | -0.0080439 | 8.92898 |
| 340419 | -0.0103778 | 3.92242 |
| 3852 | -0.0118207 | 6.08049 |
| 4057 | -0.0105916 | 6.49726 |
| 406988 | -0.0163129 | 7.21525 |

(continued)

| Entrez Gene ID | Weight | Bias |
|---|---|---|
| 4489 | -0.0148319 | 6.74796 |
| 4604 | -0.0117356 | 4.57432 |
| 50636 | -0.0122781 | 6.52255 |
| 50652 | -0.0100098 | 5.26234 |
| 5121 | -0.0131977 | 7.62176 |
| 5354 | -0.0145474 | 4.38357 |
| 57481 | -0.0112327 | 3.55997 |
| 6401 | -0.0109283 | 5.97768 |
| 6406 | -0.0125967 | 4.23042 |
| 644844 | -0.008567 | 5.18357 |
| 693163 | -0.0087554 | 5.08739 |
| 7060 | -0.0156046 | 6.91259 |
| 7538 | -0.009639 | 9.96083 |
| 79054 | -0.0094113 | 4.86579 |
| 79689 | -0.0090982 | 8.1053 |
| 827 | -0.0185353 | 4.44087 |
| 84870 | -0.0120577 | 4.29317 |
| 9232 | 0.0102357 | 4.71269 |

Table 18 - Thirty two gene signature

| Entrez Gene ID | Weight | Bias |
|---|---|---|
| 12 | -0.010156 | 5.74546 |
| 2138 | -0.0084546 | 5.50428 |
| 2354 | -0.0105369 | 6.95494 |
| 25907 | -0.0093177 | 8.06342 |
| 27063 | -0.0095296 | 5.92831 |
| 2920 | -0.0082867 | 8.92898 |
| 340419 | -0.008292 | 3.92242 |
| 3852 | -0.0097028 | 6.08049 |
| 4057 | -0.0081905 | 6.49726 |
| 406988 | -0.0120927 | 7.21525 |
| 4316 | -0.0073912 | 6.75672 |
| 4489 | -0.012495 | 6.74796 |
| 4604 | -0.0121787 | 4.57432 |
| 50636 | -0.0122014 | 6.52255 |
| 50652 | -0.0102362 | 5.26234 |
| 5121 | -0.010326 | 7.62176 |

(continued)

| Entrez Gene ID | Weight | Bias |
|---|---|---|
| 5179 | -0.0077226 | 4.51486 |
| 5354 | -0.0133628 | 4.38357 |
| 57481 | -0.0095722 | 3.55997 |
| 6401 | -0.010634 | 5.97768 |
| 6406 | -0.0118163 | 4.23042 |
| 644844 | -0.0099334 | 5.18357 |
| 693163 | -0.0098705 | 5.08739 |
| 7060 | -0.0142594 | 6.91259 |
| 7538 | -0.0103042 | 9.96083 |
| 79054 | -0.0101624 | 4.86579 |
| 79689 | -0.0093796 | 8.1053 |
| 827 | -0.0166256 | 4.44087 |
| 84870 | -0.010646 | 4.29317 |
| 9232 | 0.00927419 | 4.71269 |
| 9506 | -0.008145 | 7.07391 |
| 9955 | -0.007857 | 4.23278 |

Table 19 - Thirty six gene signature

| Entrez Gene ID | Weight | Bias |
|---|---|---|
| 12 | -0.0093135 | 5.74546 |
| 130733 | -0.0075817 | 7.59453 |
| 2138 | -0.0084016 | 5.50428 |
| 2354 | -0.0099522 | 6.95494 |
| 25907 | -0.0091246 | 8.06342 |
| 27063 | -0.0096954 | 5.92831 |
| 283194 | -0.0076884 | 4.98038 |
| 2920 | -0.0082441 | 8.92898 |
| 340419 | -0.0081949 | 3.92242 |
| 3852 | -0.0098646 | 6.08049 |
| 4057 | -0.0080168 | 6.49726 |
| 406988 | -0.0121601 | 7.21525 |
| 4316 | -0.008168 | 6.75672 |
| 4489 | -0.0123296 | 6.74796 |
| 4604 | -0.0103293 | 4.57432 |
| 50636 | -0.0106303 | 6.52255 |
| 50652 | -0.008396 | 5.26234 |
| 51050 | -0.0074885 | 4.85872 |

(continued)

| Entrez Gene ID | Weight | Bias |
|---|---|---|
| 5121 | -0.0106667 | 7.62176 |
| 5179 | -0.0079247 | 4.51486 |
| 5317 | -0.0073104 | 5.91219 |
| 5354 | -0.012805 | 4.38357 |
| 57481 | -0.0094443 | 3.55997 |
| 6401 | -0.0105376 | 5.97768 |
| 6406 | -0.0117042 | 4.23042 |
| 644844 | -0.007735 | 5.18357 |
| 693163 | -0.0085964 | 5.08739 |
| 7060 | -0.0129938 | 6.91259 |
| 7538 | -0.009653 | 9.96083 |
| 79054 | -0.0084699 | 4.86579 |
| 79689 | -0.0078376 | 8.1053 |
| 827 | -0.0155276 | 4.44087 |
| 84870 | -0.0103741 | 4.29317 |
| 9232 | 0.00860486 | 4.71269 |
| 9506 | -0.0083385 | 7.07391 |
| 9955 | -0.0078923 | 4.23278 |

Table 20 - Forty gene signature

| Entrez Gene ID | Weight | Bias |
|---|---|---|
| 12 | -0.0088635 | 5.74546 |
| 130733 | -0.0073773 | 7.59453 |
| 2138 | -0.0081002 | 5.50428 |
| 2354 | -0.0089276 | 6.95494 |
| 23764 | -0.0070488 | 8.49795 |
| 25907 | -0.0086677 | 8.06342 |
| 27063 | -0.0091158 | 5.92831 |
| 283194 | -0.0077222 | 4.98038 |
| 2920 | -0.0074337 | 8.92898 |
| 340419 | -0.0079644 | 3.92242 |
| 3852 | -0.0093986 | 6.08049 |
| 4057 | -0.0076408 | 6.49726 |
| 406988 | -0.0117445 | 7.21525 |
| 4316 | -0.0078189 | 6.75672 |
| 4489 | -0.0117016 | 6.74796 |
| 4588 | -0.0072195 | 6.64004 |

(continued)

| Entrez Gene ID | Weight | Bias |
|---|---|---|
| 4604 | -0.0102513 | 4.57432 |
| 5054 | -0.007115 | 6.69187 |
| 50636 | -0.0102281 | 6.52255 |
| 50652 | -0.0081408 | 5.26234 |
| 51050 | -0.007475 | 4.85872 |
| 5121 | -0.0102856 | 7.62176 |
| 5179 | -0.0076867 | 4.51486 |
| 5317 | -0.0072532 | 5.91219 |
| 5354 | -0.0124218 | 4.38357 |
| 57481 | -0.0091711 | 3.55997 |
| 6401 | -0.0097774 | 5.97768 |
| 6406 | -0.0108845 | 4.23042 |
| 644844 | -0.0074985 | 5.18357 |
| 693163 | -0.0079773 | 5.08739 |
| 7060 | -0.012659 | 6.91259 |
| 7083 | 0.00689113 | 5.58133 |
| 7538 | -0.0089554 | 9.96083 |
| 79054 | -0.0080402 | 4.86579 |
| 79689 | -0.0074587 | 8.1053 |
| 827 | -0.0150968 | 4.44087 |
| 84870 | -0.0101513 | 4.29317 |
| 9232 | 0.00824867 | 4.71269 |
| 9506 | -0.0081624 | 7.07391 |
| 9955 | -0.0075526 | 4.23278 |

Table 21 - Forty five gene signature

| Entrez Gene ID | Weight | Bias |
|---|---|---|
| 12 | -0.0084719 | 5.74546 |
| 130733 | -0.0071653 | 7.59453 |
| 2138 | -0.0076354 | 5.50428 |
| 2354 | -0.0086978 | 6.95494 |
| 23764 | -0.0068137 | 8.49795 |
| 25907 | -0.0081883 | 8.06342 |
| 27063 | -0.0095258 | 5.92831 |
| 283194 | -0.0073756 | 4.98038 |
| 2920 | -0.0074016 | 8.92898 |
| 340419 | -0.0072676 | 3.92242 |

(continued)

| Entrez Gene ID | Weight | Bias |
|---|---|---|
| 3852 | -0.0086227 | 6.08049 |
| 4057 | -0.0076939 | 6.49726 |
| 406988 | -0.0109582 | 7.21525 |
| 4316 | -0.007433 | 6.75672 |
| 4489 | -0.0109596 | 6.74796 |
| 4588 | -0.0068952 | 6.64004 |
| 4604 | -0.0089751 | 4.57432 |
| 5054 | -0.0070642 | 6.69187 |
| 50636 | -0.0095383 | 6.52255 |
| 50652 | -0.0076953 | 5.26234 |
| 51050 | -0.0067347 | 4.85872 |
| 5121 | -0.0090383 | 7.62176 |
| 5166 | -0.0064467 | 4.17409 |
| 5179 | -0.0069808 | 4.51486 |
| 5317 | -0.0069448 | 5.91219 |
| 5354 | -0.0114369 | 4.38357 |
| 563 | -0.0062549 | 8.19118 |
| 57481 | -0.008131 | 3.55997 |
| 6401 | -0.0090862 | 5.97768 |
| 6406 | -0.0097387 | 4.23042 |
| 644844 | -0.0069075 | 5.18357 |
| 693163 | -0.007503 | 5.08739 |
| 7060 | -0.0117799 | 6.91259 |
| 7083 | 0.00695478 | 5.58133 |
| 7538 | -0.008409 | 9.96083 |
| 7739 | -0.0062004 | 6.90054 |
| 79054 | -0.0076792 | 4.86579 |
| 79689 | -0.0072917 | 8.1053 |
| 827 | -0.0138725 | 4.44087 |
| 84870 | -0.0094612 | 4.29317 |
| 84889 | -0.0067268 | 4.649 |
| 91661 | -0.0062403 | 3.97633 |
| 9232 | 0.00773594 | 4.71269 |
| 9506 | -0.0074141 | 7.07391 |
| 9955 | -0.0072818 | 4.23278 |

Table 22 - Fifty gene signature

| Entrez Gene ID | Weight | Bias |
|---|---|---|
| 100616163 | -0.0060146 | 10.5365 |
| 1164 | 0.00596174 | 6.50398 |
| 12 | -0.00788 | 5.74546 |
| 130733 | -0.0070582 | 7.59453 |
| 152 | -0.005916 | 7.07838 |
| 1672 | -0.0057271 | 6.82549 |
| 2138 | -0.0069005 | 5.50428 |
| 2354 | -0.0074259 | 6.95494 |
| 23764 | -0.0060195 | 8.49795 |
| 25907 | -0.0076929 | 8.06342 |
| 27063 | -0.0084041 | 5.92831 |
| 283194 | -0.0075818 | 4.98038 |
| 2920 | -0.0062969 | 8.92898 |
| 340419 | -0.006979 | 3.92242 |
| 3832 | 0.00580874 | 3.91767 |
| 3852 | -0.0073413 | 6.08049 |
| 4057 | -0.0068257 | 6.49726 |
| 406988 | -0.0093852 | 7.21525 |
| 4316 | -0.0070704 | 6.75672 |
| 4489 | -0.0103164 | 6.74796 |
| 4588 | -0.0065059 | 6.64004 |
| 4604 | -0.0088755 | 4.57432 |
| 5054 | -0.0064482 | 6.69187 |
| 50636 | -0.0093967 | 6.52255 |
| 50652 | -0.0078998 | 5.26234 |
| 51050 | -0.0064943 | 4.85872 |
| 5121 | -0.0085839 | 7.62176 |
| 5166 | -0.0061711 | 4.17409 |
| 5179 | -0.0066949 | 4.51486 |
| 5317 | -0.0069413 | 5.91219 |
| 5354 | -0.0110133 | 4.38357 |
| 563 | -0.0062503 | 8.19118 |
| 57481 | -0.0076625 | 3.55997 |
| 6401 | -0.0082619 | 5.97768 |
| 6406 | -0.0090315 | 4.23042 |
| 644844 | -0.0073783 | 5.18357 |
| 693163 | -0.0068836 | 5.08739 |
| 7060 | -0.012155 | 6.91259 |

(continued)

| Entrez Gene ID | Weight | Bias |
|---|---|---|
| 7083 | 0.00620598 | 5.58133 |
| 7538 | -0.0076694 | 9.96083 |
| 7739 | -0.0060281 | 6.90054 |
| 79054 | -0.0078154 | 4.86579 |
| 79689 | -0.0071002 | 8.1053 |
| 827 | -0.0134928 | 4.44087 |
| 84870 | -0.0091115 | 4.29317 |
| 84889 | -0.0067284 | 4.649 |
| 91661 | -0.0062814 | 3.97633 |
| 9232 | 0.00694781 | 4.71269 |
| 9506 | -0.0070319 | 7.07391 |
| 9955 | -0.0067662 | 4.23278 |

Table 23 - Fifty six gene signature

| Entrez Gene ID | Weight | Bias |
|---|---|---|
| 100616163 | -0.005861 | 10.5365 |
| 10257 | -0.0050496 | 5.23038 |
| 1164 | 0.00569625 | 6.50398 |
| 12 | -0.0073822 | 5.74546 |
| 130733 | -0.006436 | 7.59453 |
| 152 | -0.0058338 | 7.07838 |
| 1672 | -0.0055123 | 6.82549 |
| 2138 | -0.0068171 | 5.50428 |
| 2354 | -0.0071035 | 6.95494 |
| 23764 | -0.0056449 | 8.49795 |
| 23784 | -0.0055006 | 4.82498 |
| 25907 | -0.0075056 | 8.06342 |
| 27063 | -0.0082314 | 5.92831 |
| 283194 | -0.0066926 | 4.98038 |
| 2920 | -0.0062953 | 8.92898 |
| 340419 | -0.0068818 | 3.92242 |
| 3832 | 0.00560094 | 3.91767 |
| 3852 | -0.0072034 | 6.08049 |
| 4057 | -0.0066854 | 6.49726 |
| 406988 | -0.0090297 | 7.21525 |
| 4316 | -0.006866 | 6.75672 |
| 4489 | -0.0101527 | 6.74796 |

(continued)

| Entrez Gene ID | Weight | Bias |
|---|---|---|
| 4588 | -0.0062002 | 6.64004 |
| 4604 | -0.008045 | 4.57432 |
| 5054 | -0.0059681 | 6.69187 |
| 50636 | -0.008568 | 6.52255 |
| 50652 | -0.0069136 | 5.26234 |
| 51050 | -0.006074 | 4.85872 |
| 5121 | -0.0084668 | 7.62176 |
| 5166 | -0.0062193 | 4.17409 |
| 5179 | -0.0067401 | 4.51486 |
| 5317 | -0.0062775 | 5.91219 |
| 5346 | 0.00544079 | 4.62939 |
| 5354 | -0.0107509 | 4.38357 |
| 563 | -0.0057774 | 8.19118 |
| 57176 | 0.0054321 | 5.22346 |
| 57481 | -0.0075962 | 3.55997 |
| 6401 | -0.0079086 | 5.97768 |
| 6406 | -0.0089768 | 4.23042 |
| 644844 | -0.0063947 | 5.18357 |
| 6614 | 0.00529568 | 5.50375 |
| 693163 | -0.0062258 | 5.08739 |
| 7060 | -0.0113086 | 6.91259 |
| 7083 | 0.00606898 | 5.58133 |
| 7538 | -0.0073458 | 9.96083 |
| 7739 | -0.0059453 | 6.90054 |
| 79054 | -0.0069339 | 4.86579 |
| 79689 | -0.0063605 | 8.1053 |
| 827 | -0.0130713 | 4.44087 |
| 84870 | -0.0092604 | 4.29317 |
| 84889 | -0.0064006 | 4.649 |
| 9076 | -0.0053751 | 4.96028 |
| 91661 | -0.0056536 | 3.97633 |
| 9232 | 0.00664308 | 4.71269 |
| 9506 | -0.0069717 | 7.07391 |
| 9955 | -0.0067533 | 4.23278 |

Table 24 - Sixty three gene signature

| Entrez Gene ID | Weight | Bias |
|---|---|---|
| 100616163 | -0.005042 | 10.5365 |
| 101928017 | -0.0048527 | 6.06588 |
| 10257 | -0.0056574 | 5.23038 |
| 1164 | 0.0052823 | 6.50398 |
| 12 | -0.0073342 | 5.74546 |
| 130733 | -0.0062765 | 7.59453 |
| 152 | -0.0051502 | 7.07838 |
| 1672 | -0.0052785 | 6.82549 |
| 1847 | -0.0048311 | 5.76268 |
| 2138 | -0.0056248 | 5.50428 |
| 2354 | -0.0064848 | 6.95494 |
| 23764 | -0.0051811 | 8.49795 |
| 23784 | -0.0058458 | 4.82498 |
| 25907 | -0.0062868 | 8.06342 |
| 27063 | -0.0071516 | 5.92831 |
| 283194 | -0.0071346 | 4.98038 |
| 285016 | -0.0045118 | 6.6646 |
| 2920 | -0.0056286 | 8.92898 |
| 29951 | -0.0049994 | 4.75233 |
| 340419 | -0.0056458 | 3.92242 |
| 3832 | 0.00505389 | 3.91767 |
| 3852 | -0.0064458 | 6.08049 |
| 4057 | -0.0063934 | 6.49726 |
| 406988 | -0.0083826 | 7.21525 |
| 4316 | -0.0069549 | 6.75672 |
| 4489 | -0.0087025 | 6.74796 |
| 4588 | -0.0062676 | 6.64004 |
| 4604 | -0.0080954 | 4.57432 |
| 5054 | -0.0056402 | 6.69187 |
| 50636 | -0.0080538 | 6.52255 |
| 50652 | -0.0072374 | 5.26234 |
| 51050 | -0.0056617 | 4.85872 |
| 5121 | -0.0071957 | 7.62176 |
| 5166 | -0.0052681 | 4.17409 |
| 5179 | -0.0052589 | 4.51486 |
| 5317 | -0.0062761 | 5.91219 |
| 5346 | 0.00537235 | 4.62939 |
| 5354 | -0.009133 | 4.38357 |

(continued)

| Entrez Gene ID | Weight | Bias |
|---|---|---|
| 563 | -0.0057921 | 8.19118 |
| 56849 | -0.0048508 | 4.81933 |
| 57176 | 0.00516736 | 5.22346 |
| 57481 | -0.0063163 | 3.55997 |
| 6401 | -0.0069775 | 5.97768 |
| 6406 | -0.0081782 | 4.23042 |
| 6422 | -0.0048345 | 7.90126 |
| 644844 | -0.0064333 | 5.18357 |
| 6614 | 0.00520155 | 5.50375 |
| 693163 | -0.0060983 | 5.08739 |
| 7060 | -0.0108538 | 6.91259 |
| 7083 | 0.00523833 | 5.58133 |
| 7538 | -0.0065682 | 9.96083 |
| 7739 | -0.0050779 | 6.90054 |
| 79054 | -0.0071048 | 4.86579 |
| 79689 | -0.0063567 | 8.1053 |
| 8076 | -0.0047141 | 4.12918 |
| 827 | -0.011285 | 4.44087 |
| 84870 | -0.0075344 | 4.29317 |
| 84889 | -0.0058044 | 4.649 |
| 9076 | -0.0052058 | 4.96028 |
| 91661 | -0.0054622 | 3.97633 |
| 9232 | 0.00626422 | 4.71269 |
| 9506 | -0.0058269 | 7.07391 |
| 9955 | -0.0055209 | 4.23278 |

In the invention, the expression level of PDK4 alone is not measured. PDK4 expression may be measured in combination with THBS4 and at least 10 further genes up to all 68 further genes from table 1. In some embodiments, PDK4 expression is determined using an assay targeting a sequence within the full sequences of SEQ ID NO: 52, 53, 63, 108, 09, 152, 153, 157, 158, 184, 194 and/or 216 respectively. In some embodiments, PDK4 expression is determined using an assay targeting a sequence within the target sequences of SEQ ID NO: 284, 285, 295, 340, 341, 384, 385, 389, 390, 416, 426 and/or 448 respectively. In some embodiments PDK4 expression is determined using one or more probes selected from SEQ ID Nos: 1011-1021, 1022-1032, 1132-1142, 1627-1637, 1638-1648, 2122-2132, 2133-2143, 2177-2187, 2188-2198, 2474-2484, 2584-2594 and 2834-2844 or probe sets of SEQ ID Nos: 1011-1021, 1022-1032, 1132-1142, 1627-1637, 1638-1648, 2122-2132, 2133-2143, 2177-2187, 2188-2198, 2474-2484, 2584-2594 and/or 2834-2844. In some embodiments, PDK4 expression is determined using an amplification (PCR, or qPCR) assay employing primers of SEQ ID NO: 3053 and/or 3121 respectively.

In the invention, the expression level of KIF11, PTTG1 or TK1 alone is not measured. In some instances, the expression levels of KIF11, PTTG1 and TK1 may be measured together as a 3 gene signature. In some embodiments, the expression levels of KIF11, PTTG1 and/or TK1 may be measured in combination with THBS4 and further genes from Table 1, to form at least a 12 gene signature including forming the 70 gene signature. In some embodiments, KIF11 expression is determined using an assay targeting a sequence within the full sequences of SEQ ID NO: 180 and/or 181 respectively. In some embodiments, KIF11 expression is determined using an assay targeting a sequence within the target sequences of SEQ ID NO: 412 and/or 413 respectively. In some embodiments KIF11 expression is determined using one or more probes selected from SEQ ID Nos: 2430 -2440 and 2441-2451 or probe sets of SEQ ID Nos: 2430 -2440 and/or 2441- 2451. In some embodiments, KIF11 expression is determined using an amplification (PCR, or qPCR) assay employing primers of SEQ ID NO: 3062 and/or 3130 respectively.

In some embodiments, PTTG1 expression is determined using an assay targeting a sequence within the full sequences of SEQ ID NO: 62 and/or 201 respectively. In some embodiments, PTTG1 expression is determined using an assay targeting a sequence within the target sequences of SEQ ID NO: 294 and/or 433 respectively. In some embodiments PTTG1 expression is determined using one or more probes selected from SEQ ID Nos: 1121 -1131 and 2661-2671 or probe sets of SEQ ID Nos: 1121-1131 and/or 2661-2671. In some embodiments, PTTG1 expression is determined using an amplification (PCR, or qPCR) assay employing primers of SEQ ID NO: 3037 and/or 3105 respectively.

In some embodiments, TK1 expression is determined using an assay targeting a sequence within the full sequence of SEQ ID NO: 197. In some embodiments, TK1 expression is determined using an assay targeting a sequence within the target sequence of SEQ ID NO: 429. In some embodiments TK1 expression is determined using one or more probes selected from SEQ ID Nos: 2617- 2627 or probe sets of SEQ ID Nos: 2617- 2627. In some embodiments, TK1 expression is determined using an amplification (PCR, or qPCR) assay employing primers of SEQ ID NO: 3060 and/or 3128 respectively.

In the invention, the expression level of ANO7 or MYBPC1 alone is not measured. In some instances, the expression levels of ANO7 and MYBPC1 may be measured together as a 2 gene signature. In some embodiments, the expression levels of ANO7 and/or MYBPC1 may be measured in combination with THBS4 and further genes from Table 1, to form at least a 12 gene signature including forming the 70 gene signature.

In some embodiments, ANO7 expression is determined using an assay targeting a sequence within the full sequences of SEQ ID NO: 37, 38, 125, 205 and/or 206 respectively. In some embodiments, ANO7 expression is determined using an assay targeting a sequence within the target sequences of SEQ ID NO: 269, 270, 357, 437 and/or 438 respectively. In some embodiments ANO7 expression is determined using one or more probes selected from SEQ ID Nos: 849-859, 860-870, 1825-1835, 2715-2724 and 2725-2735 or probe sets of SEQ ID Nos: 849-859, 860-870, 1825-1835, 2715-2724 and/or 2725-2735. In some embodiments, ANO7 expression is determined using an amplification (PCR, or qPCR) assay employing primers of SEQ ID NO: 3022 and/or 3090 respectively.

In some embodiments, MYBPC1 expression is determined using an assay targeting a sequence within the full sequences of SEQ ID NO: 39, 40, 74, 75, 101, 102, 103 and/or 144 respectively. In some embodiments, MYBPC1 expression is determined using an assay targeting a sequence within the target sequences of SEQ ID NO: 271, 272, 306, 307, 333, 334, 335 and/or 376 respectively. In some embodiments MYBPC1 expression is determined using one or more probes selected from SEQ ID Nos: 871-881, 882-892, 1253-1263, 1264-1274, 1550-1560, 1561-1571, 1572-1582 and 2034- 2044 or probe sets of SEQ ID Nos: 871-881, 882-892, 1253-1263, 1264-1274, 1550-1560, 1561-1571, 1572-1582 and/or 2034-2044.

In some embodiments, MYBPC1 expression is determined using an amplification (PCR, or qPCR) assay employing primers of SEQ ID NO: 3025 and/or 3093 respectively.

By "characterization" is meant classification and/or evaluation of the cancer, such as prostate cancer or ER positive breast cancer. Thus, the methods of the invention allow cancers with high metastatic potential to be identified for example. The methods rely upon determining whether the cancer is a metastatic biology cancer or a non-metastatic biology cancer. The methods permit cancers to be identified that are likely to recur. Prognosis refers to predicting the likely outcome of the cancer, such as prostate cancer or ER positive breast cancer for the subject. A bad or poor prognosis as determined herein, indicates an increased likelihood of metastases and/or a higher likelihood or recurrence. By diagnosis is meant identifying the presence of a cancer, of a particular type such as prostate cancer or ER positive breast cancer with an increased metastatic potential. Thus, it will be readily apparent that there is some overlap between the terms "characterization", "prognosis" and "diagnosis" as adopted herein. The use of relative terms indicates the position *vis a vis* cancers which do not display the relevant gene expression characteristics and thus have lower metastatic potential, are less likely to recur and/or have a good prognosis. The gene signatures described herein may be useful to stratify (prostate) cancer patients who have been diagnosed, in particular at an early stage, and identify those at increased risk of developing more aggressive high risk disease. This more aggressive disease may develop within 3-5 years of treatment. The initial treatment may be radiotherapy and/or surgery (prostatectomy) for example. Upon identification of the aggressive disease, the methods may require treatments as described herein to be utilized. In the absence of cancer with high metastatic potential, the subject may be placed under active surveillance and not further treated, at least initially. Further monitoring, by any suitable means (including use of PSA monitoring or by performing the methods of the invention) can be used to determine whether further intervention is required.

In some embodiments the characterisation of and/or prognosis for the cancer, such as prostate cancer or ER positive breast cancer may comprise, consist essentially of or consist of predicting an increased likelihood of recurrence. Cancers with the metastatic biology are shown herein to be more likely to recur. The characterisation of and/or prognosis for the cancer, such as prostate cancer or ER positive breast cancer may comprise, consist essentially of or consist of predicting a reduced time to recurrence. Recurrence may be considered co-terminus with relapse, as would be understood by the skilled person.

Recurrence may be clinical recurrence, metastatic recurrence or biochemical recurrence. In the context of prostate cancer biochemical recurrence means a rise in the level of PSA in a subject after treatment for prostate cancer. Biochemical recurrence may indicate that the prostate cancer has not been treated effectively or has recurred. Recurrence may be following surgery, for example radical prostatectomy and/or following radiotherapy.

In some embodiments, the characterisation of and/or prognosis for the cancer, such as prostate cancer or ER positive breast cancer may comprise, consist essentially of or consist of predicting an increased likelihood of metastasis. Metastasis, or metastatic disease, is the spread of a cancer from one organ or part to another non-adjacent organ or part. The new occurrences of disease thus generated are referred to as metastases. In certain embodiments, the methods of the invention are used to facilitate metastases staging of cancer, in particular prostate cancer. Thus, determined expression levels (e.g. determination of a gene signature positive sample) can be used to stage a subject as M1. M1 means that metastases are present (i.e. the cancer has spread to other parts of the body). For gene signature negative samples, that subject may be staged as M0. M0 means that the cancer has not yet spread to other parts of the body. Such methods may be used in conjunction with other measures used to identify metastases e.g. imaging/scanning techniques. Thus, the invention provides a method for metastases staging of a cancer comprising determining the expression level of at least one gene selected from Table 1 in a sample from the subject wherein the determined expression level is used to identify whether a subject has a M1 or M0 cancer. Thus, in some embodiments, the methods may comprise:

(i) determining the expression level of THBS4 and at least 11 genes selected from Table 1 in a sample from the subject; and

(ii) assessing from the expression level the genes whether the sample from the subject is positive or negative for a gene signature comprising the genes. Suitable gene signatures and derivations of signature scores are discussed in further detail herein.

In some embodiments, characterisation of and/or prognosis for the cancer, such as prostate cancer or ER positive breast cancer may also comprise, consist essentially of or consist of determining whether the cancer has a poor prognosis. A poor prognosis may be a reduced likelihood of cause-specific, i.e. cancer-specific, or long term survival. Cause- or Cancer-specific survival is a net survival measure representing cancer survival in the absence of other causes of death. Cancer survival may be for 6, 7, 8, 9, 10, 11, 12 months or 1, 2, 3, 4, 5 etc. years. Long-term survival may be survival for 1 year, 5 years, 10 years or 20 years following diagnosis. A cancer, such as prostate cancer or ER positive breast cancer with a poor prognosis may be aggressive, fast growing, and/or show resistance to treatment.

In certain embodiments an increased expression level of at least one gene selected from Table 1 with a positive weight indicates an increased likelihood of recurrence and/or metastasis and/or a poor prognosis.

In further embodiments a decreased expression level of at least one gene selected from Table 1 with a negative weight indicates an increased likelihood of recurrence and/or metastasis and/or a poor prognosis.

Expression levels are weighted accordingly, to account for their contribution to gene signature score as discussed herein. A threshold of expression may be set relative to a median level against which "signature positive" and "signature negative" expression values can be set. Examples of such median threshold expression levels and corresponding signature positive and negative values are set forth in table 25 immediately below. As can be seen, the median values are set individually for each dataset as would be understood by one skilled in the art:

Table 25 - Median threshold expression levels for genes in 70 gene signature

| Gene Name | R0185 | | | Taylor | | | Clinical Validation | | |
|---|---|---|---|---|---|---|---|---|---|
| | Up/Down Regulation | | | Up/Down Regulation | | | Up/Down Regulation | | |
| | Median Threshold | Sig Pos | Sig Neg | Median Threshold | Sig Pos | Sig Neg | Median Threshold | Sig Pos | Sig Neg |
| CAPN6 | 4.42188 | 2.04472 | 6.43372 | 5.5318 | 5.3482 | 5.6302 | 6.315475 | 4.074 | 6.559 |
| THBS4 | 7.06852 | 5.02893 | 8.08507 | 6.09006 | 5.6854 | 6.2519 | 8.91341 | 8.7459 | 8.9505 |
| PLP1 | 4.5448 | 2.06305 | 6.49898 | 4.31333 | 4.3854 | 4.2517 | 3.456275 | 2.4345 | 3.7365 |
| MT1A | 6.387205 | 4.06229 | 8.97844 | 4.93781 | 4.5807 | 5.1455 | 6.518785 | 5.6427 | 6.7175 |
| MIR205HG | 8.00701 | 4.87658 | 9.24825 | 7.57876 | 7.1084 | 7.8151 | 8.97736 | 7.025 | 9.2159 |
| SEMG1 | 2.69399 | 2.3506 | 4.17395 | 3.37923 | 3.5178 | 3.2859 | 2.69531 | 2.6214 | 2.6953 |
| RSPO3 | 4.82032 | 2.0699 | 5.78781 | 8.8968 | 8.8373 | 8.9397 | 4.2128 | 2.2819 | 4.5188 |
| ANO7 | 6.46441 | 5.67131 | 7.44695 | 8.4678 | 8.3131 | 8.5449 | 8.683835 | 7.5313 | 8.7909 |
| PCP4 | 8.503335 | 5.4613 | 9.81501 | 7.95265 | 7.4887 | 8.2149 | 10.01705 | 8.9437 | 10.12 |
| ANKRD1 | 5.610625 | 3.90673 | 7.45987 | 4.25165 | 4.0009 | 4.3893 | 5.15809 | 3.17 | 5.6713 |
| MYBPC1 | 4.45984 | 2.87008 | 5.58119 | 3.16997 | 3.027 | 3.2647 | 6.173885 | 5.0181 | 6.3699 |
| MMP7 | 7.64552 | 3.63728 | 8.81375 | 2.21155 | 2.2597 | 2.1786 | 8.26743 | 6.6757 | 8.4475 |
| SERPINA3 | 5.8349 | 4.17103 | 6.62491 | 8.08507 | 5.9558 | 8.9948 | 6.869015 | 5.3198 | 7.0793 |
| SELE | 6.69364 | 3.42413 | 7.66659 | 4.86743 | 4.5184 | 5.0608 | 5.46303 | 4.3339 | 5.704 |
| KRT5 | 6.719415 | 3.43284 | 7.9083 | 8.22671 | 8.2267 | 8.2313 | 7.707815 | 6.5433 | 7.9614 |
| LTF | 5.83487 | 5.06191 | 7.70167 | 4.45153 | 4.174 | 4.5963 | 7.3738 | 6.3697 | 7.7314 |
| KIAA1210 | 2.74592 | 1.56824 | 5.50166 | 4.76043 | 4.9023 | 4.6617 | 4.578835 | 2.6833 | 4.7082 |
| TMEM158 | 8.40747 | 6.66104 | 9.3172 | 8.39763 | 8.1777 | 8.4845 | 7.655895 | 6.768 | 7.7878 |
| ZFP36 | 10.39315 | 8.80059 | 11.1231 | 9.73981 | 8.5152 | 10.592 | 10.6163 | 9.1513 | 10.895 |
| FOSB | 7.316875 | 5.1803 | 8.05011 | 8.35888 | 7.219 | 9.0206 | 7.957285 | 5.6257 | 8.6746 |
| PCA3 | 4.782625 | 4.3872 | 4.90232 | 11.4346 | 10.271 | 12.114 | 8.352805 | 8.0957 | 8.3847 |
| TRPM8 | 4.860835 | 4.0207 | 5.13583 | 4.78668 | 4.5937 | 4.9832 | 6.09048 | 6.1888 | 6.0901 |
| PTTG1 | 4.38243 | 5.40862 | 3.73654 | 3.05421 | 2.9145 | 3.135 | 3.73654 | 4.0886 | 3.6952 |

| Gene Name | R0185 Up/Down Regulation | | | Taylor Up/Down Regulation | | | Clinical Validation Up/Down Regulation | | |
|---|---|---|---|---|---|---|---|---|---|
| | Median Threshold | Sig Pos | Sig Neg | Median Threshold | Sig Pos | Sig Neg | Median Threshold | Sig Pos | Sig Neg |
| #N/A | 4.87794 | 4.92985 | 4.85895 | 6.1573 | 5.7808 | 6.5764 | 6.20071 | 6.1789 | 6.2063 |
| PAGE4 | 7.78752 | 4.79959 | 8.60591 | 5.2044 | 5.1045 | 5.3075 | 7.20806 | 5.2471 | 7.3508 |
| STEAP4 | 8.12307 | 7.29677 | 8.41974 | 3.26122 | 3.2612 | 3.2423 | 10.4898 | 10.657 | 10.466 |
| TMEM178A | 7.314555 | 6.61022 | 7.5254 | 4.57785 | 4.5071 | 4.5939 | 8.681645 | 8.4749 | 8.7561 |
| CXCL2 | 9.261335 | 7.34194 | 10.048 | 9.24825 | 9.0011 | 9.4489 | 8.75985 | 7.2643 | 9.0269 |
| HS3SBA1 | 4.45439 | 2.69531 | 5.32664 | 4.82805 | 4.9046 | 4.6609 | 5.18552 | 4.3254 | 5.3928 |
| EYA1 | 6.07141 | 3.60874 | 6.91569 | 4.19606 | 4.1531 | 4.2517 | 5.809395 | 4.6238 | 5.9532 |
| RSPO2 | 3.84235 | 1.98492 | 5.30295 | 2.61807 | 2.5402 | 2.6731 | 2.76883 | 2.1794 | 2.9415 |
| PKP1 | 6.112415 | 5.26861 | 6.34026 | 4.61452 | 4.3254 | 4.7781 | 5.22822 | 4.7867 | 5.2662 |
| MUC6 | 6.01117 | 5.96861 | 6.05794 | 8.69215 | 8.7469 | 8.582 | 6.73111 | 6.5614 | 6.7738 |
| PENK | 4.0716 | 2.34573 | 6.28444 | 8.74017 | 8.8199 | 8.6943 | 2.810335 | 2.5609 | 2.8701 |
| DEFB1 | 7.25831 | 4.86935 | 8.44625 | 6.346 | 5.9395 | 6.5493 | 6.238925 | 3.5331 | 6.7243 |
| SLC7A3 | 4.517555 | 3.83265 | 5.12394 | 3.06899 | 29415 | 3.1712 | 5.131285 | 4.6388 | 5.2528 |
| MIR578 | 5.23268 | 4.15688 | 5.74198 | 3.60874 | 3.3985 | 3.7449 | 3.83251 | 3.0482 | 4.0207 |
| PI15 | 5.175905 | 3.18336 | 5.8754 | 9.11409 | 7.7045 | 9.9628 | 6.06872 | 4.8925 | 6.2305 |
| UBXN10-AS1 | 6.333035 | 3.50707 | 7.96714 | 5.06221 | 4.6847 | 5.2619 | 5.20983 | 3.7088 | 5.5369 |
| PDK4 | 3.907115 | 2.34383 | 5.47102 | 3.16997 | 3.2654 | 3.1022 | 4.05588 | 3.1565 | 4.2233 |
| PHGR1 | 4.83498 | 4.68471 | 4.91059 | 4.07399 | 4.074 | 4068 | 7.31838 | 6.8104 | 7.4198 |
| SERPINE1 | 6.748165 | 5.89172 | 7.29677 | 4.57785 | 4.7107 | 4.3841 | 6.454425 | 5.8998 | 6.6472 |
| PDZRN4 | 5.065115 | 2.79653 | 6.28318 | 9.92587 | 10.04 | 9.8607 | 4.384745 | 2.7757 | 4.6154 |
| ZNF185 | 7.015235 | 5.24706 | 8.18067 | 5.24706 | 5.2471 | 5.2477 | 6.330095 | 5.767 | 6.3871 |
| ADRA2C | 7.300155 | 5.78671 | 7.99285 | 7.68072 | 7.7405 | 7.6252 | 6.58485 | 6.2063 | 6.6863 |
| AZGP1 | 8.64502 | 6.63277 | 9.1771 | 7.2166 | 6.5614 | 7.6067 | 8.821125 | 7.4957 | 9.031 |

| Gene Name | R0185 Up/Down Regulation | | | Taylor Up/Down Regulation | | | Clinical Validation Up/Down Regulation | | |
|---|---|---|---|---|---|---|---|---|---|
| | Median Threshold | Sig Pos | Sig Neg | Median Threshold | Sig Pos | Sig Neg | Median Threshold | Sig Pos | Sig Neg |
| TK1 | 5.12958 | 6.43788 | 4.55892 | 7.33302 | 7.5376 | 7.2099 | 4.209675 | 4.4515 | 4.1974 |
| POTEH | 5.033025 | 4.68471 | 5.41636 | 3.49675 | 3.3158 | 3.6403 | 4.387175 | 4.3664 | 4.3872 |
| KIF11 | 3.77959 | 5.07156 | 3.16997 | 3.38809 | 3.5827 | 3.2756 | 3.0616 | 3.1386 | 3.0463 |
| CLDN1 | 5.175105 | 4.07399 | 5.69935 | 5.25653 | 5.0154 | 5.5078 | 4.69244 | 4.132 | 4.7867 |
| MIR4530 | 10.9443 | 9.2709 | 11.6184 | 11.1975 | 11.081 | 11.277 | 11.3313 | 10.086 | 11.504 |
| MAFF | 8.49114 | 7.27831 | 9.26613 | 6.22565 | 6.0525 | 6.3947 | 9.6093 | 8.8522 | 9.7909 |
| ZNF765 | 3.602255 | 3.31332 | 3.71212 | 4.82805 | 4.5185 | 5.0909 | 4.70517 | 4.3841 | 4.7675 |
| CKS2 | 6.468755 | 6.98567 | 6.19645 | 2.60809 | 2.7465 | 2.3706 | 4.020185 | 2.8152 | 4.2086 |
| TCEAL7 | 5.114575 | 3.29422 | 6.17888 | 5.42383 | 5.3301 | 5.5736 | 5.06191 | 2.9046 | 5.2486 |
| PLIN1 | 4.436085 | 5.08342 | 3.74916 | 3.48572 | 3.666 | 3.2654 | 3.456275 | 3.8327 | 3.3792 |
| SIGLEC1 | 5.176255 | 6.12635 | 4.81258 | 6.27516 | 6.4169 | 6.1338 | 5.02289 | 5.1045 | 5.0181 |
| FAM1508 | 7.000985 | 5.10447 | 8.07842 | 6.77336 | 6.8114 | 6.6669 | 5.69935 | 4.4515 | 5.8569 |
| MFAP5 | 4.10253 | 2.34383 | 5.57364 | 3.80478 | 3.7365 | 3.8325 | 4.97069 | 2.9415 | 5.2471 |
| SFRP1 | 8.42439 | 6.87325 | 8.84832 | 5.40862 | 5.4486 | 5.3868 | 9.00425 | 8.4318 | 9.0461 |
| DUSPS | 6.049365 | 4.07026 | 6.89159 | 6.47079 | 6.5916 | 6.3697 | 3.380615 | 2 609 | 3.7498 |
| VARS2 | 5.144165 | 5.55841 | 4.68206 | 3.66826 | 3.4695 | 3. 8069 | 3.710975 | 3.4595 | 3.7374 |
| ABCC4 | 5.20667 | 4.77776 | 5.43315 | 5.64272 | 5.0619 | 5.9743 | 6.13912 | 6.2684 | 6.1369 |
| SH3BP4 | 4.840135 | 4.25165 | 5.42961 | 4.57785 | 4.4515 | 4.6512 | 5.320995 | 4.8281 | 5.4599 |
| SORD | 9.140035 | 9.07048 | 9.15822 | 7.74808 | 7.2239 | 8.0572 | 8.33616 | 8.2458 | 8.3401 |
| MTERFD1 | 5.513935 | 6.02508 | 5.22508 | 4.51834 | 4.7242 | 4.3928 | 3.69208 | 3.7427 | 3.6104 |
| DPP4 | 4.75566 | 3.70312 | 5.57364 | 4.24098 | 4.3217 | 4.2055 | 6.243255 | 5.4332 | 6.3479 |
| #N/A | 4.890245 | 5.51612 | 4.48785 | 3.49859 | 3.3219 | 3.6486 | 3.538075 | 3.5905 | 3.5304 |
| FAM3B | 7.73412 | 7.02685 | 8.0087 | 4.82805 | 4.8423 | 4.8124 | 9.0795 | 7.7829 | 9.1889 |

(continued)

| Gene Name | R0185 Up/Down Regulation | | | Taylor Up/Down Regulation | | | Clinical Validation Up/Down Regulation | | |
|---|---|---|---|---|---|---|---|---|---|
| | Median Threshold | Sig Pos | Sig Neg | Median Threshold | Sig Pos | Sig Neg | Median Threshold | Sig Pos | Sig Neg |
| KLK3 | 10.63635 | 10.611 | 10.7045 | 10.6617 | 10.395 | 10.802 | 12.8215 | 12.822 | 12.822 |

In certain embodiments the methods described herein may comprise determining the expression level of at least one of the genes with a negative weight listed in Table 1 together with at least one gene with a positive weight listed in Table 1. Thus, the methods may rely upon a combination of an up-regulated marker and a down-regulated marker. The combined up and down regulated marker expression levels, as appropriately weighted, may then contribute to, or make up, the final signature score.

In certain embodiments the methods described herein comprise comparing the expression level of one or more genes to a reference value or to the expression level in one or more control samples or to the expression level in one or more control cells in the same sample. The control cells may be normal (i.e. cells characterised by an independent method as non-cancerous) cells. The one or more control samples may consist of non-cancerous cells or may include a mixture of cancer cells (prostate, ER positive breast or otherwise) and non-cancerous cells. The expression level may be compared to the expression level of the same gene in one or more control samples or control cells.

The reference value may be a threshold level of expression of at least one gene set by determining the level or levels in a range of samples from subjects with and without the relevant cancer. The cancer, such as prostate cancer or ER positive breast cancer may be cancer with and/or without an increased likelihood of recurrence and/or metastasis and/or a poor prognosis. Suitable methods for setting a threshold are well known to those skilled in the art. The threshold may be mathematically derived from a training set of patient data. The score threshold thus separates the test samples according to presence or absence of the particular condition. The interpretation of this quantity, i.e. the cut-off threshold may be derived in a development or training phase from a set of patients with known outcome. The threshold may therefore be fixed prior to performance of the claimed methods from training data by methods known to those skilled in the art and as detailed herein in relation to generation of the various gene signatures.

The reference value may also be a threshold level of expression of at least one gene set by determining the level of expression of the at least one gene in a sample from a subject at a first time point. The determined levels of expression at later time points for the same subject are then compared to the threshold level. Thus, the methods of the invention may be used in order to monitor progress of disease in a subject, namely to provide an ongoing characterization and/or prognosis of disease in the subject. For example, the methods may be used to identify (or "diagnose") a cancer, such as prostate cancer or ER positive breast cancer that has developed into a more aggressive or potentially metastatic form. This may be used to guide treatment decisions as discussed in further detail herein. In some embodiments, such monitoring methods determine whether treatment should be administered or not. If the cancer is identified within the metastatic biology group the cancer should be treated. If the cancer is identified as "non-metastatic" further monitoring can be performed to ensure that the cancer remains stable (i.e. does not evolve into the metastatic form). In such circumstances, no further treatment may be applied.

For genes whose expression level does not differ between normal cells and cells from a cancer, such as prostate cancer or ER positive breast cancer that does not have an increased likelihood of recurrence and/or metastasis and/or a poor prognosis the expression level of the same gene in normal cells in the same sample can be used as a control.

Different may be statistically significantly different. By statistically significant is meant unlikely to have occurred by chance alone. A suitable statistical assessment may be performed according to any suitable method.

The methods described herein may further comprise determining the expression level of a reference gene. A reference gene may be required if the target gene expression level differs between normal cells and cells from a cancer, such as prostate cancer or ER positive breast cancer that does not have an increased likelihood of recurrence and/or metastasis and/or a poor prognosis.

In certain embodiments the expression level of at least one gene selected from Table 1 is compared to the expression level of a reference gene.

The reference gene may be any gene with minimal expression variance across all cancer, such as prostate cancer or ER positive breast cancer samples. Thus, the reference gene may be any gene whose expression level does not vary with likelihood of recurrence and/or metastasis and/or a poor prognosis. The skilled person is well able to identify a suitable reference gene based upon these criteria. The expression level of the reference gene may be determined in the same sample as the expression level of at least one gene selected from Table 1.

The expression level of the reference gene may be determined in a different sample. The different sample may be a control sample as described above. The expression level of the reference gene may be determined in normal cells and/or cancer, such as prostate cancer or ER positive breast cancer, cells in a sample.

The expression level of the at least one gene in the sample from the subject may be analysed using a statistical model. In specific embodiments where the expression level of THBS4 and at least 11 genes, up to all 70 genes from Table 1, is measured the genes may be weighted. As used herein, the term "weight" refers to the relative importance of an item in a statistical calculation. The weight of each gene may be determined on a data set of patient samples using analytical methods known in the art. An overall score, termed a "signature score", may be calculated and used to provide a characterisation of and/or prognosis for the cancer, such as prostate cancer or ER positive breast cancer. Typically, the score represents the sum of

the weighted gene expression levels. Suitable weights for calculating the 70 gene signature score are set forth in Table 1 and may be employed according to the methods of the invention. Similarly, suitable weights for exemplary smaller signatures are set forth in Tables 2 to 24.

Thus, according to all aspects of the invention, the methods may comprise:

(i) determining the expression level of THBS4 and at least 11 genes selected from Table 1 in a sample comprising nucleic acids from the primary tumour from the subject; and
(ii) assessing from the expression level of the genes whether the sample from the subject is positive or negative for a gene signature comprising the genes.

As discussed herein, if the sample is positive for the gene signature this identifies the cancer as of the high metastatic potential type. This may indicate a (relatively) poor prognosis, or any other pertinent associated characterisation, prognosis or diagnosis as described herein. By corollary, a sample negative for the gene signature identifies the cancer as not of the high metastatic potential type. This may indicate a (relatively) good prognosis, or any other pertinent associated characterisation, prognosis or diagnosis as described herein.

Thus, at its simplest, an increased level of expression of one or more genes defines a sample as positive for the gene signature. For certain genes, a decreased level of expression of one or more gene defines a sample as positive for the gene signature. However, where the expression level of a plurality of genes is measured, the combination of expression levels is typically aggregated in order to determine whether the sample is positive for the gene signature. Thus, some genes may display increased expression and some genes may display decreased expression. This can be achieved in various ways, as discussed in detail herein.

In specific embodiments, the signature score may be calculated according to the following equation:

$$ SignatureScore = \sum_i w_i \times (ge_i - b_i) + k $$

Where $w_i$ is a weight for each gene, $b_i$ is a gene-specific bias, $ge_i$ is the gene expression after pre-processing, and $k$ is a constant offset.

Similarly, each gene in the signature may be attributed a bias score. Example bias scores for the 70 gene signature are specified in table 1 and may be adopted according to the performance of the methods of the invention. Of course, where different signatures are utilised, representing a subset of the 70 gene signature, the bias values would be recalculated. Examples are provided in Tables 2 to 24.

As indicated, k is a constant offset. Where the bias and weight values of table 1 are adopted for the 70 gene signature, the constant offset may have a value of 0.4365. Again, where different signatures are utilised, representing a subset of the 70 gene signature, the value of k would be recalculated. The value of k varies dependent upon where the threshold for "signature positive" is set. This threshold may be set dependent upon which considerations are most important, e.g. to maximize sensitivity and/or specificity as against a particular outcome or characterisation. Suitable thresholds may be determined as described above.

In some embodiments, a score above the threshold may indicate a poor prognosis (or other pertinent characterisation, prognosis or diagnosis as described herein). In those embodiments, a score equal to or below threshold may indicate a good prognosis. In other embodiments, a score above or equal to the threshold may indicate a poor prognosis (or other pertinent characterisation, prognosis or diagnosis as described herein). In those embodiments, a score below threshold may indicate a good prognosis. The skilled person would also appreciate that a simple mathematical transformation could be used to invert the score and "above" and "below" should be construed accordingly unless indicated otherwise.

By "signature score" is meant a compound decision score that summarizes the expression levels of the genes. This may be compared to a threshold score that is mathematically derived from a training set of patient data. The threshold score is established with the purpose of maximizing the ability to separate cancers into those that are positive for the biomarker signature and those that are negative. The patient training set data is preferably derived from cancer tissue samples having been characterized by sub-type, prognosis, likelihood of recurrence, long term survival, clinical outcome, treatment response, diagnosis, cancer classification, or personalized genomics profile. Expression profiles, and corresponding decision scores from patient samples may be correlated with the characteristics of patient samples in the training set that are on the same side of the mathematically derived score decision threshold. In certain example embodiments, the threshold of the (linear) classifier scalar output is optimized to maximize the sum of sensitivity and specificity under cross-validation as observed within the training dataset.

The overall expression data for a given sample may be normalized using methods known to those skilled in the art in order to correct for differing amounts of starting material, varying efficiencies of the extraction and amplification reactions, etc.

In one embodiment, the biomarker expression levels in a sample are evaluated by a (linear) classifier. As used herein, a (linear) classifier refers to a weighted sum of the individual biomarker intensities into a compound decision score ("decision function"). The decision score is then compared to a pre-defined cut-off score threshold, corresponding to a certain set-point in terms of sensitivity and specificity which indicates if a sample is equal to or above the score threshold (decision function positive) or below (decision function negative).

Using a (linear) classifier on the normalized data to make a call (e.g. positive or negative for a biomarker signature) effectively means to split the data space, i.e. all possible combinations of expression values for all genes in the classifier, into two disjoint segments by means of a separating hyperplane. This split is empirically derived on a (large) set of training examples. Without loss of generality, one can assume a certain fixed set of values for all but one biomarker, which would automatically define a threshold value for this remaining biomarker where the decision would change from, for example, positive or negative for the biomarker signature. The precise value of this threshold depends on the actual measured expression profile of all other genes within the classifier, but the general indication of certain genes remains fixed. Therefore, in the context of the overall gene expression classifier, relative expression can indicate if either up- or down-regulation of a certain biomarker is indicative of being positive for the signature or not. In certain example embodiments, a sample expression score above the threshold expression score indicates the sample is positive for the biomarker signature. In certain other example embodiments, a sample expression score above a threshold score indicates the subject has a poor clinical prognosis compared to a subject with a sample expression score below the threshold score.

In certain other example embodiments, the expression signature is derived using a decision tree (Hastie et al. The Elements of Statistical Learning, Springer, New York 2001), a random forest (Breiman, 2001 Random Forests, Machine Learning 45:5), a neural network (Bishop, Neural Networks for Pattern Recognition, Clarendon Press, Oxford 1995), discriminant analysis (Duda et al. Pattern Classification, 2nd ed., John Wiley, New York 2001), including, but not limited to linear, diagonal linear, quadratic and logistic discriminant analysis, a Prediction Analysis for Microarrays (PAM, (Tibshirani et al., 2002, Proc. Natl. Acad. Sci. USA 99:6567-6572)) or a Soft Independent Modeling of Class Analogy analysis. (SIMCA, (Wold, 1976, Pattern Recogn. 8:127-139)). Classification trees (Breiman, Leo; Friedman, J. H.; Olshen, R. A.; Stone, C. J. (1984). Classification and regression trees. Monterey, CA: Wadsworth & Brooks/Cole Advanced Books & Software. ISBN 978-0-412-04841-8) provide a means of predicting outcomes based on logic and rules. A classification tree is built through a process called binary recursive partitioning, which is an iterative procedure of splitting the data into partitions/branches. The goal is to build a tree that distinguishes among pre-defined classes. Each node in the tree corresponds to a variable. To choose the best split at a node, each variable is considered in turn, where every possible split is tried and considered, and the best split is the one which produces the largest decrease in diversity of the classification label within each partition. This is repeated for all variables, and the winner is chosen as the best splitter for that node. The process is continued at the next node and in this manner, a full tree is generated. One of the advantages of classification trees over other supervised learning approaches such as discriminant analysis, is that the variables that are used to build the tree can be either categorical, or numeric, or a mix of both. In this way it is possible to generate a classification tree for predicting outcomes based on say the directionality of gene expression.

Random forest algorithms (Breiman, Leo (2001). "Random Forests". Machine Learning 45 (1): 5-32. doi:10.1023/A:1010933404324) provide a further extension to classification trees, whereby a collection of classification trees are randomly generated to form a "forest" and an average of the predicted outcomes from each tree is used to make inference with respect to the outcome.

Biomarker expression values may be defined in combination with corresponding scalar weights on the real scale with varying magnitude, which are further combined through linear or non-linear, algebraic, trigonometric or correlative means into a single scalar value via an algebraic, statistical learning, Bayesian, regression, or similar algorithms which together with a mathematically derived decision function on the scalar value provide a predictive model by which expression profiles from samples may be resolved into discrete classes of responder or non-responder, resistant or non-resistant, to a specified drug, drug class, molecular subtype, or treatment regimen. Such predictive models, including biomarker membership, are developed by learning weights and the decision threshold, optimized for sensitivity, specificity, negative and positive predictive values, hazard ratio or any combination thereof, under cross-validation, bootstrapping or similar sampling techniques, from a set of representative expression profiles from historical patient samples with known drug response and/or resistance.

In one embodiment, the genes are used to form a weighted sum of their signals, where individual weights can be positive or negative. The resulting sum ("expression score") is compared with a pre-determined reference point or value. The comparison with the reference point or value may be used to diagnose, or predict a clinical condition or outcome.

As described above, one of ordinary skill in the art will appreciate that the genes included in the classifier provided in the various Tables will carry unequal weights in a classifier. Therefore, while as few as one biomarker may be used to diagnose or predict a clinical prognosis or response to a therapeutic agent, the specificity and sensitivity or diagnosis or prediction accuracy may increase using more genes.

In certain example embodiments, the expression signature is defined by a decision function. A decision function is a set of weighted expression values derived using a (linear) classifier. All linear classifiers define the decision function using the following equation:

$$f(x) = w' \cdot x + b = \sum wi \cdot xi + b \quad (1)$$

All measurement values, such as the microarray gene expression intensities xi, for a certain sample are collected in a vector x. Each intensity is then multiplied with a corresponding weight wi to obtain the value of the decision function f(x) after adding an offset term b. In deriving the decision function, the linear classifier will further define a threshold value that splits the gene expression data space into two disjoint sections. Example (linear) classifiers include but are not limited to partial least squares (PLS), (Nguyen et al., Bioinformatics 18 (2002) 39-50), support vector machines (SVM) (Schölkopf et al., Learning with Kernels, MIT Press, Cambridge 2002), and shrinkage discriminant analysis (SDA) (Ahdesmäki et al., Annals of applied statistics 4, 503-519 (2010)). In one example embodiment, the (linear) classifier is a PLS linear classifier.

The decision function is empirically derived on a large set of training samples, for example from patients showing a good or poor clinical prognosis. The threshold separates a patient group based on different characteristics such as, but not limited to, clinical prognosis before or after a given therapeutic treatment. The interpretation of this quantity, i.e. the cut-off threshold, is derived in the development phase ("training") from a set of patients with known outcome. The corresponding weights and the responsiveness/resistance cut-off threshold for the decision score are fixed a priori from training data by methods known to those skilled in the art. In one example embodiment, Partial Least Squares Discriminant Analysis (PLS-DA) is used for determining the weights. (L. Stahle, S. Wold, J. Chemom. 1 (1987) 185-196; D. V. Nguyen, D.M. Rocke, Bioinformatics 18 (2002) 39-50).

Effectively, this means that the data space, i.e. the set of all possible combinations of biomarker expression values, is split into two mutually exclusive groups corresponding to different clinical classifications or predictions, for example, one corresponding to good clinical prognosis and poor clinical prognosis. In the context of the overall classifier, relative over-expression of a certain biomarker can either increase the decision score (positive weight) or reduce it (negative weight) and thus contribute to an overall decision of, for example, a good clinical prognosis.

In certain example embodiments of the invention, the data is transformed non-linearly before applying a weighted sum as described above. This non-linear transformation might include increasing the dimensionality of the data. The non-linear transformation and weighted summation might also be performed implicitly, for example, through the use of a kernel function. (Schölkopf et al. Learning with Kernels, MIT Press, Cambridge 2002).

In certain example embodiments, the patient training set data is derived by isolated RNA from a corresponding cancer tissue sample set and determining expression values by hybridizing the (cDNA amplified from) isolated RNA to a microarray. In certain example embodiments, the microarray used in deriving the expression signature is a transcriptome array. As used herein a "transcriptome array" refers to a microarray containing probe sets that are designed to hybridize to sequences that have been verified as expressed in the diseased tissue of interest. Given alternative splicing and variable poly-A tail processing between tissues and biological contexts, it is possible that probes designed against the same gene sequence derived from another tissue source or biological context will not effectively bind to transcripts expressed in the diseased tissue of interest, leading to a loss of potentially relevant biological information. Accordingly, it is beneficial to verify what sequences are expressed in the disease tissue of interest before deriving a microarray probe set. Verification of expressed sequences in a particular disease context may be done, for example, by isolating and sequencing total RNA from a diseased tissue sample set and cross-referencing the isolated sequences with known nucleic acid sequence databases to verify that the probe set on the transcriptome array is designed against the sequences actually expressed in the diseased tissue of interest. Methods for making transcriptome arrays are described in United States Patent Application Publication No. 2006/0134663,. In certain example embodiments, the probe set of the transcriptome array is designed to bind within 300 nucleotides of the 3' end of a transcript. Methods for designing transcriptome arrays with probe sets that bind within 300 nucleotides of the 3' end of target transcripts are disclosed in United States Patent Application Publication No. 2009/0082218. In certain example embodiments, the microarray used in deriving the gene expression profiles of the present invention is the Almac Prostate Cancer DSA™ microarray (Almac Group, Craigavon, United Kingdom).

An optimal (linear) classifier can be selected by evaluating a (linear) classifier's performance using such diagnostics as "area under the curve" (AUC). AUC refers to the area under the curve of a receiver operating characteristic (ROC) curve, both of which are well known in the art. AUC measures are useful for comparing the accuracy of a classifier across the complete data range. (Linear) classifiers with a higher AUC have a greater capacity to classify unknowns correctly between two groups of interest (e.g., ovarian cancer samples and normal or control samples). ROC curves are useful for plotting the performance of a particular feature (e.g., any of the genes described herein and/or any item of additional biomedical information) in distinguishing between two populations (e.g., individuals responding and not responding to a therapeutic agent). Typically, the feature data across the entire population (e.g., the cases and controls) are sorted in ascending order based on the value of a single feature. Then, for each value for that feature, the true positive and false positive rates for the data are calculated. The true positive rate is determined by counting the number of cases above the value for that feature and then dividing by the total number of positive cases. The false positive rate is determined by counting the number of controls above the value for that feature and then dividing by the total number of controls. Although this definition refers to scenarios in which a feature is elevated in cases compared to controls, this definition also applies to scenarios in which a feature is lower in cases compared to the controls (in such a scenario, samples below the value for that feature would be counted). ROC curves can be generated for a single feature as well as for other single outputs, for example, a combination of two or more features can be mathematically combined (e.g., added, subtracted, multiplied, etc.) to provide a single sum value, and this single sum value can be plotted in a ROC curve. Additionally, any combination of multiple features, in which the combination derives a single output value, can be plotted in a ROC curve. These combinations of features may comprise a test. The ROC curve is the plot of the true positive rate (sensitivity) of a test against the false positive rate (1 -specificity) of the test.

Alternatively, an optimal classifier can be selected by evaluating performance against time-to-event endpoints using methods such as Cox proportional hazards (PH) and measures of performance across all possible thresholds assessed via the concordance-index (C-index) (Harrell, Jr. 2010). The C-Index is analagous to the "area under the curve" (AUC) metric (used for dichotomised endpoints), and it is used to measure performance with respect to association with survival data. Note that the extension of AUC to time-to-event endpoints is the C-index, with threshold selection optimised to maximise the hazard ratio (HR) under cross-validation. In this instance, the partial Cox regression algorithm (Li and Gui, 2004) was chosen for the biomarker discovery analyses. It is analogous to principal components analysis in that the first few latent components explain most of the information in the data. Implementation is as described in Ahdesmaki et al 2013.

C-index values can be generated for a single feature as well as for other single outputs, for example, a combination of two or more features can be mathematically combined (e.g., added, subtracted, multiplied, etc.) to provide a single sum value, and this single sum value can be evaluated for statistical significance. Additionally, any combination of multiple features, in which the combination derives a single output value, can be evaluated as a C-index for assessing utility for time-to-event class separation. These combinations of features may comprise a test. The C-index (Harrell, Jr. 2010, see Equation 4) of the continuous cross-validation test set risk score predictions was evaluated as the main performance measure.

Methods for determining the expression levels of the at least one gene from Table 1 (biomarkers) are described in greater detail herein. Typically, the methods may involve contacting a sample obtained from a subject with a detection agent, such as primers and/or probes, or an antibody or functionally equivalent binding reagent, (as discussed in detail herein) specific for the gene and detecting expression products. The detection agent may be labelled as discussed herein. A comparison may be made against expression levels determined in a control sample to provide a characterization and/or a prognosis for the cancer, such as prostate cancer or ER positive breast cancer.

According to all aspects of the invention the expression level of the at least 12 genes comprising THBS4 may be measured by any suitable method. In the invention the expression level is determined at the level of RNA

The expression level of any of the genes described herein may be detected by detecting the appropriate RNA. The assays may investigate specific regions of the genes, as described herein. For example, the assays may investigate the regions flanked by specific primer binding sites and/or regions of the gene to which the probe sets described herein hybridize. The assays may investigate, promoter, terminator, exonic and/or intronic regions of the genes as appropriate. The assays may investigate one or more of the full sequences or target sequences, or regions thereof, as specified in Table 1 for the respective genes.

In certain embodiments, according to all aspects of the invention, expression of the at least 12 genes comprising THBS4 may be determined using one or more probes or primers (primer pairs) designed to hybridize with one or more of the target sequences or full sequences listed in Table 1. The probes and probesets identified in table 1 (and detailed further in Table 1A) may be employed according to all aspects of the invention. The primers and primer pairs listed in Table 1B and identified as SEQ ID NOs 3151-3154 may be employed according to all aspects of the invention.

Accordingly, in specific embodiments the expression level is determined by microarray, northern blotting, RNA-seq (RNA sequencing), in situ RNA detection or nucleic acid amplification. Nucleic acid amplification includes PCR and all variants thereof such as real-time and end point methods and quantitative PCR (qPCR). Other nucleic acid amplification techniques are well known in the art, and include methods such as NASBA, 3SR and Transcription Mediated Amplification (TMA). Other suitable amplification methods include the ligase chain reaction (LCR), selective amplification of target polynucleotide sequences (US Patent No. 6,410,276), consensus sequence primed polymerase chain reaction (US Patent No 4,437,975), arbitrarily primed polymerase chain reaction (WO 90/06995), invader technology, strand displacement technology, and nick displacement amplification (WO 2004/067726). This list is not intended to be exhaustive; any nucleic acid amplification technique may be used provided the appropriate nucleic acid product is specifically amplified. Design of suitable primers and/or probes is within the capability of one skilled in the art. Various primer design tools are freely available to assist in this process such as the NCBI Primer-BLAST tool. Primers and/or probes may be at least 15, 16, 17, 18, 19, 20, 21, 22, 23, 24 or 25 (or more) nucleotides in length. mRNA expression levels may be measured by reverse transcription quantitative polymerase chain reaction (RT-PCR followed with qPCR). RT-PCR is used to create a cDNA from the mRNA. The cDNA may be used in a qPCR assay to produce fluorescence as the DNA amplification process progresses. By comparison to a standard curve, qPCR can produce an absolute measurement such as number of copies of mRNA per cell. Northern blots, microarrays, Invader assays, and RT-PCR combined with capillary electrophoresis have all been used to measure expression levels of mRNA in a sample. See Gene Expression Profiling: Methods and Protocols, Richard A. Shimkets, editor, Humana Press, 2004. Many detection technologies are well known and commercially available, such as TAQMAN®, MOLECULAR BEACONS®, AMPLIFLUOR® and SCORPION®, DzyNA®, Plexor™ etc.

Suitable amplification assays (PCR or qPCR) have been designed by the inventors and are described in further detail in Table 1B. The forward and reverse primers listed therein for each gene may be utilized according to all aspects of the invention. Similarly, the primers of SEQ ID NOs 3151-3154 may be used to amplify MIR578 and MIR4530 respectively.

RNA-seq uses next-generation sequencing to measure changes in gene expression. RNA may be converted into cDNA or directly sequenced. Next generation sequencing techniques include pyrosequencing, SOLiD sequencing, Ion Torrent semiconductor sequencing, Illumina dye sequencing, single-molecule real-time sequencing or DNA nanoball sequencing. RNA-seq allows quantitation of gene expression levels.

In situ RNA detection involves detecting RNA without extraction from tissues and cells. In situ RNA detection includes in situ hybridization (ISH) which uses a labeled (e.g. radio labelled, antigen labelled or fluorescence labelled) probe (complementary DNA or RNA strand) to localize a specific RNA sequence in a portion or section of tissue, or in the entire tissue (whole mount ISH), or in cells. The probe labeled with either radio-, fluorescent- or antigen-labeled bases (e.g., digoxigenin) may be localized and quantified in the tissue using either autoradiography, fluorescence microscopy or immunohistochemistry, respectively. ISH can also use two or more probes to simultaneously detect two or more transcripts. A branched DNA assay can also be used for RNA in situ hybridization assays with single molecule sensitivity. This approach includes ViewRNA assays. Samples (cells, tissues) are fixed, then treated to allow RNA target accessibility (RNA un-masking). Target-specific probes hybridize to each target RNA. Subsequent signal amplification is predicated on specific hybridization of adjacent probes (individual oligonucleotides that bind side by side on RNA targets). A typical target-specific probe will contain 40 oligonucleotides. Signal amplification is achieved via a series of sequential hybridization steps. A pre-amplifier molecule hybridizes to each oligo pair on the target-specific RNA, then multiple amplifier molecules hybridize to each pre-amplifier. Next, multiple label probe oligonucleotides (conjugated to an enzyme such as alkaline phosphatase or directly to fluorophores) hybridize to each amplifier molecule. Separate but compatible signal amplification systems enable multiplex assays. The signal can be visualized by measuring fluorescence or light emitted depending upon the detection system employed. Detection may involve using a high content imaging system, or a fluorescence or brightfield microscope in some embodiments.

Described herein is the use of a kit for characterising and/or prognosing cancer, such as prostate cancer or ER positive breast cancer. The kit for (in situ) characterising and/or prognosing prostate cancer in a subject may comprise one or more oligonucleotide probes specific for an RNA product of at least one gene selected from Table 1. Suitable probes and probesets for each gene are listed in Table 1 and may be incorporated in the kits. By "probeset" is meant the collection of probes designed to target (by hybridization) a single gene. The groupings are apparent from table 1 (and Table 1A).

The kit may further comprise one or more of the following components:

   a) A blocking probe

   b) A PreAmplifier

   c) An Amplifier and/or

   d) A Label molecule

The components of the kit may be suitable for conducting a viewRNA assay (https://www.panomics.com/products/rna-in-situ-analysis/view-rna-overview).

The components of the kit may be nucleic acid based molecules, optionally DNA (or RNA). The blocking probe is a molecule that acts to reduce background signal by binding to sites on the target not bound by the target specific probes (probes specific for the RNA product of the at least one gene). The PreAmplifier is a molecule capable of binding to a (a pair of) target specific probe(s) when target bound. The Amplifier is a molecule capable of binding to the PreAmplifier. Alternatively, the Amplifier may be capable of binding directly to a (a pair of) target specific probe(s) when target bound. The Amplifier has binding sites for multiple label molecules (which may be label probes).

RNA expression may be determined by hybridization of RNA to a set of probes. The probes may be arranged in an array. Microarray platforms include those manufactured by companies such as Affymetrix, Illumina and Agilent. Examples of microarray platforms manufactured by Affymetrix include the U133 Plus2 array, the Almac proprietary Xcel™ array and the Almac proprietary Cancer DSAs®, including the Prostate Cancer DSA®.

In specific embodiments, according to all aspects of the invention, expression of the at least 12 genes comprising THBS4 may be determined using one or more probes selected from those listed in Table 1.

In certain embodiments, according to all aspects of the invention, expression of the at least 12 genes comprising THBS4 may be determined using one or more probes or primers designed to hybridize with the target sequences or full sequences listed in Table 1.

These probes may also be incorporated into the kits. The probe sequences may also be used in order to design primers for detection of expression, for example by RT-PCR. Such primers may also be included in the kits. Suitable primers are listed in Table 1B and SEQ ID NOs 3151-3154.

The corresponding target sequences are listed in Table 1 below for the relevant probesets. The invention may involve use of different probes that target any one or more of these target sequences.

Similarly, the full gene sequences are listed in Table 1 for the relevant probesets. The invention may involve use of different probes that target any one or more of these full gene sequences as target sequences.

Increased rates of DNA methylation at or near promoters have been shown to correlate with reduced gene expression levels. DNA methylation is the main epigenetic modification in humans. It is a chemical modification of DNA performed by enzymes called methyltransferases, in which a methyl group (m) is added to specific cytosine (C) residues in DNA. In mammals, methylation occurs only at cytosine residues adjacent to a guanosine residue, i.e. at the sequence CG or at the CpG dinucleotide.

Described herein is a method for characterising and/or prognosing cancer, such as prostate cancer or ER positive breast cancer in a subject comprising:

determining the methylation status of at least one gene selected from Table 1 in a sample from the subject wherein the determined methylation status is used to provide a characterisation of and/or a prognosis for the cancer, such as prostate cancer or ER positive breast cancer.

Methylation typically results in a down regulation of gene expression. Thus, methylation (which may be hypermethylation) of the genes with a negative weighting in table 1 may be determined in order to indicate a poor prognosis (or related outcome as described herein). Additionally or alternatively, a lack of methylation (which may be hypomethylation) of the genes with a positive weighting in table 1 may be determined in order to indicate a poor prognosis (or related outcome as described herein).

Determination of the methylation status may be achieved through any suitable means. Suitable examples include bisulphite genomic sequencing and/or by methylation specific PCR. Various techniques for assessing methylation status are known in the art and can be used: sequencing (including NGS), methylation-specific PCR (MS-PCR), melting curve methylation-specific PCR(McMS-PCR), MLPA with or without bisulphite treatment, QAMA (Zeschnigk et al, 2004), MSRE-PCR (Melnikov et al, 2005), MethyLight (Eads et al., 2000), ConLight-MSP (Rand et al., 2002), bisulphite conversion-specific methylation-specific PCR (BS-MSP)(Sasaki et al., 2003), COBRA (which relies upon use of restriction enzymes to reveal methylation dependent sequence differences in PCR products of sodium bisulphite - treated DNA), methylation-sensitive single-nucleotide primer extension conformation(MS-SNuPE), methylation-sensitive single-strand conformation analysis (MS-SSCA), Melting curve combined bisulphite restriction analysis (McCOBRA)(Akey et al., 2002), PyroMethA, HeavyMethyl (Cottrell et al. 2004), MALDI-TOF, MassARRAY, Quantitative analysis of methylated alleles (QAMA), enzymatic regional methylation assay (ERMA), QBSUPT, MethylQuant, Quantitative PCR sequencing and oligonucleotide-based microarray systems, Pyrosequencing, Meth-DOP-PCR. A review of some useful techniques for DNA methylation analysis is provided in Nucleic acids research, 1998, Vol. 26, No. 10, 2255-2264, Nature Reviews, 2003, Vol.3, 253-266; Oral Oncology, 2006, Vol. 42, 5-13.

Techniques for assessing methylation status are based on distinct approaches. Some include use of endonucleases. Such endonucleases may either preferentially cleave methylated recognition sites relative to non-methylated recognition sites or preferentially cleave non-methylated relative to methylated recognition sites. Some examples of the former are Acc III, Ban I, BstN I, Msp I, and Xma I. Examples of the latter are Acc II, Ava I, BssH II, BstU I, Hpa II, and Not I. Differences in cleavage pattern are indicative for the presence or absence of a methylated CpG dinucleotide. Cleavage patterns can be detected directly, or after a further reaction which creates products which are easily distinguishable. Means which detect altered size and/or charge can be used to detect modified products, including but not limited to electrophoresis, chromatography, and mass spectrometry.

Alternatively, the identification of methylated CpG dinucleotides may utilize the ability of the methyl binding domain (MBD) of the MeCP2 protein to selectively bind to methylated DNA sequences (Cross et al, 1994; Shiraishi et al, 1999). The MBD may also be obtained from MBP, MBP2, MBP4, poly-MBD (Jorgensen et al., 2006) or from reagents such as antibodies binding to methylated nucleic acid. The MBD may be immobilized to a solid matrix and used for preparative column chromatography to isolate highly methylated DNA sequences. Variant forms such as expressed His-tagged methyl-CpG binding domain may be used to selectively bind to methylated DNA sequences. Eventually, restriction endonuclease digested genomic DNA is contacted with expressed His-tagged methyl-CpG binding domain. Other methods are well known in the art and include amongst others methylated-CpG island recovery assay (MIRA). Another method, MB-PCR, uses a recombinant, bivalent methyl-CpG-binding polypeptide immobilized on the walls of a PCR vessel to capture methylated DNA and the subsequent detection of bound methylated DNA by PCR.

Further approaches for detecting methylated CpG dinucleotide motifs use chemical reagents that selectively modify either the methylated or non-methylated form of CpG dinucleotide motifs. Suitable chemical reagents include hydrazine and bisulphite ions. The methods may use bisulphite ions. The bisulphite conversion relies on treatment of DNA samples with sodium bisulphite which converts unmethylated cytosine to uracil, while methylated cytosines are maintained (Furuichi et al., 1970). This conversion finally results in a change in the sequence of the original DNA. It is general knowledge that the resulting uracil has the base pairing behaviour of thymidine which differs from cytosine base pairing behaviour. This makes the discrimination between methylated and non-methylated cytosines possible. Useful conventional techniques of molecular biology and nucleic acid chemistry for assessing sequence differences are well known in the art and explained in the literature. See, for example, Sambrook, J., et al., Molecular cloning: A laboratory Manual, (2001) 3rd edition, Cold Spring Harbor, NY; Gait, M.J.(ed.), Oligonucleotide Synthesis, A Practical Approach, IRL Press (1984); Hames B.D., and Higgins, S.J. (eds.), Nucleic Acid Hybridization, A Practical Approach, IRL Press (1985); and the series, Methods in Enzymology, Academic Press, Inc.

Some techniques use primers for assessing the methylation status at CpG dinucleotides. Two approaches to primer design are possible. Firstly, primers may be designed that themselves do not cover any potential sites of DNA methylation. Sequence variations at sites of differential methylation are located between the two primers and visualisation of the sequence variation requires further assay steps. Such primers are used in bisulphite genomic sequencing, COBRA, Ms-SnuPE and several other techniques. Secondly, primers may be designed that hybridize specifically with either the methylated or unmethylated version of the initial treated sequence. After hybridization, an amplification reaction can be performed and amplification products assayed using any detection system known in the art. The presence of an amplification product indicates that a sample hybridized to the primer. The specificity of the primer indicates whether the DNA had been modified or not, which in turn indicates whether the DNA had been methylated or not. If there is a sufficient region of complementarity, e.g., 12, 15, 18, or 20 nucleotides, to the target, then the primer may also contain additional nucleotide residues that do not interfere with hybridization but may be useful for other manipulations. Examples of such other residues may be sites for restriction endonuclease cleavage, for ligand binding or for factor binding or linkers or repeats. The oligonucleotide primers may or may not be such that they are specific for modified methylated residues.

A further way to distinguish between modified and unmodified nucleic acid is to use oligonucleotide probes. Such probes may hybridize directly to modified nucleic acid or to further products of modified nucleic acid, such as products obtained by amplification. Probe-based assays exploit the oligonucleotide hybridisation to specific sequences and subsequent detection of the hybrid. There may also be further purification steps before the amplification product is detected e.g. a precipitation step. Oligonucleotide probes may be labeled using any detection system known in the art. These include but are not limited to fluorescent moieties, radioisotope labeled moieties, bioluminescent moieties, luminescent moieties, chemiluminescent moieties, enzymes, substrates, receptors, or ligands.

In the MSP approach, DNA may be amplified using primer pairs designed to distinguish methylated from unmethylated DNA by taking advantage of sequence differences as a result of sodium-bisulphite treatment (WO 97/46705). For example, bisulphite ions modify non-methylated cytosine bases, changing them to uracil bases. Uracil bases hybridize to adenine bases under hybridization conditions. Thus an oligonucleotide primer which comprises adenine bases in place of guanine bases would hybridize to the bisulphite-modified DNA, whereas an oligonucleotide primer containing the guanine bases would hybridize to the non-modified (methylated) cytosine residues in the DNA. Amplification using a DNA polymerase and a second primer yield amplification products which can be readily observed, which in turn indicates whether the DNA had been methylated or not. Whereas PCR is a preferred amplification method, variants on this basic technique such as nested PCR and multiplex PCR are also included within the scope of the invention.

As mentioned earlier, assessing the methylation status of the relevant gene may require amplification to yield amplification products. The presence of amplification products may be assessed directly using methods well known in the art, and the ensuing discussion also applies to all other amplification embodiments as described herein.. They simply may be visualized on a suitable gel, such as an agarose or polyacrylamide gel. Detection may involve the binding of specific dyes, such as ethidium bromide, which intercalate into double-stranded DNA and visualisation of the DNA bands under a UV illuminator for example. Another means for detecting amplification products comprises hybridization with oligonucleotide probes. Alternatively, fluorescence or energy transfer can be measured to determine the presence of the methylated DNA.

A specific example of the MSP technique is designated real-time quantitative MSP (QMSP), and permits reliable quantification of methylated DNA in real time or at end point. Real-time methods are generally based on the continuous optical monitoring of an amplification procedure and utilise fluorescently labelled reagents whose incorporation in a product can be quantified and whose quantification is indicative of copy number of that sequence in the template. One such reagent is a fluorescent dye, called SYBR Green I that preferentially binds double-stranded DNA and whose fluorescence is greatly enhanced by binding of double-stranded DNA. Alternatively, labelled primers and/or labelled probes can be used for quantification. They represent a specific application of the well-known and commercially available real-time amplification techniques such as TAQMAN®, MOLECULAR BEACONS®, AMPLIFLUOR® and SCORPION®, DzyNA®, PlexorTM etc. In the real-time PCR systems, it is possible to monitor the PCR reaction during the exponential phase where the first significant increase in the amount of PCR product correlates to the initial amount of target template.

Real-Time PCR detects the accumulation of amplicon during the reaction. Real-time methods do not need to be utilised, however. Many applications do not require quantification and Real-Time PCR is used only as a tool to obtain convenient results presentation and storage, and at the same time to avoid post-PCR handling. Thus, analyses can be performed only to confirm whether the target DNA is present in the sample or not. Such end-point verification is carried out after the amplification reaction has finished.

The expression level of one or more genes from Table 1 may be determined by immunohistochemistry. By Immunohistochemistry is meant the detection of proteins in cells of a tissue sample by using a binding reagent such as an antibody or aptamer that binds specifically to the proteins. Thus, the expression level as determined by immunohistochemistry is a protein level. The sample may be a tissue sample and may comprise cancer (tumour) cells, normal tissue cells and, optionally, infiltrating immune cells. In embodiments applicable to prostate cancer, the sample may be a prostate tissue sample and may comprise prostate cancer (tumour) cells, prostatic intraepithelial neoplasia (PIN) cells, normal prostate epithelium, stroma and, optionally, infiltrating immune cells. In some embodiments the expression level of the at least 12 genes comprising THBS4 in the cancer (tumour) cells in a sample is compared to the expression level of the same genes (and/or a reference gene) in the normal cells in the same sample. In some embodiments the expression level of the at least 12 genes comprising THBS4 in the cancer (tumour) cells in a sample is compared to the expression level of the same genes (and/or a reference gene) in the normal cells in a control sample. The normal cells may comprise, consist essentially of or consist of normal (non-cancer) epithelial cells. In certain embodiments the normal cells do not comprise PIN cells and/or stroma cells. In certain embodiments the prostate cancer (tumour) cells do not comprise PIN cells and/or stroma cells. In further embodiments the expression level of the at least one gene in the prostate cancer (tumour) cells in a sample is (additionally) compared to the expression level of a reference gene in the same cells or in the prostate cancer cells in a control sample. In yet further embodiments the expression level of the at least 12 genes comprising THBS4 in the cancer (tumour) cells in a sample is scored using a method based on intensity, proportion and/or localisation of expression in the cancer (tumour) cells (without comparison to normal cells). The scoring method may be derived in a development or training phase from a set of patients with known outcome.

Described herein is an antibody or aptamer that binds specifically to a protein product of at least one gene selected from Table 1. The epitope to which the antibody or aptomer binds may be derived from the amino acid sequences corresponding to the full sequences or target sequences identified in Table 1.

The antibody may be of monoclonal or polyclonal origin. Fragments and derivative antibodies may also be utilised, to include without limitation Fab fragments, ScFv, single domain antibodies, nanoantibodies, heavy chain antibodies, aptamers etc. which retain peptide-specific binding function and these are included in the definition of "antibody". Such antibodies are useful in the methods described herein. They may be used to measure the level of a particular protein, or in some instances one or more specific isoforms of a protein. The skilled person is well able to identify epitopes that permit specific isoforms to be discriminated from one another.

Methods for generating specific antibodies are known to those skilled in the art. Antibodies may be of human or non-human origin (e.g. rodent, such as rat or mouse) and be humanized etc. according to known techniques (Jones et al., Nature (1986) May 29-Jun. 4;321(6069):522-5; Roguska et al., Protein Engineering, 1996, 9(10):895-904; and Studnicka et al., Humanizing Mouse Antibody Frameworks While Preserving 3-D Structure. Protein Engineering, 1994, Vol.7, pg 805).

In certain instances the expression level is determined using an antibody or aptamer conjugated to a label. By label is meant a component that permits detection, directly or indirectly. For example, the label may be an enzyme, optionally a peroxidase, or a fluorophore.

A label is an example of, and may form part of, a detection agent. By detection agent is meant an agent that may be used to assist in the detection of the complex between binding reagent (which may be an antibody, primer or probe for example) and target. The binding agent may form part of the overall detection agent. Where the antibody is conjugated to an enzyme the detection agent may be comprise a chemical composition such that the enzyme catalyses a chemical reaction to produce a detectable product. The products of reactions catalyzed by appropriate enzymes can be, without limitation, fluorescent, luminescent, or radioactive or they may absorb visible or ultraviolet light. Examples of detectors suitable for detecting such detectable labels include, without limitation, x-ray film, radioactivity counters, scintillation counters, spectrophotometers, colorimeters, fluorometers, luminometers, and densitometers. In certain instances the detection agent may comprise a secondary antibody. The expression level is then determined using an unlabeled primary antibody that binds to the target protein and a secondary antibody conjugated to a label, wherein the secondary antibody binds to the primary antibody.

Disclosed herein is the use of an antibody or aptamer as described above for characterising and/or prognosing a cancer, such as prostate cancer or ER positive breast cancer in a subject.

Additional techniques for determining expression level at the level of protein include, for example, Western blot, immunoprecipitation, immunocytochemistry, mass spectrometry, ELISA and others (see ImmunoAssay: A Practical Guide, edited by Brian Law, published by Taylor & Francis, Ltd., 2005 edition). To improve specificity and sensitivity of an assay method based on immunoreactivity, monoclonal antibodies are often used because of their specific epitope recognition. Polyclonal antibodies have also been successfully used in various immunoassays because of their increased affinity for the target as compared to monoclonal antibodies.

According to all aspects of the invention samples may be of any suitable form. The sample comprises nucleic acids (DNA and/or RNA) from the primary tumour (even if no longer contained within the tumour cells e.g. shed into the circulation). The sample may comprise, consist essentially of or consist of cells, such as prostate or breast cells and often a suitable tissue sample (such as a prostate or breast tissue sample). The sample may comprise or be a primary tumour sample. The cells or tissue may comprise cancer cells, such as prostate cancer cells or ER positive breast cancer cells. In specific embodiments the sample comprises, consists essentially of or consists of a biopsy sample, which may be fixed, such as a formalin-fixed paraffin-embedded biopsy sample. The tissue sample may be obtained by any suitable technique. Examples include a biopsy procedure, optionally a fine needle aspirate biopsy procedure. Body fluid samples may also be utilised. Samples may comprise resection material (e.g. where radical prostatectomy has been performed). Suitable sample types include blood, to encompass whole blood, serum and plasma samples, urine and semen.

The methods described herein may further comprise extracting nucleic acids, DNA and/or RNA from the sample. Suitable methods are known in the art and include use of commercially available kits such as Rneasy and GeneJET RNA purification kit.

Described herein are methods which may further comprise obtaining the sample from the subject. In the invention, the methods are in vitro methods performed on an isolated sample.

The methods of the invention may prove useful for determining which patients should undergo a more aggressive therapeutic regime, by identifying high risk cancers (i.e, those within the high metastatic potential group and thus having a poor prognosis).

The methods described herein may comprise selecting a treatment for cancer, such as prostate cancer or ER positive breast cancer in a subject and optionally performing the treatment. In certain embodiments if the characterisation of and/or prognosis for the cancer, such as prostate cancer or ER positive breast cancer is an increased likelihood of recurrence and/or metastasis and/or a poor prognosis the treatment selected may be one or more of

   a) an anti-hormone treatment
   b) a cytotoxic agent
   c) a biologic
   d) radiotherapy
   e) targeted therapy
   f) surgery

By anti-hormone treatment (or hormone therapy) is meant a form of treatment which reduces the level and/or activity of selected hormones, in particular testosterone. The hormones may promote tumour growth and/or metastasis. The anti-hormone treatment may comprise a luteinizing hormone blocker, such as goserelin (also called Zoladex), buserelin, leuprorelin (also called Prostap), histrelin (Vantas) and triptorelin (also called Decapeptyl). The anti-hormone treatment may comprise a gonadotrophin release hormone (GnRH) blocker such as degarelix (Firmagon) or an anti-androgen such as flutamide (also called Drogenil) and bicalutamide (also called Casodex). In specific embodiments the anti-hormone treatment may be bicalutamide and/or abiraterone.

The cytotoxic agent may be administered as an adjuvant therapy. The cytotoxic agent may be a platinum based agent and/or a taxane. In specific embodiments the platinum based agent is selected from cisplatin, carboplatin and oxaliplatin. The taxane may be paclitaxel, cabazitaxel or docetaxel. The cytotoxic agent may also be a vinca alkaloid, such as vinorelbine or vinblastine. The cytotoxic agent may be a topoisomerase inhibitor such as etoposide or an anthracycline (antibiotic) such as doxorubicin. The cytotoxic agent may be an alkylating agent such as estramustine. Adjuvant taxane and/or topoisomerase inhibitor therapy may be particularly suitable for treatment of ER positive breast cancer.

By biologic is meant a medicinal product that is created by a biological process. A biologic may be, for example, a vaccine, blood or blood component, cells, gene therapy, tissue, or a recombinant therapeutic protein. Optionally the biologic is an antibody and/or a vaccine. The biologic may be Sipuleucel-T. The biologic may be a cancer immunotherapy.

In certain embodiments the radiotherapy is extended radiotherapy, preferably extended-field radiotherapy. In specific embodiments, the radiotherapy comprises or is (pelvic) lymph node irradiation. Adjuvant radiation may be employed.

Surgery may comprise radical prostatectomy. By radical prostatectomy is meant removal of the entire prostate gland, the seminal vesicles and the vas deferens. In further embodiments surgery comprises tumour resection i.e. removal of all or part of the tumour. Surgery may comprise or be extended nodal dissection.

By targeted therapy is meant treatment using targeted therapeutic agents which are directed towards a specific drug target for the treatment of a cancer, such as prostate cancer or ER positive breast cancer. In specific embodiments this may mean inhibitors directed towards targets such as PARP, AKT, MET, VEGFR etc. PARP inhibitors are a group of pharmacological inhibitors of the enzyme poly ADP ribose polymerase (PARP). Several forms of cancer are more dependent on PARP than regular cells, making PARP an attractive target for cancer therapy. Examples (in clinical trials) include iniparib, olaparib, rucaparib, veliparib, CEP 9722, MK 4827, BMN-673 and 3-aminobenzamide. AKT, also known as Protein Kinase B (PKB), is a serine/threonine-specific protein kinase that plays a key role in multiple cellular processes such as glucose metabolism, apoptosis, cell proliferation, transcription and cell migration. AKT is associated with tumor cell survival, proliferation, and invasiveness. Examples of AKT inhibitors include VQD-002, Perifosine, Miltefosine and AZD5363. MET is a proto-oncogene that encodes hepatocyte growth factor receptor (HGFR). The hepatocyte growth factor receptor protein possesses tyrosine-kinase activity. Examples of kinase inhibitors for inhibition of MET include K252a, SU11274, PHA-66752, ARQ197, Foretinib, SGX523 and MP470. MET activity can also be blocked by inhibiting the interaction with HGF. Many suitable antagonists including truncated HGF, anti-HGF antibodies and uncleavable HGF are known. VEGF receptors are receptors for vascular endothelial growth factor (VEGF). Various inhibitors are known such as lenvatinib, motesanib, pazopanib and regorafenib.

If the method identifies the cancer as not within the high metastatic potential group, then different decisions may be taken. If the cancer has already been treated e.g. by radiotherapy or surgery, the decision may be taken not to treat the cancer further. The decision may be taken to continue to monitor the cancer, by any suitable means (e.g. by PSA levels or using the methods of the invention), and not perform any further treatment if the cancer remains in the same state.

The methods of the present invention can guide therapy selection as well as selecting patient groups for enrichment strategies during clinical trial evaluation of novel therapeutics. For example, when evaluating a putative anti-cancer agent or treatment regime, the methods disclosed herein may be used to select individuals for clinical trials that have cancer, such as prostate cancer or ER positive breast cancer, characterized as having an increased likelihood of recurrence and/or metastasis and/or a poor prognosis.

The invention also relates to a system or device or test kit for performing a method as described herein.

In a further aspect, the present invention relates to a system, device or test kit for characterising and/or prognosing cancer, such as prostate cancer or ER positive breast cancer in a subject with a primary cancer but no known metastases, comprising:

   a) one or more testing devices that determine the expression level of THBS4 and at least 11 genes selected from Table 1 in a sample comprising nucleic acids from the primary tumour from the subject
   b) a processor; and
   c) storage medium comprising a computer application that, when executed by the processor, is configured to:

      (i) access and/or calculate the determined expression levels the genes selected from Table 1 in the sample on the one or more testing devices
      (ii) calculate whether there is an increased or decreased level of the genes selected from Table 1 in the sample; and
      (iii) output from the processor the characteristaion of and/or prognosis for the cancer, such as prostate cancer or ER positive breast cancer, wherein the characterisation of and/or prognosis for the cancer consists of predicting an increased likelihood of recurrence and/or predicting an increased likelihood of metastasis and/or a poor prognosis.

By testing device is meant a combination of components that allows the expression level of a gene to be determined. The components may include any of those described above with respect to the methods for determining expression level at the level of protein, RNA or epigenetic modification. For example the components may be antibodies, primers, detection agents and so on. Components may also include one or more of the following: microscopes, microscope slides, x-ray film, radioactivity counters, scintillation counters, spectrophotometers, colorimeters, fluorometers, luminometers, and densitometers. The discussion of the methods of the invention thus applies mutatis mutandis to these aspects of the invention.

In certain embodiments the system, device or test kit further comprises a(n electronic) display for the output from the processor.

The invention also relates to a computer application or storage medium comprising a computer application as defined above.

In certain example embodiments, provided is a computer program product for characterising and/or prognosing cancer, such as prostate cancer or ER positive breast cancer in a subject, in accordance with the methods described herein. For example, the computer program product may comprise a non-transitory computer-readable storage device having computer-readable program instructions embodied thereon that, when executed by a computer, cause the computer to characterise and/or prognose cancer, such as prostate cancer or ER positive breast cancer in a subject as described herein. For example, the computer executable instructions may cause the computer to:

(i) access and/or calculate the determined expression levels of THBS4 and at least 11 genes selected from Table 1 in a sample comprising nucleic acids from the primary tumour on one or more testing devices;
(ii) calculate whether there is an increased or decreased level of the genes selected from Table 1 in the sample; and,
(iii) provide an output regarding the characterization of and/or prognosis for the cancer, such as prostate cancer or ER positive breast cancer, wherein the characterisation of and/or prognosis for the cancer consists of predicting an increased likelihood of recurrence and/or predicting an increased likelihood of metastasis and/or a poor prognosis.

In certain example embodiments, the computer-implemented method, system, and computer program product may be embodied in a computer application, for example, that operates and executes on a computing machine and a module. When executed, the application may characterise and/or prognose cancer, such as prostate cancer or ER positive breast cancer in a subject, in accordance with the example embodiments described herein.

As used herein, the computing machine may correspond to any computers, servers, embedded systems, or computing systems. The module may comprise one or more hardware or software elements configured to facilitate the computing machine in performing the various methods and processing functions presented herein. The computing machine may include various internal or attached components such as a processor, system bus, system memory, storage media, input/output interface, and a network interface for communicating with a network, for example. The computing machine may be implemented as a conventional computer system, an embedded controller, a laptop, a server, a customized machine, any other hardware platform, such as a laboratory computer or device, for example, or any combination thereof. The computing machine may be a distributed system configured to function using multiple computing machines interconnected via a data network or bus system, for example.

The processor may be configured to execute code or instructions to perform the operations and functionality described herein, manage request flow and address mappings, and to perform calculations and generate commands. The processor may be configured to monitor and control the operation of the components in the computing machine. The processor may be a general purpose processor, a processor core, a multiprocessor, a reconfigurable processor, a microcontroller, a digital signal processor ("DSP"), an application specific integrated circuit ("ASIC"), a graphics processing unit ("GPU"), a field programmable gate array ("FPGA"), a programmable logic device ("PLD"), a controller, a state machine, gated logic, discrete hardware components, any other processing unit, or any combination or multiplicity thereof. The processor may be a single processing unit, multiple processing units, a single processing core, multiple processing cores, special purpose processing cores, co-processors, or any combination thereof. According to certain example embodiments, the processor, along with other components of the computing machine, may be a virtualized computing machine executing within one or more other computing machines.

The system memory may include non-volatile memories such as read-only memory ("ROM"), programmable read-only memory ("PROM"), erasable programmable read-only memory ("EPROM"), flash memory, or any other device capable of storing program instructions or data with or without applied power. The system memory may also include volatile memories such as random access memory ("RAM"), static random access memory ("SRAM"), dynamic random access memory ("DRAM"), and synchronous dynamic random access memory ("SDRAM"). Other types of RAM also may be used to implement the system memory. The system memory may be implemented using a single memory module or multiple memory modules. While the system memory may be part of the computing machine, one skilled in the art will recognize that the system memory may be separate from the computing machine without departing from the scope of the subject technology. It should also be appreciated that the system memory may include, or operate in conjunction with, a non-volatile storage device such as the storage media.

The storage media may include a hard disk, a floppy disk, a compact disc read only memory ("CD-ROM"), a digital versatile disc ("DVD"), a Blu-ray disc, a magnetic tape, a flash memory, other non-volatile memory device, a solid state drive ("SSD"), any magnetic storage device, any optical storage device, any electrical storage device, any semiconductor storage device, any physical-based storage device, any other data storage device, or any combination or multiplicity thereof. The storage media may store one or more operating systems, application programs and program modules such as module, data, or any other information. The storage media may be part of, or connected to, the computing machine. The storage media may also be part of one or more other computing machines that are in communication with the computing machine, such as servers, database servers, cloud storage, network attached storage, and so forth.

The module may comprise one or more hardware or software elements configured to facilitate the computing machine with performing the various methods and processing functions presented herein. The module may include one or more sequences of instructions stored as software or firmware in association with the system memory, the storage media, or both. The storage media may therefore represent examples of machine or computer readable media on which instructions or code may be stored for execution by the processor. Machine or computer readable media may generally refer to any medium or media used to provide instructions to the processor. Such machine or computer readable media associated with the module may comprise a computer software product. It should be appreciated that a computer software product comprising the module may also be associated with one or more processes or methods for delivering the module to the computing machine via a network, any signal-bearing medium, or any other communication or delivery technology. The module may also comprise hardware circuits or information for configuring hardware circuits such as microcode or configuration information for an FPGA or other PLD.

The input/output ("I/O") interface may be configured to couple to one or more external devices, to receive data from the one or more external devices, and to send data to the one or more external devices. Such external devices along with the various internal devices may also be known as peripheral devices. The I/O interface may include both electrical and physical connections for operably coupling the various peripheral devices to the computing machine or the processor. The I/O interface may be configured to communicate data, addresses, and control signals between the peripheral devices, the computing machine, or the processor. The I/O interface may be configured to implement any standard interface, such as small computer system interface ("SCSI"), serial-attached SCSI ("SAS"), fiber channel, peripheral component interconnect ("PCI"), PCI express (PCIe), serial bus, parallel bus, advanced technology attached ("ATA"), serial ATA ("SATA"), universal serial bus ("USB"), Thunderbolt, FireWire, various video buses, and the like. The I/O interface may be configured to implement only one interface or bus technology.

Alternatively, the I/O interface may be configured to implement multiple interfaces or bus technologies. The I/O interface may be configured as part of, all of, or to operate in conjunction with, the system bus. The I/O interface may include one or more buffers for buffering transmissions between one or more external devices, internal devices, the computing machine, or the processor.

The I/O interface may couple the computing machine to various input devices including mice, touch-screens, scanners, electronic digitizers, sensors, receivers, touchpads, trackballs, cameras, microphones, keyboards, any other pointing devices, or any combinations thereof. The I/O interface may couple the computing machine to various output devices including video displays, speakers, printers, projectors, tactile feedback devices, automation control, robotic components, actuators, motors, fans, solenoids, valves, pumps, transmitters, signal emitters, lights, and so forth.

The computing machine may operate in a networked environment using logical connections through the network interface to one or more other systems or computing machines across the network. The network may include wide area networks (WAN), local area networks (LAN), intranets, the Internet, wireless access networks, wired networks, mobile networks, telephone networks, optical networks, or combinations thereof. The network may be packet switched, circuit switched, of any topology, and may use any communication protocol. Communication links within the network may involve various digital or an analog communication media such as fiber optic cables, free-space optics, waveguides, electrical conductors, wireless links, antennas, radio-frequency communications, and so forth. The processor may be connected to the other elements of the computing machine or the various peripherals discussed herein through the system bus. It should be appreciated that the system bus may be within the processor, outside the processor, or both. According to some embodiments, any of the processor, the other elements of the computing machine, or the various peripherals discussed herein may be integrated into a single device such as a system on chip ("SOC"), system on package ("SOP"), or ASIC device.

Embodiments may comprise a computer program that embodies the functions described and illustrated herein, wherein the computer program is implemented in a computer system that comprises instructions stored in a machine-readable medium and a processor that executes the instructions. However, it should be apparent that there could be many different ways of implementing embodiments in computer programming, and the embodiments should not be construed as limited to any one set of computer program instructions. Further, a skilled programmer would be able to write such a computer program to implement one or more of the disclosed embodiments described herein. Therefore, disclosure of a particular set of program code instructions is not considered necessary for an adequate understanding of how to make and use embodiments. Further, those skilled in the art will appreciate that one or more aspects of embodiments described herein may be performed by hardware, software, or a combination thereof, as may be embodied in one or more computing systems. Moreover, any reference to an act being performed by a computer should not be construed as being performed by a single computer as more than one computer may perform the act.

The example embodiments described herein can be used with computer hardware and software that perform the methods and processing functions described previously. The systems, methods, and procedures described herein can be embodied in a programmable computer, computer-executable software, or digital circuitry. The software can be stored on computer-readable media. For example, computer-readable media can include a floppy disk, RAM, ROM, hard disk, removable media, flash memory, memory stick, optical media, magneto-optical media, CD-ROM, etc. Digital circuitry can include integrated circuits, gate arrays, building block logic, field programmable gate arrays (FPGA), etc.

Reagents, tools, and/or instructions for performing the methods described herein can be provided in a kit. Such a kit can include reagents for collecting a tissue sample from a patient, such as by biopsy, and reagents for processing the tissue. Thus, the kit may include suitable fixatives, such as formalin and embedding reagents, such as paraffin. The kit can also include one or more reagents for performing an expression level analysis, such as reagents for performing nucleic acid amplification, including RT-PCR and qPCR, NGS (RNA-seq), northern blot, proteomic analysis, or immunohistochemistry to determine expression levels of biomarkers in a sample of a patient. For example, primers for performing RT-PCR, probes for performing northern blot analyses or bDNA assays, and/or antibodies or aptamers, as discussed herein, for performing proteomic analysis such as Western blot, immunohistochemistry and ELISA analyses can be included in such kits. Appropriate buffers for the assays can also be included. Detection reagents required for any of these assays can also be included. The kits may be array or PCR based kits for example and may include additional reagents, such as a polymerase and/or dNTPs for example. The kits featured herein can also include an instruction sheet describing how to perform the assays for measuring expression levels.

There is provided a kit for characterising and/or prognosing cancer in a subject comprising one or more primers and/or primer pairs for amplifying and/or which specifically hybridize with at least one gene, full sequence or target sequence selected from Table 1. There is also provided a kit for characterising and/or prognosing cancer in a subject comprising one or more probes that specifically hybridize with at least one gene, full sequence or target sequence selected from Table 1.

The kit may include one or more primer pairs and/or probes complementary to at least one gene selected from Table 1. The kits may include one or more probes or primers (primer pairs) designed to hybridize with the target sequences or full sequences listed in Table 1 and thus permit expression levels to be determined. The probes and probesets identified in table 1 and 1A may be employed according to all aspects of the invention. The primers and primer pairs identified in Table 1B may also be employed according to all aspects of the invention.

The kits may include primers/primer pairs/probes/probesets to form any of the gene signatures specified herein (see for example the gene signatures of Tables 1 to 24).

The kits may also include one or more primer pairs complementary to a reference gene.

Such a kit can also include primer pairs complementary to at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69 or 70 of the genes listed in Table 1.

Described herein is a kit for (in situ) characterising and/or prognosing prostate cancer in a subject comprising one or more oligonucleotide probes specific for an RNA product of at least one gene selected from Table 1. Suitable probes and probesets for each gene are listed in Table 1 and may be incorporated in the kits.. By "probeset" is meant the collection of probes designed to target (by hybridization) a single gene. The groupings are apparent from table 1 (and Table 1A).

The kit may further comprise one or more of the following components:

a) A blocking probe

b) A PreAmplifier

c) An Amplifier and/or

d) A Label molecule

The components of the kit may be suitable for conducting a viewRNA assay (https://www.panomics.com/products/rna-in-situ-analysis/view-rna-overview).

The components of the kit may be nucleic acid based molecules, optionally DNA (or RNA). The blocking probe is a molecule that acts to reduce background signal by binding to sites on the target not bound by the target specific probes (probes specific for the RNA product of the at least one gene). The PreAmplifier is a molecule capable of binding to a (a pair of) target specific probe(s) when target bound. The Amplifier is a molecule capable of binding to the PreAmplifier. Alternatively, the Amplifier may be capable of binding directly to a (a pair of) target specific probe(s) when target bound. The Amplifier has binding sites for multiple label molecules (which may be label probes).

Kits for characterising and/or prognosing cancer, such as prostate cancer or ER positive breast cancer in a subject may permit the methylation status of at least one gene selected from Table 1 to be determined. The determined methylation status, which may be hypermethylation or hypomethylation as appropriate, is used to provide a characterisation of and/or a prognosis for the cancer, such as prostate cancer or ER positive breast cancer. Such kits may include primers and/or probes for determining the methylation status of the gene or genes directly. They may thus comprise methylation specific primers and/or probes that discriminate between methylated and unmethylated forms of DNA by hybridization. Such primers and/or probes may include derivatives of the primers and probes described herein, which are adapted to reflect selective modification of the cytosine residues in the target sequence depending upon whether they are methylated or not. Thus, sets of "methylated-specific" and "unmethylated-specific" primers (to include primer pairs) and probes may be designed in order to probe particular cytosine-containing target sequences. Such kits will typically also contain a reagent that selectively modifies either the methylated or non-methylated form of CpG dinucleotide motifs. Suitable chemical reagents comprise hydrazine and bisulphite ions. An example is sodium bisulphite. The kits may, however, contain other reagents as discussed hereinabove to determine methylation status such as restriction endonucleases. Methylation specific PCR primers may be derived from the primer pairs of Table 1B and of SEQ ID NOs 3151-3154, to take account of bisulphite conversion of CpG dinucleotide pairs if present in the unmethylated form (unmethylated-specific) or lack of conversion if the CpG dinucleotide is methylated (methylated-specific).

Described herein is a kit for characterising and/or prognosing cancer, such as prostate cancer or ER positive breast cancer in a subject comprising one or more antibodies or aptamers as described above and which are useful in the methods of the invention.

Informational material included in the kits can be descriptive, instructional, marketing or other material that relates to the methods described herein and/or the use of the reagents for the methods described herein. For example, the informational material of the kit can contain contact information, e.g., a physical address, email address, website, or telephone number, where a user of the kit can obtain substantive information about performing a gene expression analysis and interpreting the results.

The kit may further comprise a computer application or storage medium as described above.

Although specific embodiments have been described above in detail, the description is merely for purposes of illustration. It should be appreciated, therefore, that many aspects described above are not intended as required or essential elements unless explicitly stated otherwise.

## DESCRIPTION OF THE FIGURES

Figure 1: Heat map showing unsupervised hierarchical clustering of gene expression data using the 1000 most variable genes in the 126 prostate FFPE tumour samples. Gene expression across all samples is represented horizontally. Functional processes corresponding to each gene cluster are labeled along the right of the figure.

Figure 2: AUC calculated under cross validation with respect to associating the signature scores with discriminating the molecular subgroups (cluster 1 and 2 V cluster 3 and 4). The number of genes in each signature is depicted along the x-axis and the AUC on the y-axis.

Figure 3: C-index calculated under cross validation with respect to associating the signature scores with time to metastatic recurrence in the Taylor primary tumour samples. The number of genes in each signature is depicted along the x-axis and the C-index on the y-axis.

Figure 4: Standard Deviation (SD) calculated as a percentage of the signature score range under cross validation within the five sections that were profiled to evaluate the impact of biological heterogeneity on signature score The number of genes in each signature is depicted along the x-axis and the percent SD on the y-axis.

Figure 5: Kaplan Meier generated in the Taylor primary tumour samples using the time to metastatic recurrence endpoint and the Good/Poor prognosis 70 gene signature predictions. Univariate hazard ratio = 0.62 [1.98,20.20]; p < 0.0001

Figure 6: Kaplan Meier generated in the Taylor primary tumour samples using the time to biochemical recurrence endpoint and the Good/Poor prognosis 70 gene signature predictions. Univariate hazard ratio = 3.76 [1.70, 8.34]; p < 0.0001

Figure 7: Wald test of multivariate Cox analysis of key prognostic factors from Taylor analysis

Figure 8A: ROC curve in the Glinsky data using the 70 gene signature scores and the corresponding biochemical recurrence outcome for each patient. The AUC = 0.69 [0.57, 0.79]; p = 0.0032.

Figure 8B: ROC curve in the Erho data using the 70 gene signature scores and the corresponding metastatic recurrence outcome for each patient. The AUC = 0.61 [0.57, 0.65]; p < 0.0001.

Figure 9: Kaplan Meier generated in the breast cancer data (GSE2034) ER positive tumour samples using the time to relapse endpoint (time in months) and the Good/Poor prognosis 70 gene signature predictions; signature_call_median 1 (poor prognosis) and signature_call_median 0 (good prognosis). Univariate hazard ratio = 1.24 [0.80, 1.92]

Figure 10: ROC curve in the breast cancer data (GSE2034) ER positive tumour samples using the 70 gene signature scores and the corresponding recurrence outcome for each patient. The AUC = 0.62; p = 0.002

Figure 11: Kaplan Meier generated in the breast cancer data (GSE7390) ER positive tumour samples using the relapse free survival endpoint (time in days) and the Good/Poor prognosis 70 gene signature predictions; signature_call_median 1 (poor prognosis) and signature_call_median 0 (good prognosis). Univariate hazard ratio = 1.74 [1.04, 2.93]

Figure 12: Kaplan Meier generated in the breast cancer data (GSE7390) ER positive tumour samples using the distant metastasis free survival endpoint (time in days) and the Good/Poor prognosis 70 gene signature predictions; signature_call_median 1 (poor prognosis) and signature_call_median 0 (good prognosis). Univariate hazard ratio = 2.01 [1.02, 3.96]

Figure 13: Kaplan Meier generated in the breast cancer data (GSE7390) ER positive tumour samples using the overall survival endpoint (time in days) and the Good/Poor prognosis 70 gene signature predictions; signature_call_median 1 (poor prognosis) and signature_call_median 0 (good prognosis). Univariate hazard ratio = 2.54 [1.24, 5.18]

Figure 14: Kaplan Meier generated in the breast cancer data (GSE2990) ER positive tumour samples using the relapse free survival endpoint (time in years) and the Good/Poor prognosis 70 gene signature predictions; signature_call_median 1 (poor prognosis) and signature_call_median 0 (good prognosis). Univariate hazard ratio = 1.91 [1.17, 3.09]

Figure 15: Kaplan Meier generated in the breast cancer data (GSE2990) ER positive tumour samples using the distant metastasis free survival endpoint (time in years) and the Good/Poor prognosis 70 gene signature predictions; signature_call_median 1 (poor prognosis) and signature_call_median 0 (good prognosis). Univariate hazard ratio = 2.37 [1.26, 4.44]

Figure 16 - Kaplan Meier survival analysis over 10-years showing the association of the 70-gene signature at predicting time to biochemical recurrence in the resection validation cohort following surgery. Surivival probability (%) showed reduced progression-free survival (PFS) in months of the 'Met-like' subgroup (blue) of 81 patients when compared to the 'Non Met-like' subgroup (green) of 241 patients (HR = 1.74 [1.18-2.56]; p = 0.0009).

Figure 17 - Kaplan Meier survival analysis over 10-years showing the association of the 70-gene signature at predicting time to metastatic disease progression in the resection validation cohort following surgery. Surivival probability (%) showed reduced progression-free survival (PFS) in months of the 'Met-like' subgroup (blue) of 81 patients when compared to the 'Non Met-like' subgroup (green) of 241 patients (HR = 3.60 [1.81-7.13]; p < 0.0001).

Figure 18 - Kaplan Meier survival analysis over 10-years showing the association of the 70-gene signature at predicting time to biochemical recurrence in the FASTMAN biopsy validation cohort following curative radiotherapy. Surivival probability (%) showed reduced progression-free survival (PFS) in months of the 'Met-like' subgroup (blue) of 54 patients when compared to the 'Non Met-like' subgroup (green) of 194 patients (HR = 2.18 [1.14-4.17]; p = 0.0042).

Figure 19 - Kaplan Meier survival analysis over 10 years showing the association of the 70-gene signature at predicting time to metastatic disease progression in the FASTMAN biopsy validation cohort following radiotherapy with curative intent. Surivival probability (%) showed reduced progression-free survival (PFS) in months of the 'Met-like' subgroup (blue) of 54 patients when compared to the 'Non Met-like' subgroup (green) of 194 patients (HR = 3.50 [1.28-9.56]; p= 0.0017).

Figure 20 - Core set analysis for FASTMAN Biopsy Validation dataset.

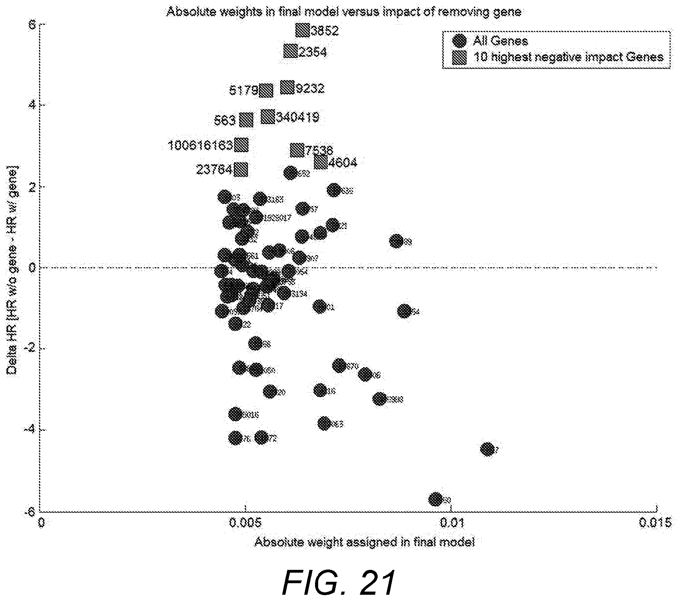

Figure 21 - Core set analysis for internal resection validation dataset.

Figure 22 - Minimum gene set analysis for FASTMAN Biopsy Validation dataset.

Figure 23 - Minimum gene set analysis for internal resection validation dataset.

**EXAMPLES**

The present invention will be further understood by reference to the following experimental examples.

**Example 1: Tissue processing, hierarchical clustering and subtype identification**

***Tumor Material***

70 primary prostate cancers with no known concomitant metastases, 20 primary prostate cancers with known lymph node metastases, 11 lymph nodes containing metastatic prostate cancer, 25 normal prostate samples.

***Gene Expression Profiling from FFPE***

Total RNA was extracted from macrodissected FFPE tissue using the High Pure RNA Paraffin Kit (Roche Diagnostics GmbH, Mannheim, Germany). RNA was converted into complementary deoxyribonucleic acid (cDNA), which was subsequently amplified and converted into single-stranded form using the SPIA® technology of the WT-Ovation™ FFPE RNA Amplification System V2 (NuGEN Technologies Inc., San Carlos, CA, USA). The amplified single-stranded cDNA was then fragemented and biotin labeled using the FL-Ovation™ cDNA Biotin Module V2 (NuGEN Technologies Inc.). The fragmented and labeled cDNA was then hybridized to the Almac Prostate Cancer DSA™. Almac's Prostate Cancer DSA™ research tool has been optimised for analysis of FFPE tissue samples, enabling the use of valuable archived tissue banks. The Almac Prostate Cancer DSA™ research tool is an innovative microarray platform that represents the transcriptome in both normal and cancerous prostate tissues. Consequently, the Prostate Cancer DSA™ provides a comprehensive representation of the transcriptome within prostate disease and tissue setting, not available using generic microarray platforms. Arrays were scanned using the Affymentrix Genechip® Scanner 7G (Affymetrix Inc., Santa Clara, CA).

***Data preparation***

Quality Control (QC) of profiled samples was carried out using MAS5 pre-processing algorithm. Various technical aspects were assessed including: average noise and background homogeneity, percentage of present call (array quality), signal quality, RNA quality and hybridization quality. Distributions and Median Absolute Deviation of corresponding parameters were analyzed and used to identify possible outliers.

Almac's Prostate Cancer DSA™ contains probes that primarily target the area within 300 nucleotides from the 3' end. Therefore standard Affymetrix RNA quality measures were adapted - for housekeeping genes intensities of 3' end probe sets with ratios of 3' end probe set intensity to the average background intensity were used in addition to usual 3'/5' ratios. Hybridization controls were checked to ensure that their intensities and present calls conform to the requirements specified by Affymetrix.

***Hierarchical Clustering and Functional Analysis***

Sample pre-processing was carried out using Robust Multi-Array analysis (RMA) [1]. The data matrix was initially summarised to Entrez gene ID level using Ensemble annotation version 75, specifically ustilising the probe set that was least associated to present call for each Entrez gene. Probe sets that 1) did not map to an Entrez gene ID or 2) mapped to multiple Entrez gene IDs were removed. The resulting gene level data matrix was sorted by decreasing variance and intensity and incremental subsets of the data matrix were tested for cluster stability: the GAP statistic [2] was applied to calculate the number of sample and gene clusters while the stability of cluster composition was assessed using partition comparison methods. The final most variable gene list was determined based on the smallest and most stable data matrix for the selected number of sample cluster.

Following standardization of the data matrix to the median gene expression values, agglomerative hierarchical clustering was performed using Euclidean distance and Ward's linkage method [3]. The optimal number of sample and gene clusters was determined using the GAP statistic [2] which compares the change in with-cluster dispersion with that expected under a reference null distribution. The significance of the distribution of clinical parameter factor levels across sample clusters was assessed using ANOVA (continuous factor) or chi-squared analysis (discrete factor) and corrected for false discovery rate (product of p-value and number of tests performed). A corrected p-value threshold of 0.05 was used as criterion for significance.

Functional enrichment analysis was conducted to identify and rank biological entities which were found to be associated with the clustered gene sets using the Gene Ontology biological processes classification [4]. Entities were ranked according to a statistically derived enrichment score [5] and adjusted for multiple testing [6]. A corrected p-value of 0.05 was used as significance threshold. The identified enriched processes were summarised into an overall group function for each gene cluster.

From the hierarchical clustering analysis, primary tumour samples clustering with metastatic samples will be labelled as 'bad whereas primary tumour samples clustering with normal samples will be labelled as 'good'.

## *Signature generation*

Following the identification of class labels a gene signature was derived to enable prospective identification of the bad prognosis group within the primary tumour samples. The following steps summarise the procedure for developing the gene signature:

1. Cross-validation: The samples were randomly split into 5 cross-validation (CV) folds for signature training/testing, and this was repeated 10 times to allow an unbiased estimation of the model performance.
2. Pre-processing: RMA background correction of the data at the probe intensity level, followed by a median summary of the intensities of probes to probe sets and subsequently probe sets to Entrez gene ID. The Entrez gene level summarised data matrix was log2 transformed and quantile normalised. Note that samples in the CV test set were normalised using a quantile normalisation model from the corresponding CV training set to ensure that all estimates of model performance are based on signature scores pre-processed on a per sample basis.
3. Filtering: A gene filter was applied before model development to remove 75 percent of genes with low variance and low intensity.
4. Machine Learning: Partial Least Squares (PLS) was used to train the algorithm against the "good/poor prognosis" endpoint.
5. Feature Selection: A wrapper based method for feature selection was implemented, where genes (those remaining after the initial filter) are ranked using the respective weights defined by the PLS algorithm and 10 percent of genes with the lowest absolute weights are removed. This process is repeated after each round of feature elimination (within cross validation) where the genes are re-ranked in order to determine the genes with the lowest absolute weights and removing 10 percent each time until only 2 genes remained.
6. Interim validation data set 1: A public data set (Taylor et al) was used for interim evaluation were the primary tumour samples from this data set were predicted (signature scores calculated) alongside each CV test set.
7. Interim validation data set 2: Five sections across an FFPE tumour block were profiled in order to evaluate the impact of biological heterogeneity on the signature score. Signature scores for each of these sections were calculated under CV alongside each CV test set.

Model selection included the following steps:

1. Evaluating the Area Under the Receiver Operating Characteristic (ROC) Curve (AUC) in the training data under cross validation.
2. Evaluating the C-index in the interim validation Taylor data under cross validation. The C-index is a measure of performance (analogous to AUC) relating to predicting time-to-event data in absence of the threshold for dichotomising the scores for assigning "good" and "poor" prognosis groups.
3. Evaluating the variability in signature scores across the five sections of an FFPE block which were predicted under CV. The variability was determined by calculating the standard deviation (SD) of the signature scores across the five samples and expressing the SD as a fraction of the signature score range (i.e. calculating a percent SD).

The signature length that yielded a high AUC in training set; a high C-index in the Taylor set; and a low SD in the heterogeneity samples was selected.

Multivariate analysis

Of interest is the time until biochemical recurrence in prostate cancer patients in the Taylor dataset. Multivariable Cox survival modelling was used to test for and describe interactions with the biomarker, understand prognostic factors and model the relative effect of prognostic factors. Based on clinical judgement pre-operative PSA (4 ng/ml), pathology stage ("T2 A/B/C","T3 A/B/C" ,"T4"), Gleason (<7, 7, 8-9) and the dichotomised signature score were used as independent predictor variables. A log 2 transformation of pre-operative PSA was applied. Multiple imputation was used to ensure all available events were used in the analysis. The sample size is 168 patients with 46 biochemical recurrence events and the median time until biochemical recurrence approximately 15 years. A formal test of the proportional hazard assumption, assessment of the functional form of the log transformation of Pre PSA and the model fit using a graphical plot of the Nelson-Aalen cumulative hazard function all provided no cause for concern. Twelve influential data points defined by a change to the regression coefficient equal to or greater than 2 standard errors on removal from the analysis were identified. These were not removed or investigated further.

Following model selection two independent prostate cancer data sets were further evaluated with the final model:

1. 70 publically available primary prostate tumour samples (Glinsky et al) which were profiled on the Affymetrix U133A platform.

    a. Clinical information included biochemical recurrence (as a binary outcome only)

2. 545 publically available primary prostate tumour samples (Erho et al 2013) which were profiled on the Affymetrix Human Exon array platform.

    a. Clinical information included metastatic recurrence (as a binary outcome only)

Performance of each of these data sets was evaluated using AUC, to establish if the signature could discriminate patients with recurrences from those with no recurrences, under the hypothesis that higher scores are more representative of patients with metastatic-like disease (bad prognosis) therefore more likely to have a recurrence outcome.

***Evaluation of the final model in breast cancer data sets***

It was of further interest to evaluate the final signature in other hormone related data sets with respect to predicting prognosis in untreated patients. Three ER positive breast cancer data sets were evaluated:

1. Data set retrieved from Gene Expression Omnibus database, accession number GSE2034

    a. 209 Node negative ER positive patients
    b. Endpoint: Time to relapse

2. Data set retrieved from Gene Expression Omnibus database, accession number GSE7390

    a. 134 Node negative ER positive patients
    b. Endpoint 1: relapse free survival (RFS)
    c. Endpoint 2: distant metastasis free survival (DMFS)
    d. Endpoint 3: overall survival (OS)

3. Data set retrieved from Gene Expression Omnibus database, accession number GSE2990

    a. 149 ER positive patients
    b. Endpoint 1: relapse free survival (RFS)
    c. Endpoint 2: distant metastasis free survival (DMFS)

For each data set a median signature score cut-off was applied to predict patients as either signature positive (metastatic-like) if they scored above the median value, or signature negative (non-metastatic-like) otherwise. Kaplan Meier curve was used to observe the survival differences between the two subgroups of patients. Cox proportional hazard regression analysis of the signature calls against each endpoint was used to calculate a univariate hazard ratio for the signature as a measure of performance against the respective clinical endpoint.

***Results***

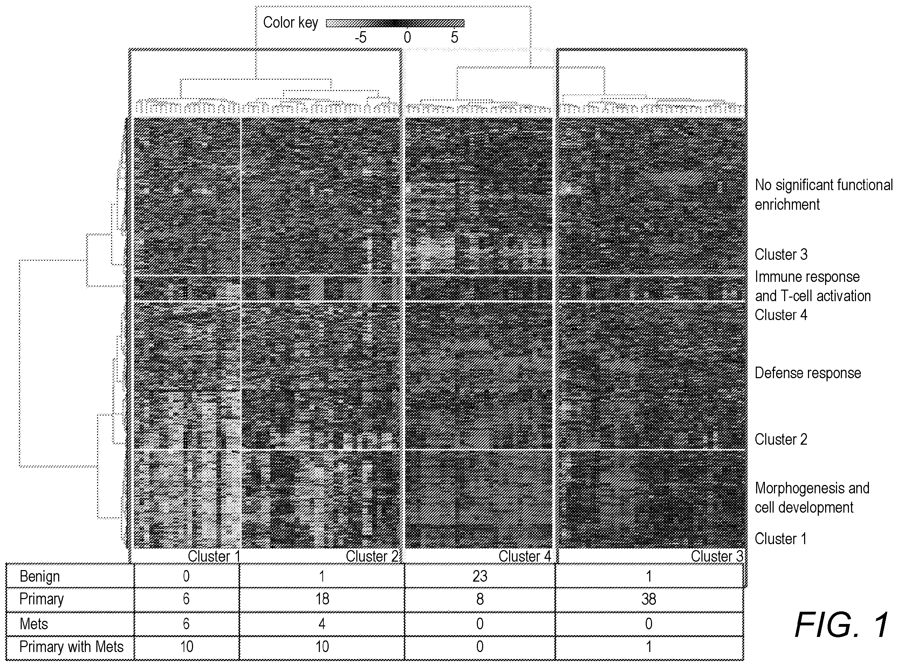

126 samples passed microarray QC and subsequently underwent unsupervised hierarchical clustering based on 1000 most variable genes. Four sample clusters and four gene clusters were identified (Figure 1). There was a significant association between sample clusters and tumour type: cluster 1 and 2 (highlighted with blue box) comprised mainly metastatic and primary tumours and cluster 3 (highlighted with red box) and 4 (highlighted with yellow box) comprised benign and primary tumours respectively (p < 0.0001, Table 1). Functional analysis (Figure 1) revealed that clusters 1 and 2 (metastatic and primary like metastatic tumours) were characterized by down-regulation of genes associated with cell adhesion, cell differentiation and cell development, up-regulation of Androgen related processes and Epithelial to mesenchymal transition (EMT) (cluster 1 and 2 referred to as "bad prognosis" group forthwith). Cluster 3 and cluster 4 (benign and primary like benign tumours) were associated with up-regulation of genes associated with cell adhesion, inflammatory responses and cell development (cluster 3 and cluster 4 referred to as "good prognosis" forthwith). Patients in cluster 1 and cluster 2 were class labelled "bad prognosis" and patients in cluster 3 and cluster 4 were class labelled as "good prognosis" for the purpose of signature development.

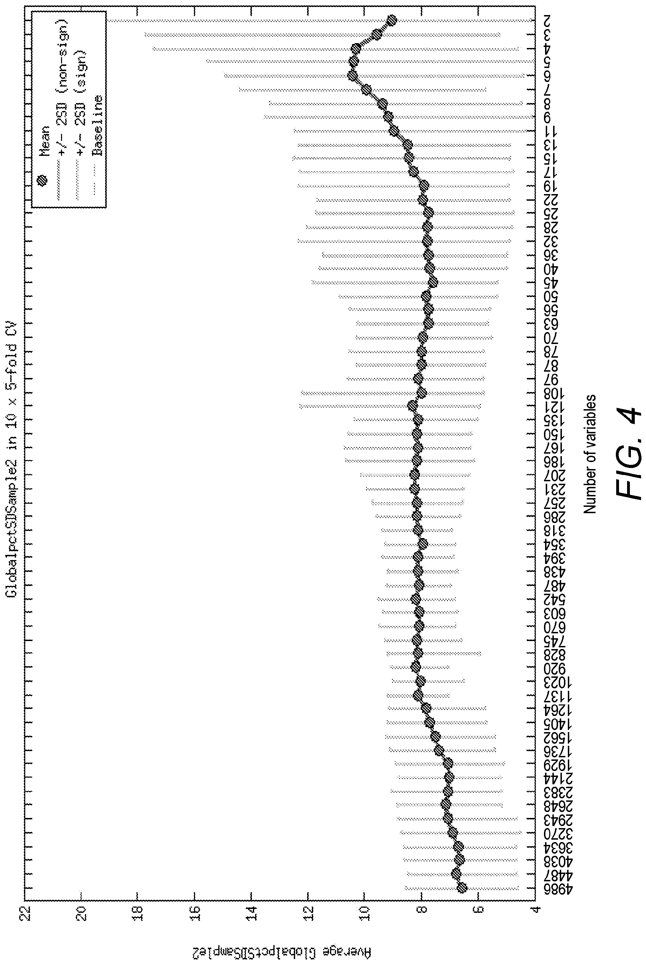

The results from signature development at all considered signature lengths are provided in Figure 2, Figure 3 and Figure 4 which respectively show; the AUC in the training set for predicting the endpoint; the C-index in the Taylor data with respect to time to metastatic recurrence; and the percent SD in the heterogeneity samples. A signature length of 70 genes was selected as this was the signature length whereby the AUC remained high (Figure 2); the SD remained low (Figure 4); and is the smallest signature length were the c-index values remained high in the Taylor samples (Figure 3).

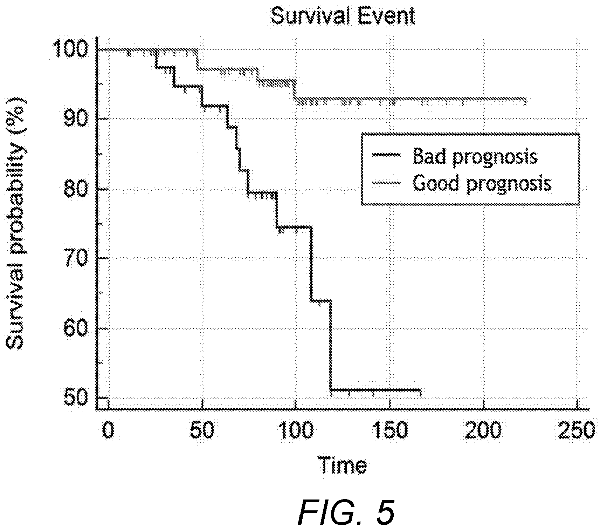

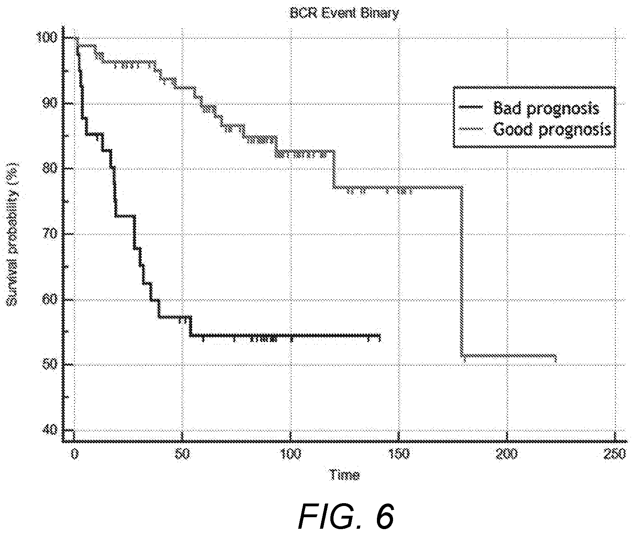

The signature content and weightings of the final 70 gene model are listed in Table 1. The 70 gene scores calculated in the Taylor data were dichotomised at a threshold of 0.4241 where patients with a signature score > 0.4241 were classified as "bad prognosis" and patients with a signature score $\leq$ 0.4241 were classified as "good prognosis". The signature classifications into good and poor prognosis were used to generate a Kaplan Meier curve to show the differences in survival probabilities for the two predicted groups. Figure 5 represents the Kaplan Meier for the time to metastatic recurrence endpoint (univariate hazard ratio = 6.32 [1.98, 20.20]) and Figure 6 represents the Kaplan Meier for the time to biochemical recurrence endpoint (univariate hazard ratio = 3.76 [1.70, 8.34]).

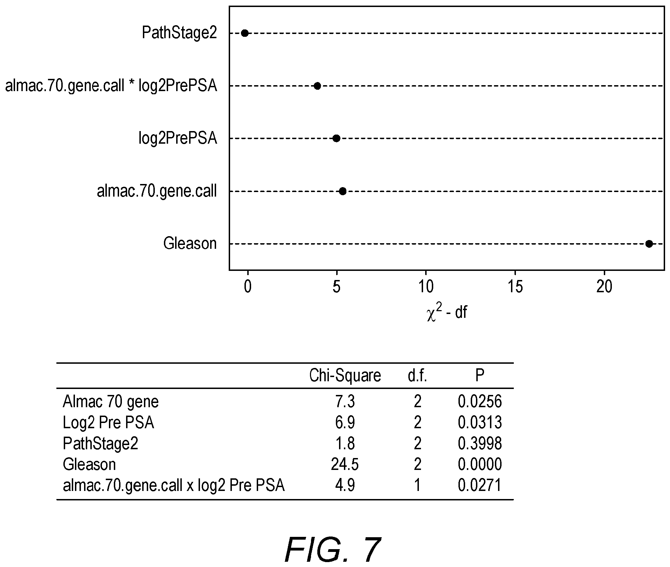

Figure 7 and the associated table present the results of the multivariable analysis. The plot displays the Wald chi squared statistic minus its degrees of freedom for assessing the partial effect of each variable in the model. Gleason is the most important factor followed by the biomarker (i.e gene signature) and pre-operative PSA. These results demonstrate that the biomarker provides additional prognostic information over and above standard pathological factors. Due to the interaction of the biomarker and pre-operative PSA, one potential would be to combine these variables (and/or other prognostic factors) together to generate a combined risk score. The 70 gene signature model was applied to two independent prostate cancer data sets.

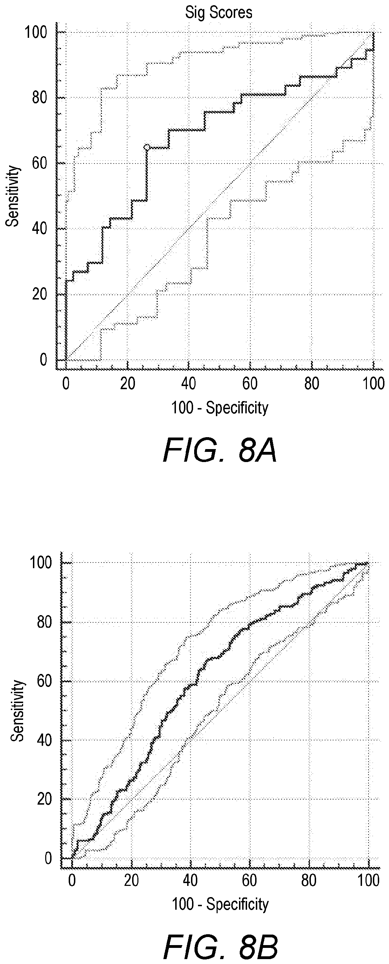

Figure 8A and Figure 8B show the ROC curves from assessing the signature scores against the recurrence outcomes for the Glinksy and the Erho data sets respectively. The AUC in the Glinsky data for predicting biochemical recurrence was 0.69 [0.57, 0.79] and the AUC in the Erho data for predicting metastatic recurrence was 0.61 [0.57, 0.65].

***Evaluation of the final model in breast cancer data sets***

The results of evaluating the 70 gene signature in three breast cancer data sets is described below:

1. Data set retrieved from Gene Expression Omnibus database, accession number GSE2034

   a. 209 Node negative ER positive patients
   b. Endpoint: Time to relapse

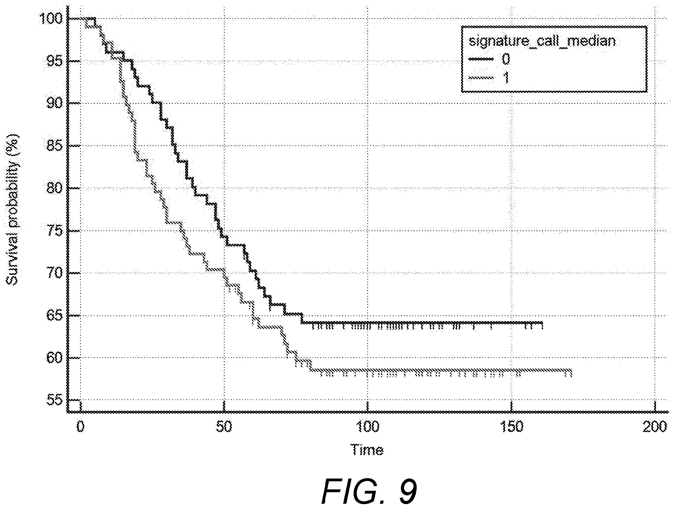

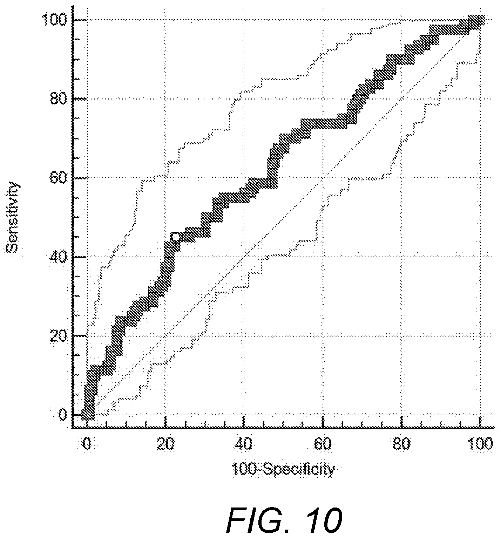

   i. Hazard ratio = 1.24 [0.80, 1.92] (Kaplan Meier is shown in Figure 9)
   ii. AUC for predicting relapse = 0.62; p=0.002 (ROC curve shown in Figure 10)

2. Data set retrieved from Gene Expression Omnibus database, accession number GSE7390

   a. 134 Node negative ER positive patients
   b. Endpoint 1: relapse free survival (RFS)

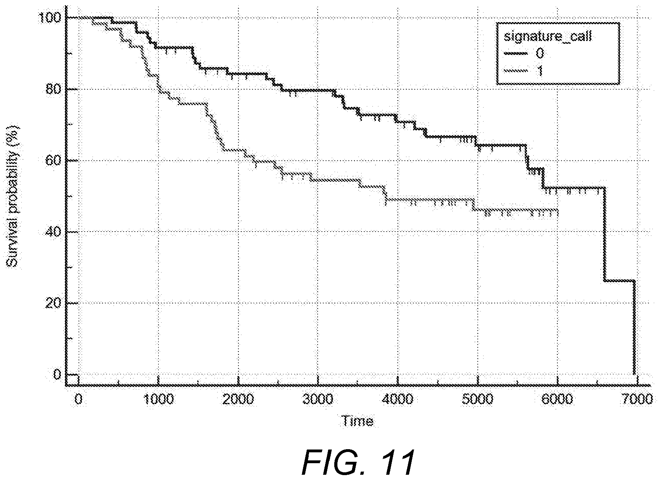

   i. Hazard ratio = 1.74 [1.04, 2.93] (Kaplan Meier is shown in Figure 11)

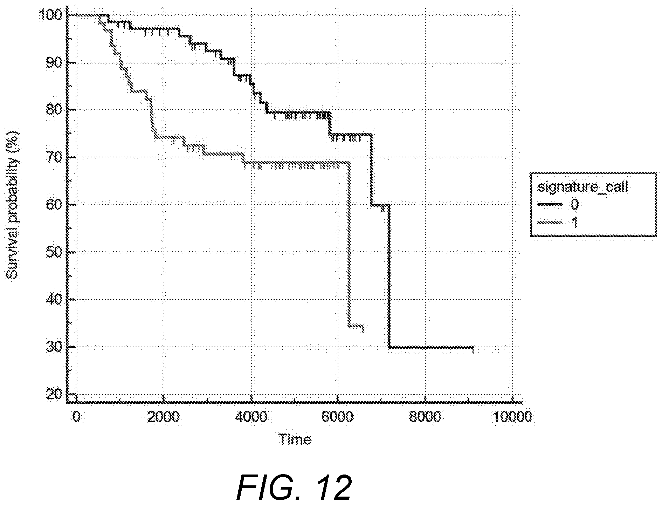

   c. Endpoint 2: distant metastasis free survival (DMFS) i. Hazard ratio = 2.01 [1.02, 3.96] (Kaplan Meier is shown in Figure 12)
   d. Endpoint 3: overall survival (OS)

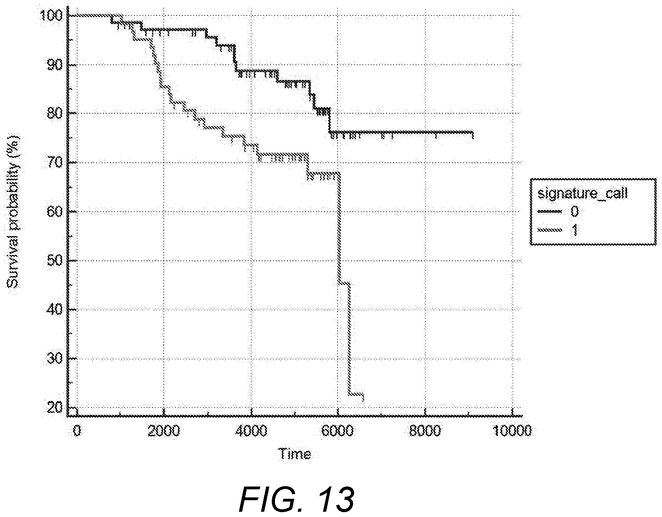

   i. Hazard ratio = 2.54 [1.24, 5.18] (Kaplan Meier is shown in Figure 13)

3. Data set retrieved from Gene Expression Omnibus database, accession number GSE2990

   a. 149 ER positive patients

b. Endpoint 1: relapse free survival (RFS)

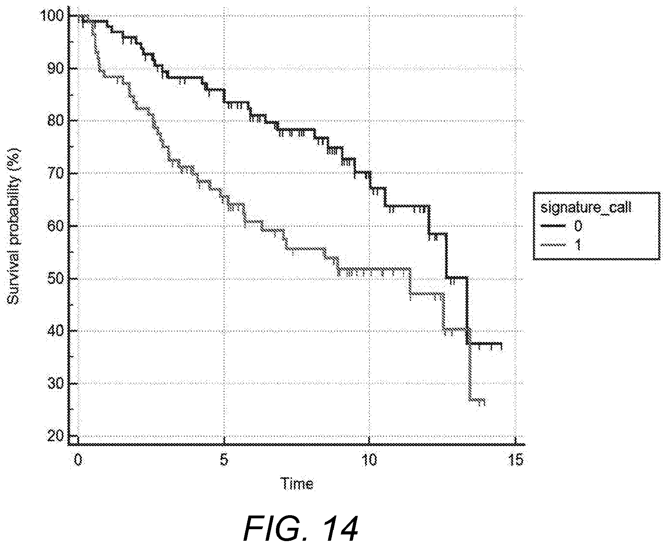

   i. Hazard ratio = 1.91 [1.17, 3.09] (Kaplan Meier is shown in Figure 14)

c. Endpoint 2: distant metastasis free survival (DMFS)

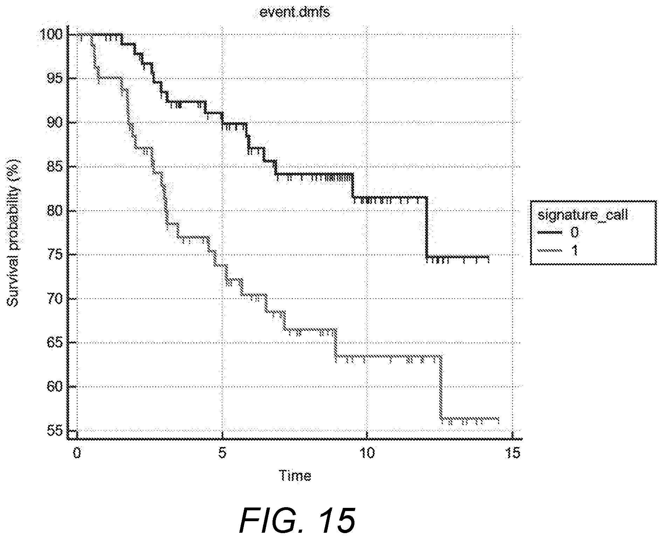

   i. Hazard ratio = 2.37 [1.26, 4.44] (Kaplan Meier is shown in Figure 15)

**References**

1. Irizarry RA, Bolstad BM, Collin F, Cope LM, Hobbs B, Speed TP. Summaries of Affymetrix GeneChip probe level data. Nucleic acids research 2003;31:e15.

2.Tibshirani R, Walther G, Hastie T. Estimating the number of clusters in a data set via the gap statistic. J Roy Stat Soc B 2001 ;63:411-23.

3. Ward JH. Hierarchical Grouping to Optimize an Objective Function. Journal of the American Statistical Association 1963;58:236-&.

4. Ashburner M, Ball CA, Blake JA, et al. Gene ontology: tool for the unification of biology. The Gene Ontology Consortium. Nature genetics 2000;25:25-9.

5. Cho RJ, Huang MX, Campbell MJ, et al. Transcriptional regulation and function during the human cell cycle. Nature genetics 2001 ;27:48-54.

6. Benjamini Y, Hochberg Y. Controlling the False Discovery Rate - a Practical and Powerful Approach to Multiple Testing. J Roy Stat Soc B Met 1995;57:289-300.

**Example 2** - **Confirmation of effectiveness of all probesets**

**Purpose:**

The purpose of this analysis is to evaluate the performance of the 70 gene signature when a random probeset per gene is selected. This is to provide evidence of the importance of certain probesets associated to the signature genes.

**Data:**

Table 26 outlines the number of probesets available per signature gene. The table shows that the number of probesets that can be selected per gene varies from 1 to a maximum of 21 probesets per gene.

**Table 26** - **Number of probesets available per signature gene**

| Entrez Gene ID | Signature Weight | Signature Bias | Weight (abs) | Rank by Weight | # Probesets |
|---|---|---|---|---|---|
| 827 | -0.01090 | 4.44087 | 0.01090 | 1 | 1 |
| 7060 | -0.00963 | 6.91259 | 0.00963 | 2 | 1 |
| 5354 | -0.00889 | 4.38357 | 0.00889 | 3 | 2 |
| 4489 | -0.00868 | 6.74796 | 0.00868 | 4 | 2 |
| 406988 | -0.00828 | 7.21525 | 0.00828 | 5 | 4 |
| 6406 | -0.00793 | 4.23042 | 0.00793 | 6 | 1 |
| 84870 | -0.00730 | 4.29317 | 0.00730 | 7 | 2 |
| 50636 | -0.00716 | 6.52255 | 0.00716 | 8 | 5 |
| 5121 | -0.00714 | 7.62176 | 0.00714 | 9 | 1 |

(continued)

| Entrez Gene ID | Signature Weight | Signature Bias | Weight (abs) | Rank by Weight | # Probesets |
|---|---|---|---|---|---|
| 27063 | -0.00692 | 5.92831 | 0.00692 | 10 | 1 |
| 4604 | -0.00684 | 4.57432 | 0.00684 | 11 | 8 |
| 4316 | -0.00684 | 6.75672 | 0.00684 | 12 | 1 |
| 12 | -0.00683 | 5.74546 | 0.00683 | 13 | 3 |
| 6401 | -0.00681 | 5.97768 | 0.00681 | 14 | 1 |
| 3852 | -0.00640 | 6.08049 | 0.00640 | 15 | 1 |
| 4057 | -0.00640 | 6.49726 | 0.00640 | 16 | 3 |
| 57481 | -0.00638 | 3.55997 | 0.00638 | 17 | 1 |
| 25907 | -0.00631 | 8.06342 | 0.00631 | 18 | 1 |
| 7538 | -0.00627 | 9.96083 | 0.00627 | 19 | 1 |
| 2354 | -0.00611 | 6.95494 | 0.00611 | 20 | 4 |
| 50652 | -0.00610 | 5.26234 | 0.00610 | 21 | 8 |
| 79054 | -0.00606 | 4.86579 | 0.00606 | 22 | 14 |
| 9232 | 0.00602 | 4.71269 | 0.00602 | 23 | 2 |
| 283194 | -0.00595 | 4.98038 | 0.00595 | 24 | 18 |
| 9506 | -0.00584 | 7.07391 | 0.00584 | 25 | 1 |
| 79689 | -0.00568 | 8.10530 | 0.00568 | 26 | 4 |
| 130733 | -0.00565 | 7.59453 | 0.00565 | 27 | 1 |
| 2920 | -0.00560 | 8.92898 | 0.00560 | 28 | 1 |
| 9955 | -0.00559 | 4.23278 | 0.00559 | 29 | 3 |
| 2138 | -0.00558 | 5.50428 | 0.00558 | 30 | 5 |
| 340419 | -0.00556 | 3.92242 | 0.00556 | 31 | 2 |
| 5317 | -0.00555 | 5.91219 | 0.00555 | 32 | 2 |
| 4588 | -0.00552 | 6.64004 | 0.00552 | 33 | 1 |
| 5179 | -0.00551 | 4.51486 | 0.00551 | 34 | 2 |
| 1672 | -0.00540 | 6.82549 | 0.00540 | 35 | 2 |
| 84889 | -0.00539 | 4.64900 | 0.00539 | 36 | 1 |
| 693163 | -0.00536 | 5.08739 | 0.00536 | 37 | 1 |
| 51050 | -0.00526 | 4.85872 | 0.00526 | 38 | 6 |
| 101928017 | -0.00526 | 6.06588 | 0.00526 | 39 | 1 |
| 5166 | -0.00525 | 4.17409 | 0.00525 | 40 | 12 |
| 644844 | -0.00521 | 5.18357 | 0.00521 | 41 | 1 |
| 5054 | -0.00519 | 6.69187 | 0.00519 | 42 | 6 |
| 29951 | -0.00515 | 4.75233 | 0.00515 | 43 | 4 |
| 7739 | -0.00511 | 6.90054 | 0.00511 | 44 | 1 |
| 152 | -0.00505 | 7.07838 | 0.00505 | 45 | 1 |
| 563 | -0.00502 | 8.19118 | 0.00502 | 46 | 3 |
| 7083 | 0.00497 | 5.58133 | 0.00497 | 47 | 1 |

(continued)

| Entrez Gene ID | Signature Weight | Signature Bias | Weight (abs) | Rank by Weight | # Probesets |
|---|---|---|---|---|---|
| 23784 | -0.00496 | 4.82498 | 0.00496 | 48 | 4 |
| 3832 | 0.00493 | 3.91767 | 0.00493 | 49 | 2 |
| 9076 | -0.00492 | 4.96028 | 0.00492 | 50 | 6 |
| 100616163 | -0.00491 | 10.53645 | 0.00491 | 51 | 1 |
| 23764 | -0.00490 | 8.49795 | 0.00490 | 52 | 3 |
| 91661 | -0.00486 | 3.97633 | 0.00486 | 53 | 2 |
| 1164 | 0.00486 | 6.50398 | 0.00486 | 54 | 1 |
| 56849 | -0.00486 | 4.81933 | 0.00486 | 55 | 2 |
| 5346 | 0.00483 | 4.62939 | 0.00483 | 56 | 1 |
| 6614 | 0.00477 | 5.50375 | 0.00477 | 57 | 1 |
| 285016 | -0.00477 | 6.66460 | 0.00477 | 58 | 1 |
| 8076 | -0.00477 | 4.12918 | 0.00477 | 59 | 2 |
| 6422 | -0.00476 | 7.90126 | 0.00476 | 60 | 2 |
| 1847 | -0.00472 | 5.76268 | 0.00472 | 61 | 3 |
| 57176 | 0.00468 | 5.22346 | 0.00468 | 62 | 1 |
| 10257 | -0.00466 | 5.23038 | 0.00466 | 63 | 21 |
| 23677 | -0.00462 | 4.88271 | 0.00462 | 64 | 9 |
| 6652 | -0.00457 | 8.95841 | 0.00457 | 65 | 4 |
| 51001 | 0.00452 | 5.33420 | 0.00452 | 66 | 1 |
| 1803 | -0.00451 | 4.65975 | 0.00451 | 67 | 6 |
| 284837 | 0.00450 | 4.90531 | 0.00450 | 68 | 1 |
| 54097 | -0.00444 | 7.38807 | 0.00444 | 69 | 3 |
| 354 | -0.00442 | 10.22644 | 0.00442 | 70 | 5 |

**Analysis:**

The following analysis steps were performed:

- Training data matrix pre-processing (n=126 samples)

  ◦ RMA background correction
  ◦ Quantile normalisation
  ◦ RMA summary

- Generate signature scores for training samples using a random probeset which is annotated to each signature gene, 1000 times
- Calculate AUC performance using the signature scores with respect to the subtype labels
- Min(AUC) = 0.9964 & Max(AUC) = 1.00
- This indicates that all probesets are effective in the signature for identifying the subtype

For completeness, it is noted that the random selection of probeset per signature gene will only be applicable for signature genes with > 1 probeset i.e. 30 of the signature genes have only 1 probeset per gene, so for these genes, the same probeset is being selected each time.

**Example 3** - **Validation study for 70 gene signature**

**Introduction**

As outlined in the earlier examples, using the transcriptional profile and hierarchical clustering of the Discovery cohort of prostate cancer samples, we have identified a distinct molecular subgroup of primary prostate cancers that clustered with metastatic disease and prostate cancers known to have concomitant metastases. This subgroup of primary tumour samples clustered with metastatic samples represented a poor prognostic population, whilst the benign like primary tumours defined a good prognostic subgroup. Functional analysis of the subgroup identified biological processes known to be involved in metastasis such as Epithelial Mesenchymal Transition (EMT) and cell migration. This cluster was hence defined as the 'Metastatic-Like' subgroup and for the purposes of this specification will be referred to throughout as 'Met-like'.

We developed a 70-gene signature to prospectively identify the 'Met-like' subgroup of patients. This 70-gene assay can be used to prospectively assess disease progression from a primary tumour, to determine the likelihood of disease recurrence and/or metastatic progression. We have also previously shown that the 70-gene signature also displays good performance in heterogeneity studies, maintaining subgroup detection and signature score stability.

We have also demonstrated the prognostic significance of this molecular subgroup using the 70-gene signature in three independent in silico datasets with different clinical endpoints. In the Glinksy dataset (79 prostate cancer cases), the signature showed a good discrimination of biochemical recurrence endpoint with a statistically significant AUC =0.69 [0.57-0.79], p = 0.0032 (Glinsky et al 2004). Also in the Erho dataset (545 prostate cancer cases), a statistically significant modest discrimination was observed with the signature for classifying patients metastatic recurrence endpoint (AUC 0.612 [0.569-0.653], p <0.0001) (Erho et al 2013). Finally, in the Taylor dataset, the signature had statistically significant association with patients time to metastatic recurrence (HR = 6.32 [1.98-20.20], p <0.0001) and time to biochemical recurrence with HR 3.76 [1.70-8.34], p < 0.0001 (Taylor et al 2010). Importantly, the metastatic biology subgroup has also been shown to predict poor outcome as identified by disease recurrence following surgical removal of the prostate independent of known prognostic factors such as Gleason score.

The identification of prostate cancer patients at high risk of recurrence following curative surgery or radiation is a key clinical requirement to identify those men that should receive adjuvant chemotherapy or radiation treatment whilst avoiding unnecessary interventions and side-effects in those who do not require further treatment. Based on this, the ability and performance of our 70-gene assay in identifying this high-risk population of patients required comprehensive clinical validation in independent cohorts of clinical prostate samples, either resections following curative surgery or biopsy specimens following curative radiotherapy.

**OBJECTIVES**

To further assess the performance of the prostate prognostic 70-gene assay in primary prostate resections.

To clinically validate the prostate prognostic 70-gene assay in an independent cohort of primary localised prostate cancer resections with the ability to identify a subgroup of prostate cancer patients at increased risk of developing biochemical recurrence and/or metastatic disease progression following surgery with curative intent.

To assess the performance of the prostate prognostic 70-gene assay in prostate biopsies in comparison to resection specimens.

To clinically validate the prostate prognostic 70-gene assay in an independent cohort of primary prostate biopsies with the ability to identify a subgroup of prostate cancer patients at increased risk of developing biochemical recurrence and/or metastatic disease progression following radiation treatment.

**MATERIALS & METHODS**

Processing and clinical validations of the 70 gene prognostic assay was performed in a blinded and randomised manner to avoid technical or biological confounding in the expression data which could have the potential to compromise data quality, integrity and validation objectives.

**Prostate Cancer Tumour Material**

This study performed gene expression analysis of two separate cohort of prostate cancer specimens. The first validation cohort was collected internally by Almac Diagnostics and included 349 prostate resection FFPE tissue samples obtained from four clinical sites; University College Dublin (62 samples), Wales Cancer Bank (100 samples), University of Surrey (41 samples) and University Hospital of Oslo (146 samples). This cohort consisted of samples across three key clinical groups, Non-recurrence patients (189 samples), Biochemical recurrence (also referred to as PSA recurrence) patients (112 samples) and Metastatic progression patients (48 samples). The resection dataset incorporated samples were collected based on the following inclusion criteria:

Clinical T-stage T1a-T3c (NXM0 at diagnosis)

Received radical prostatectomy surgery with curative intent

Not received neo-adjuvant hormone or therapy treatments

Patients within the non-recurrence group must not have received adjuvant treatment

3-5 years clinical follow up data available

Demographic, clinical and pathological variables utilised for the data analysis of the prostate resection cohort is summarised in Table 27.

The second validation cohort was collected in collaboration with the QUB as part of the FASTMAN Research Group and included 312 prostate biopsy FFPE tissue samples. This cohort consisted of 60 patient failures which incorporated 58 Biochemical recurrence, 24 Metastatic progression and 18 Castrate Resistant Prostate Cancer (CRPC). The biopsy dataset incorporated samples were collected based on the following inclusion criteria:

Clinical T-stage T1a-T3c (NXM0 at diagnosis)

Received radiotherapy with curative intent

3-5 years clinical follow up data available

Demographic, clinical and pathological variables utilised for the data analysis of the prostate biopsy cohort is summarised in Table 28.

Ethical approval for the sample acquisition and dataset analysis as validation of the prostate prognostic assay was obtained from the East of England Research Ethics Committee (Ref: 14/EE/1066).

**Gene Expression Profiling of Prostate Cancer samples**

Prior to sample profiling, clinical samples were randomized into RNA extraction batches and re-randomised into cDNA amplification processing batches using a list of pre-defined factors i.e. Clinical T-stage, PSA, Gleason, Age and Response. Clinical site factor was also included for validation 1. A further randomization of reagents, equipment and operators was performed prior to sample processing.

All samples were centrally pathology reviewed (Prof E. Kay RCSI) and marked-up for macrodissection based on the tumour area with the most dominant Gleason grade. For resection samples 2 x 10 $\mu$m sections were processed whereas for biopsy samples 4 x 5 $\mu$m sections were used for profiling. Total RNA was extracted from macrodissected FFPE tissue using the Roche High Pure RNA Paraffin Kit (Roche Diagnostics GmbH, Mannheim, Germany). RNA was converted into complementary deoxyribonucleic acid (cDNA), which was subsequently amplified and converted into single-stranded form using the SPIA® technology of the WT-Ovation™ FFPE RNA Amplification System V3 (NuGEN Technologies Inc., San Carlos, CA, USA). The amplified single-stranded cDNA was then fragmented and biotin labelled using the FL-Ovation™ cDNA Biotin Module V3 (NuGEN Technologies Inc.). The fragmented and labelled cDNA was then hybridised to the Almac Prostate Cancer DSATM. Almac's Prostate Cancer DSA™ research tool has been optimised for analysis of FFPE tissue samples, enabling the use of valuable archived tissue banks. The Almac Prostate Cancer DSA™ research tool is an innovative microarray platform that represents the transcriptome in both normal and cancerous prostate tissues. Consequently, the Prostate Cancer DSA™ provides a comprehensive representation of the transcriptome within prostate disease and tissue setting, not available using generic microarray platforms. Arrays were scanned using the Affymetrix Genechip® Scanner 7G (Affymetrix Inc., Santa Clara, CA).

**Process Controls**

Stratagene Universal Human Reference (UHR) samples and ES-2 cell line material were used as process controls within each processing batch as a standard measure during profiling of clinical cohorts. The UHR control is designed to be used as a universal reference RNA for microarray profiling experiments. These controls have been generated from pooling equal quantities of DNase treated cell line RNA to make a control RNA pool. The ES-2 cell line is a human clear cell carcinoma cell line representing ovarian cancer, established from an ovarian surgical tumour. The ES-2 cell line is characterised by a fibroblast morphology and cultures as an adherent cell line. Cells are maintained in McCoy's 5a Medium Modified with 10% Foetal Calf Serum (FCS), with a doubling time of approximately 24 hours. Due to their adherent properties and their fast doubling time these cells are ideal for bulking up as standard cell line controls. Approximately $1 \times 10^6$ ES-2 cells were pelleted and fixed overnight prior to processing as a Formalin Fixed Paraffin Embedded (FFPE) tissue block. One 10 μm section of the prepared ES-2 cell line FFPE block was utilised for RNA extraction prior to downstream profiling as a Prostate Metastatic assay specific processing control.

**Data Preparation and QC**

A continual QC assessment of samples during sample processing was performed. Samples with RNA and cDNA concentrations were taken forward for microarray profiling i.e. minimum of 12.5 ng/ul for RNA concentration and minimum of 140 ng/ul for cDNA concentration.

Microarray data quality was assessed continuously throughout the profiling of these cohorts on a batch by batch basis, and also cumulatively after the completion of profiling to exclude poor quality samples prior to analysis. Samples were pre-processed using the Robust Multi-Array (RMA) average methodology (Irizarry et al. 2003). The QC assessment comprised a combination of the following quality metrics:

Array Image Analysis: Array data was examined to identify any image artefacts

GeneChip QC: Percent present (%P), average signal absent, scale factor, average background and raw Q. Samples with a %P<15% were deemed QC fail

Principal Component Analysis: Hotelling T2 and residual residual Q method was used to identify sample outliers at the expression level

Intensity Distribution Analysis: Kolmogorov-Smirnov statistic (Massey. 1951) used to examine the intensity distribution of the samples and identify outliers Pre-defined limits of acceptance for Prostate assay specific cell line ES-2 were monitored using statistical process control (SPC) charts.

**Generation of Signature Scores**

Samples were pre-processed on a per sample basis using the refRMA (Irizarry et al. 2003) pre-processing model generated during the development of the 70 gene assay. Ensemble version 75 was used to annotate the probe sets to the corresponding Entrez Gene ID. Probe set expression was summarised to an Entrez Gene ID level using the median value (and excluding anti-sense probe sets). Assay scores were calculated using the following formula from the partial least squares model:

$$\text{Signature score} = \sum_i w_i \times \left(x_i - b_i\right) + k$$

Where $w_i$ is the weight of each entrez gene, $x_i$ is the gene expression, $b_i$ is the entrez gene specific bias and k=0.4365 (Table 29). Assay calls were assigned based upon predefined cut-off for all samples Samples with a continuous signature result > cut-off were labelled 'assay positive' otherwise 'assay negative'.

**Univariate and Multivariate Analysis**

Time to event (survival) analysis using time to biochemical recurrence (BCR) and time to metastatic disease was performed to evaluate the prognostic effects of the 70 gene prognostic assay. The survival distributions of patient groups defined by assay status (positive or negative) are visualized using Kaplan-Meier (KM) survival curves.

The Cox proportional hazards regression model was used to assess 70 gene assay status and survival (BCR and Metastatic disease). The hazard ratio (HR) was used to quantify the effect (association) of assay status with survival endpoints. In addition to the univariate (unadjusted) analysis, the multivariable (adjusted) Cox model was used to assess the effect of the assay status (positive or negative) on BCR and Metastatic disease, adjusting for PSA at diagnosis, patient age and Gleason score on survival outcome. All estimated effects are reported with 95% confidence intervals from an analysis in which the assay and these standard prognostic factors were included, regardless of their significance. Interpretation of estimated parameters from Cox proportional hazards test and the level of significance, the goodness of fit of the fitted model was investigated including checking the fulfilment of the proportional hazards assumption (Gramsbsch & Therneau, 1994).

Multivariable (adjusted) Cox model was also used to assess the effect of the assay status (positive or negative) on BCR and Metastatic disease, adjusting for CAPRA score (Cooperberg et al. 2006). CAPRA scores for each sample were determined using PSA, Biopsy Gleason score, clinical T-stage, percentage of positive biopsy cores and age.

All tests of statistical significance were 2-sided at 5% level of significance. Statistical analysis was performed using MedCalc version 13.

## RESULTS

### The 70-gene signature predicts time to biochemical recurrence of the 'Met-like' subgroup in the resection validation cohort

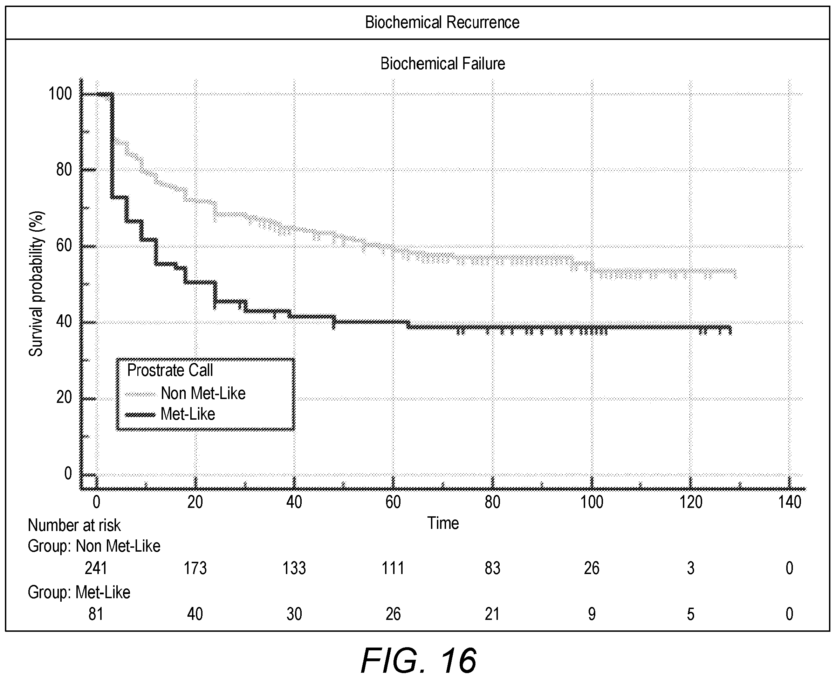

Utilising 5-10 year clinical follow up data, univariate survival analysis was performed on the 322 samples which passed microarray data QC to assess the performance of the 70-gene signature at predicting time to biochemical recurrence in the resection dataset following surgery. The Kaplan-Meier survival curve shows a significant association of the 70-gene signature at predicting earlier time to recurrence (months) of the 'Met-like' subgroup (blue) in comparison to the Non Met-like samples (green). This suggests that the samples within the 'Met-like' subgroup have an increased risk of developing biochemical disease recurrence following radical prostatectomy surgery with curative intent (HR = 1.74 [1.18 - 2.56]; p=0.0009) (Figure 16). Multivariate analysis of the dataset was performed to assess the performance of the 70-gene signature at predicting biochemical recurrence, independent of known clinical prognostic factors including age at surgery, PSA levels at diagnosis and combined Gleason score. Considering these prognostic factors, the prostate prognostic 70-gene signature was significantly associated with predicting biochemical recurrence independent of age, PSA and Gleason grade (both <7 and >7) (HR 1.65 [1.16 - 2.34]; p =0.0055) (Table 30a).

### The 70-gene signature predicts time to metastatic disease progression of the 'Met-like' subgroup in the resection validation cohort

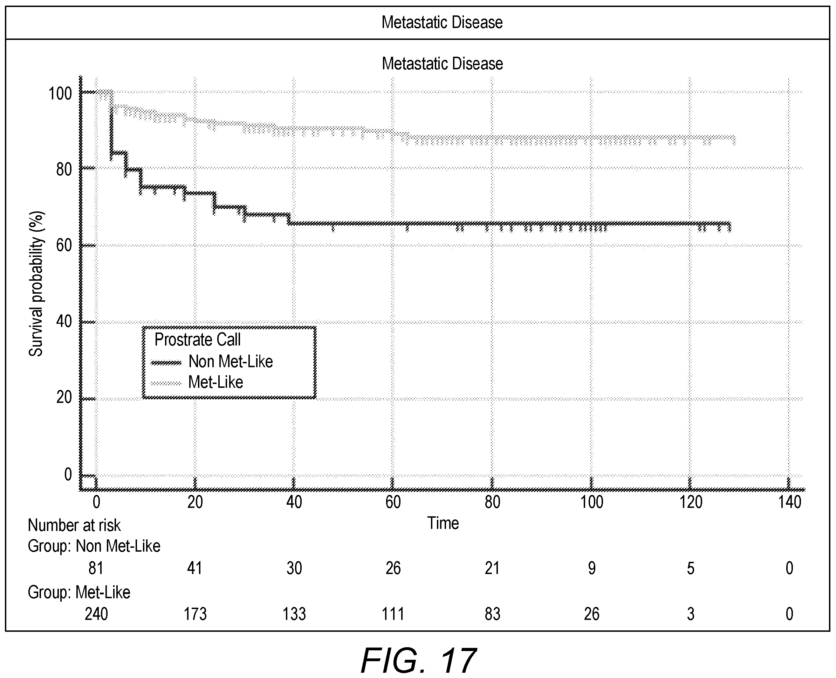

Next using the 5-10 year clinical follow up data, univariate survival analysis was also performed on the 322 samples which passed microarray data QC to assess the performance of the 70-gene signature at predicting time to metastatic progression either local or distant sites, in the resection dataset following surgery. Similarly to biochemical recurrence, the Kaplan-Meier survival curve shows a significant association of the 70-gene signature at predicting metastatic progression of the 'Met-like' subgroup (blue) in comparison to the Non Met-like samples (green). This suggests that the patients within the 'Met-like' subgroup have an increased risk of developing metastatic disease progression following radical prostatectomy surgery with curative intent (HR = 3.60 [1.81 - 7.13]; p <0.0001) (Figure17). Multivariate analysis of the resection dataset was investigated to assess the performance of the 70-gene signature at predicting metastatic progression, independent of known clinical prognostic factors including age at surgery, PSA levels at diagnosis and combined Gleason score. The prostate prognostic 70-gene signature scores of the 'Met-like' subgroup were shown to be significantly associated with predicting metastatic disease progression independent of age, PSA and Gleason grade (both <7 and >7) (HR 3.50 [1.95-6.27]; p <0.0001), hence supporting that patients within this group are 'high-risk' for progression (Table 30b). Interestingly, the 70-gene signature appears to show better performance as a prognostic factor as opposed to age, PSA and Gleason <7 for predicting metastatic disease (Table 30b).

### The 70-gene signature predicts time to biochemical recurrence of the 'Met-like' subgroup in the biopsy validation cohort

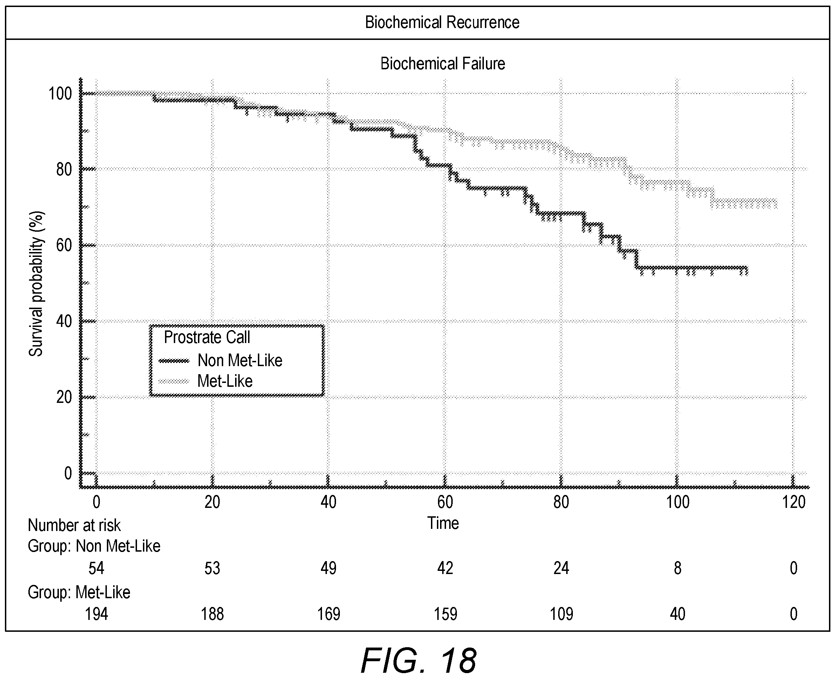

Univariate survival analysis was performed using the collated 5-10 year follow up clinical data on the 322 samples to assess the performance of the 70-gene signature at predicting time to biochemical recurrence in the biopsy dataset following radiotherapy with curative intent. The Kaplan-Meier survival curve shows a significant association of the 70-gene signature at predicting earlier time to recurrence (months) of the 'Met-like' subgroup (blue) in comparison to the Non Met-like samples (green). As with the resection dataset, this suggests that the patients within the 'Met-like' subgroup have an increased risk of developing biochemical disease recurrence following radical radiotherapy with curative intent (HR = 2.18 [1.14 - 4.17]; p=0.0042) (Figure 18). Multivariate analysis of the dataset was then performed to assess the performance of the 70-gene signature at predicting biochemical recurrence, independent of other commonly used prognostic factors including age at diagnosis, PSA levels at diagnosis and combined Gleason score. The prostate prognostic 70-gene signature of the 'Met-like' group was significantly associated with predicting biochemical recurrence independent of age, PSA and Gleason grade (both <7 and >7) (HR 1.96 [1.11 - 3.48]; p =0.0220), indicating that the patients within this subgroup are at increasing risk of developing biochemical recurrence (Table 31a). Of note, this data suggests that no other variable within the covariate analysis is significantly associated with identifying the increased risk of disease recurrence in the 'Met-like' subgroup (Table 31a).

**The 70-gene signature predicts time to metastatic disease progression of the 'Met-like' subgroup in the biopsy validation cohort**

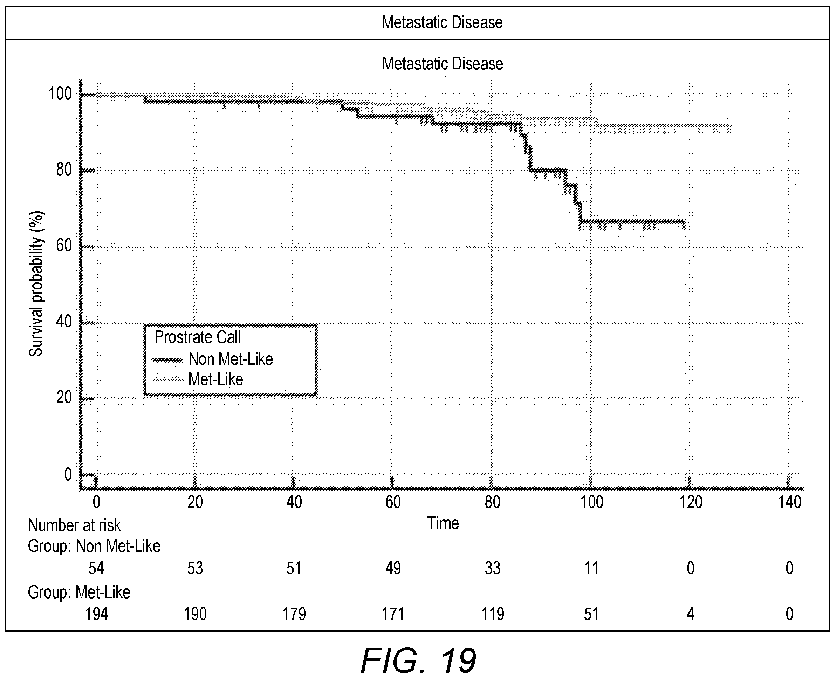

Following this, univariate survival analysis was also performed on the 248 QC pass samples to determine the performance of the 70-gene signature at predicting time to metastatic progression either local or distant sites, in the biopsy dataset following surgery. As with biochemical recurrence, the Kaplan-Meier survival curve shows a significance of the 70-gene signature at predicting metastatic progression of the 'Met-like' subgroup (blue) in comparison to the Non Met-like samples (green). This suggests that the patients within the 'Met-like' subgroup have an increased risk of developing metastatic disease progression following radical radiotherapy treatment with curative intent (HR = 3.50 [1.28 - 9.56]; p =0.0017) (Figure 19). Multivariate analysis of the biopsy dataset was performed to further assess the performance of the 70-gene signature at predicting metastatic progression, independent of other known clinical prognostic factors including age at diagnosis, PSA levels at diagnosis and combined Gleason score. The prostate prognostic 70-gene signature was shown to be significantly associated with predicting metastatic disease progression independent of age, PSA and Gleason grade (both <7 and >7) (HR 2.66 [1.10 - 6.40]; p <0.0304) (Table 31b). Similarly to the assessment of biochemical recurrence in the biopsy cohort, this data suggests that no other variable within the covariate analysis is significantly associated with identifying the increased risk of disease recurrence in the 'Met-like' subgroup (Table 31b).

Collectively, the data for both the resection and biopsy cohorts support the 70-gene signature as a prognostic assay in the field of prostate cancer which could be implemented as a patient stratifier to identify prostate cancer patients from early detection that may be at increased risk of developing more aggressive high-risk disease within 3-5 years of initial treatment.

**Performance of the 70-gene signature as a prognostic tool for biochemical and metastatic recurrence in comparison to the CAPRA scoring system**

The CAPRA and CAPRA-S scoring system for prostate cancer is a multivariate prognostic tool which has been developed to predict risk of disease recurrence using pre-operative biopsy material (CAPRA) and post-operative resected material (CAPRA-S). The scoring system can provide outcome based on a range of risk levels and is calculated on a points system taking into account PSA levels, patient age, Gleason grade and clinical T-stage whereby the higher the cumulative points the greater the risk of disease recurrence (Cooperberg et al 2005). CAPRA-S used to assess risk and prediction post-surgery also includes scoring for additional clinical factors including seminal vesicle invasion (SVI), extracapsular extension (ECE), lymph node invasion (LNI) and surgical margins. The only additional factor utilised in the CAPRA scoring system for biopsy material is the % of positive cores > or < 34%. Firstly, we investigated the prognostic performance of the novel 70-gene signature in comparison to the CAPRA-S scoring system. In multivariate analysis only the CAPRA-S scoring was significantly associated with biochemical recurrence, (HR = 1.36 [1.28-1.45], p<0.0001) however both the metastatic assay and CAPRA-S scoring were significantly associated with the development of metastatic disease (HR 2.53 [1.40 - 4.60]; p =0.0024 and HR = 1.43 [1.28-1.61], p<0.0001 (Table 32a and 32b). These data indicate that the metastatic signature provided additional information to the CAPRA-S scoring system.

Finally we also interrogated the prognostic performance of our 70-gene signature in comparison to the CAPRA scoring system. Only the 70-gene signature was significantly associated with prognostic outcome and identifying the high-risk 'Met-like' subgroup at increased chance of developing biochemical recurrence in the biopsy dataset (HR 2.05 [1.18 - 3.59]; p =0.0119) whilst the CAPRA score showing no significance independent of the prognostic assay (Table 33a). Similarly, in the biopsy validation cohort, only the 70-gene signature was significantly associated with prognostic outcome and identifying the high-risk 'Met-like' subgroup at increased chance of developing metastatic disease progression (HR 3.39 [1.44 - 7.97]; p =0.0054) (Table 33b). In sum, the comparison of the 70-gene signature to the CAPRA scoring system shows better performance in biopsy material and provides further evidence for the use of the 70-gene signature as a prognostic assay within the field of prostate cancer.

**DISCUSSION**

Approximately 35% of primary localised prostate cancer progress to a more aggressive and recurrent disease state despite radical treatment such as surgery or external beam radiotherapy, whilst a large number of primary cancers will not progress to clinically significant disease. With this in mind, a great clinical question within the field is how to easily distinguish these subgroups of patients to allow patient stratification which could ultimately determine which patients may require further and more intense treatment regimens and which patients could avoid the toxic less tolerated therapies if unnecessary. It is thought that a potential approach to stratification is the development of compound prognostics factors which is based on both a combination of single prognosticators and their associations or alternatively gene expression profiles from DNA-microarray profiling (Buhmeida et al 2006).

Utilising this approach, Almac Diagnostics have developed and validated a 70-gene signature as a potential prognostic assay which could promote the identification of a high-risk prostate cancer population at increased risk of developing more aggressive disease, either biochemical or metastatic recurrence. The data within this specification strongly supports the performance of the prostate prognostic assay in both resection and biopsy material. In two independent clinical validation cohorts of primary prostate resections and biopsies, the 70-gene signature can accurately identify a subgroup of patients with a 'Met-like' biology and a greater risk of biochemical disease relapse or metastatic disease within 3-5 years of follow up. The subgroup of patients with a 'Met-like' biology are considered the population who should receive additional treatment post-surgery, such as adjuvant hormone therapy, radiotherapy or treatment with taxanes. Conversely to this, the patients identified within the Non Met-like subgroup should be spared from further treatment and monitored throughout standard clinical follow-up. It is evident this prognostic assay has two clear clinical utilities:

Predicting a subset of a defined prostate cancer cohort from resection material who may progress with high-risk disease (either biochemical recurrence or metastatic progression) following radical prostatectomy surgery with curative intent.

Predicting a subset of a defined prostate cancer cohort from biopsy material who may progress with high-risk disease (wither biochemical or metastatic progression) following radical radiotherapy with curative intent.

**TABLE LEGENDS**

**Table 28** - Summary of demographic, clinical and pathological variables considered for analysis of the internal resection cohort. Table outlines total number of patients, the median and range of age at surgery (years), time to recurrence (months), pre-operative PSA levels (ng/ml) and the number (%) of patients from each of the four clinical sites, within each recurrence subgroup, associated with each of the representative Gleason grades, within each pathological T-stage subgroup, with lymph node invasion (LNI), seminal vesicle invasion (SVI), extracapsular extension (ECE) and patients with negative, diffuse or focal surgical margins.

**Table 29** - Summary of demographic, clinical and pathological variables considered for analysis of the FASTMAN biopsy cohort. Table outlines total number of patients, the median and range of age at diagnosis (years), time to recurrence (months), PSA levels at diagnosis (ng/ml) and the number (%) of patients, within each recurrence subgroup, associated with each of the representative Gleason grades and within each pathological T-stage subgroup.

**Table 30** - Genes, weightings and bias of the 70-gene signature.

**Table 31** - A) Multivariate analysis of the 70-gene signature in the internal resection cohort for biochemical recurrence, demonstrating assay performance independent of other prognostic clinical factors including age at surgery, PSA levels and combined Gleason score. P-values, hazard ratios (HR) and 95% confidence intervals (CI) of the HR are outlined within the table. P-values highlighted in red indicate statistical significance. B) Multivariate analysis of the 70-gene signature in the internal resection cohort for metastatic disease progression, demonstrating assay performance independent of other prognostic clinical factors including age at surgery, PSA levels and combined Gleason score. P-values, hazard ratios (HR) and 95% confidence intervals (CI) of the HR are outlined within the table. P-values highlighted in red indicate statistical significance.

**Table 32** - A) Multivariate analysis of the 70-gene signature in the FASTMAN biopsy cohort for biochemical recurrence, demonstrating assay performance independent of other prognostic clinical factors including age at diagnosis, PSA levels and combined Gleason score. P-values, hazard ratios (HR) and 95% confidence intervals (CI) of the HR are outlined within the table. P-values highlighted in red indicate statistical significance. B) Multivariate analysis of the 70-gene signature in the FASTMAN biopsy cohort for metastatic disease progression, demonstrating assay performance independent of other prognostic clinical factors including age at diagnosis, PSA levels and combined Gleason score. P-values, hazard ratios (HR) and 95% confidence intervals (CI) of the HR are outlined within the table. P-values highlighted in red indicate statistical significance.

**Table 33** - A) Covariate analysis of the 70-gene signature in comparison to the CAPRA-S scoring system within the internal resection cohort for biochemical recurrence, demonstrating assay performance against alternative prognostic scoring assays. P-values, hazard ratios (HR) and 95% confidence internals (CI) of the HR are outlined for each comparison within the table. P-values highlighted in red indicate statistical significance. B) Covariate analysis of the 70-gene signature in comparison to the CAPRA-S scoring system within the internal resection cohort for metastatic disease progression, demonstrating assay performance against alternative prognostic scoring assays. P-values, hazard ratios (HR) and 95% confidence internals (CI) of the HR are outlined for each comparison within the table. P-values highlighted in red indicate statistical significance.

**Table 34** - A) Covariate analysis of the 70-gene signature in comparison to the CAPRA scoring system within the FASTMAN biopsy cohort for biochemical recurrence, demonstrating assay performance against alternative prognostic scoring assays. P-values, hazard ratios (HR) and 95% confidence internals (CI) of the HR are outlined for each comparison within the table. P-values highlighted in red indicate statistical significance. B) Covariate analysis of the 70-gene signature in comparison to the CAPRA scoring system within the FASTMAN biopsy cohort for metastatic disease progression, demonstrating assay performance against alternative prognostic scoring assays. P-values, hazard ratios (HR) and 95% confidence internals (CI) of the HR are outlined for each comparison within the table. P-values highlighted in red indicate statistical significance.

**Table 28:** Demographic and Clinical variable summary of Resection validation cohort

| Variable | | Validation Cohort |
|---|---|---|
| **Patient Number** | *No.of Patients* | 322 |
| | | |
| **Clinical Site - n (%)** | *UCD* | 61 (19) |
| | *Oslo* | 142 (44) |
| | *Surrey* | 34 (11) |
| | *WCB* | 85 (26) |
| | | |
| **Age at Surgery** | *Median (range), Years* | 62 (41 - 75) |
| | | |
| **Recurrence Event - n (%)** | *Non-recurrence* | 172 (53) |
| | *Biochemical recurrence* | 103 (32) |
| | *Metastatic recurrence* | 47 (15) |
| | | |
| **Time to Recurrence-Median (range)** | *Biochemical recurrence* | 12 (1- 100) |
| | *Metastatic recurrence* | 6 (3 - 63) |
| | | |
| **Pre-operative PSA** | *Median (range), ng/ml* | 8.4 (2 - 253) |
| | | |
| **Gleason score - n (%)** | *<6* | 2 (1) |
| | *6* | 67 (21) |
| | *7* | 197 (61) |
| | *8-10* | 55 (17) |
| | | |

(continued)

| Variable | | Validation Cohort |
|---|---|---|
| **Pathological T-stage-n (%)** | *T1* | 1 (0.5) |
| | *T2* | 174 (54) |
| | *T3* | 146 (45) |
| | *T4* | 1 (0.5) |
| | | |
| **Lymph Node Invasion - n (%)** | *Yes* | 16 (5) |
| | *No* | 105 (33) |
| | *Unknown* | 201 (62) |
| | | |
| **Seminal Vesicle Invasion - n (%)** | *Yes* | 62 (19) |
| | *No* | 260 (81) |
| | | |
| **Extracapsular Extension - n (%)** | *Yes* | 97 (30) |
| | *No* | 190 (59) |
| | *Unknown* | 35 (11) |
| | | |
| **Surigcal Margins - n (%)** | *Negative* | 132 (41) |
| | *Focal* | 40 (12) |
| | *Diffuse* | 65 (20) |
| | *Unknown* | 85 (27) |

**Table 29:** Demographic and Clinical variable summary of Biopsy validation cohort

| Variable | | Validation Cohort |
|---|---|---|
| **Patient Number** | *No of Patients* | 248 |
| | | |
| **Clinical Site - n (%)** | *Belfast* | 248 (100) |
| | | |
| **Age at Diagnosis** | *Median (range), Years* | 68 (48-79) |
| | | |
| **Recurrence Event - n (%)** | *Non-recurrence* | 170 (68) |
| | *Biochemical recurrence* | 56 (23) |
| | *Metastatic recurrence* | 22 (9) |
| | | |
| **Time to Recurrence - Median (range)** | *Biochemical recurrence* | 82 (10 - 117) |
| | *Metastatic recurrence* | 86.5 (10 - 128) |
| | | |
| **PSA at Diagnosis** | *Median (range), ng/ml* | 17.95 (3.2- 222.3) |

(continued)

| Variable | | Validation Cohort |
|---|---|---|
| | | |
| **Gleason Grade -n (%)** | *6* | 41 (17) |
| | *7* | 100 (40) |
| | *8 - 10* | 107 (43) |
| | | |
| **Pathological T-stage - n (%)** | *T1* | 51 (21) |
| | *T2* | 76 (31) |
| | *T3* | 92 (36) |
| | *T4* | 4 (2) |
| | *Unknown* | 25 (10) |
| | | |

**Table 30:** Genes, weightings and bias of the 70-gene signature

| Gene Name | Entrez Gene ID | Weight | Bias |
|---|---|---|---|
| CAPN6 | 827 | -0.010898880 | 4.440873234 |
| THBS4 | 7060 | -0.009631509 | 6.912586369 |
| PLP1 | 5354 | -0.008885735 | 4.383572327 |
| MT1A | 4489 | -0.008680747 | 6.747956978 |
| MIR205HG | 406988 | -0.008278545 | 7.215245389 |
| SEMG1 | 6406 | -0.007934619 | 4.230422622 |
| RSPO3 | 84870 | -0.007295796 | 4.293172794 |
| ANO7 | 50636 | -0.007164357 | 6.522547774 |
| PCP4 | 5121 | -0.007138975 | 7.621758138 |
| ANKRD1 | 27063 | -0.006922498 | 5.92831485 |
| MYBPC1 | 4604 | -0.006844539 | 4.574318807 |
| MMP7 | 4316 | -0.006835450 | 6.756722063 |
| SERPINA3 | 12 | -0.006830879 | 5.745461752 |
| SELE | 6401 | -0.006809804 | 5.977682143 |
| KRT5 | 3852 | -0.006402712 | 6.080493983 |
| LTF | 4057 | -0.006400452 | 6.497259991 |
| KIAA1210 | 57481 | -0.006380629 | 3.559966010 |
| TMEM158 | 25907 | -0.006312212 | 8.063421249 |
| ZFP36 | 7538 | -0.006271047 | 9.960826690 |
| FOS8 | 2354 | -0.006108115 | 6.954936015 |
| PCA3 | 50652 | -0.006101922 | 5.262341585 |
| TRPM8 | 79054 | -0.006059944 | 4.865791397 |
| PTTG1 | 9232 | 0.006017344 | 4.712692803 |

(continued)

| Gene Name | Entrez Gene ID | Weight | Bias |
|---|---|---|---|
| #N/A | 283194 | -0.005950381 | 4.980380941 |
| PAGE4 | 9506 | -0.005837135 | 7.073906580 |
| STEAP4 | 79689 | -0.005684812 | 8.105295362 |
| TMEM178A | 130733 | -0.00564663 | 7.59452596 |
| CXCL2 | 2920 | -0.005597719 | 8.928977514 |
| HS3ST3A1 | 9955 | -0.005593197 | 4.232781732 |
| EYA1 | 2138 | -0.005581031 | 5.504276204 |
| RSPO2 | 340419 | -0.005562783 | 3.922420794 |
| PKP1 | 5317 | -0.005553136 | 5.912186171 |
| MUC6 | 4588 | -0.005522157 | 6.640037274 |
| PENK | 5179 | -0.005505761 | 4.514855049 |
| DEFB1 | 1672 | -0.005399899 | 6.825490924 |
| SLC7A3 | 84889 | -0.005389518 | 4.649003630 |
| MIR578 | 693163 | -0.005355230 | 5.087389320 |
| PI15 | 51050 | -0.005263663 | 4.858716243 |
| UBXN10-AS1 | 101928017 | -0.005259309 | 6.065877615 |
| PDK4 | 5166 | -0.005248750 | 4.174094312 |
| PHGR1 | 644844 | -0.005207500 | 5.183571143 |
| SERPINE1 | 5054 | -0.005194886 | 6.691866284 |
| PDZRN4 | 29951 | -0.005146623 | 4.752327652 |
| ZNF185 | 7739 | -0.005105327 | 6.900544220 |
| ADRA2C | 152 | -0.005054713 | 7.078376864 |
| AZGP1 | 563 | -0.005018400 | 8.191177501 |
| TK1 | 7083 | 0.004965887 | 5.581334570 |
| POTEH | 23784 | -0.004961473 | 4.824976325 |
| KIF11 | 3832 | 0.004928774 | 3.917668501 |
| CLDN1 | 9076 | -0.004924383 | 4.960282713 |
| MIR4530 | 100616163 | -0.004907676 | 10.53645223 |
| MAFF | 23764 | -0.004901224 | 8.497945251 |
| ZNF765 | 91661 | -0.004861949 | 3.976333034 |
| CKS2 | 1164 | 0.004855890 | 6.503980715 |
| TCEAL7 | 56849 | -0.004855875 | 4.819327983 |
| PLIN1 | 5346 | 0.004830634 | 4.629391793 |
| SIGLEC1 | 6614 | 0.004772601 | 5.503752383 |
| FAM1508 | 285016 | -0.004772585 | 6.664595224 |
| MFAP5 | 8076 | -0.004771653 | 4.129176546 |
| SFRP1 | 6422 | -0.004761531 | 7.901261944 |
| DUSPS | 1847 | -0.004718060 | 5.762677834 |

(continued)

| Gene Name | Entrez Gene ID | Weight | Bias |
|---|---|---|---|
| VARS2 | 57176 | 0.004675188 | 5.223455192 |
| ABCC4 | 10257 | -0.004664227 | 5.230376747 |
| SH3BP4 | 23677 | -0.004622969 | 4.882708067 |
| SORD | 6652 | -0.004573155 | 8.958411069 |
| MTERFD1 | 51001 | 0.004522466 | 5.334198783 |
| DPP4 | 1803 | -0.004505906 | 4.65974831 |
| #N/A | 284837 | 0.004502134 | 4.905312692 |
| FAM3B | 54097 | -0.004443400 | 7.388071281 |
| KLK3 | 354 | -0.004424720 | 10.226441291 |

**Table 31**: Multivariate analysis of the 70-gene signature in the internal resection cohort for a) biochemical recurrence and b) metastatic progression.

### a) Biochemical Recurrence

| Covariate | HR | 95% CI | p |
|---|---|---|---|
| Prostate Metastatic Assay : Negative | 1.65 | 1.16 to 2.34 | 0.0055 |
| Gleason="<7" | 0.59 | 0.36 to 0.97 | 0.0388 |
| Gleason=">7" | 2.10 | 1.44 to 3.07 | 0.0001 |
| Age | 1.00 | 0.97 to 1.03 | 0.9088 |
| PSA | 1.00 | 1.00 to 1.01 | 0.0089 |

Abbreviations: HR, hazard ratio
Assessment post-surgical.

### b) Metastatic Disease

| Covariate | HR | 95% CI | p |
|---|---|---|---|
| Prostate Metastatic Assay : Negative | 3.50 | 1.95 to 6.27 | <0.0001 |
| Gleason="<7" | 0.35 | 0.11 to 1.17 | 0.0906 |
| Gleason=">7" | 3.11 | 1.67 to 5.77 | 0.0004 |
| Age | 0.98 | 0.93 to 1.03 | 0.4039 |
| PSA | 1.01 | 0.99 to 1.02 | 0.3634 |

**Table 32:** Multivariate analysis of the 70-gene signature in FASTMAN biopsy cohort for a) biochemical recurrence and b) metastatic progression

| a) Biochemical Recurrence | | | |
|---|---|---|---|
| Covariate | P-value | HR | 95% CI of HR |
| Prostate 70 Gene Call: Met-Like | 0.0220 | 1.96 | 1.11 to 3.48 |
| Age at Diagnosis | 0.1375 | 0.97 | 0.93 to 1.01 |

(continued)

| a) Biochemical Recurrence | | | |
|---|---|---|---|
| Covariate | P-value | HR | 95% CI of HR |
| Prostate 70 Gene Call: Met-Like | 0.0220 | 1.96 | 1.11 to 3.48 |
| PSA at Diagnosis | 0.1308 | 1.01 | 1.00 to 1.01 |
| Combined Gleason Score ="<7" | 0.1510 | 0.49 | 0.19 to 1.29 |
| Combined Gleason Score =">7" | 0.9409 | 0.98 | 0.55 to 1.73 |
| b) Metastatic Disease | | | |
| Covariate | P-value | HR | 95% CI of HR |
| Prostate 70 Gene Call: Met-Like | 0.0304 | 2.66 | 1.10 to 6.40 |
| Age at Diagnosis | 0.7628 | 0.99 | 0.93 to 1.06 |
| PSA at Diagnosis | 0.2517 | 1.01 | 1.00 to 1.02 |
| Combined Gleason Score ="<7" | 0.3573 | 0.37 | 0.05 to 3.03 |
| Combined Gleason Score =">7" | 0.5389 | 1.35 | 0.52 to 3.46 |

Table 33: Analysis and comparison of the 70-gene signature to CAPRA scoring system in the internal resection cohort for a) biochemical recurrence and b) metastatic progression.

a) Biochemical Recurrence

| Covariate | HR | 95% CI | p |
|---|---|---|---|
| Prostate Metastatic Assay : Negative | 1.34 | 0.94 to 1.90 | 0.1079 |
| CARPA-S | 1.36 | 1.28 to 1.45 | <0.0001 |

Abbreviations: HR, hazard ratio; CAPRA-s, Cancer of the Prostate Risk Assessment post-surgical.

b) Metastatic Disease

| Covariate | HR | 95% CI | p |
|---|---|---|---|
| Prostate Metastatic Assay : Negative | 2.53 | 1.40 to 4.80 | 0.0024 |
| CARPA-S | 1.43 | 1.28 to 1.61 | <0.0001 |

Table 34: Analysis and comparison of the 70-gene signature to CAPRA scoring system in the FASTMAN biopsy cohort for a) biochemical recurrence and b) metastatic progression.

| a) Biochemical Recurrence | | | |
|---|---|---|---|
| Covariate | P-value | HR | 95% CI of HR |
| Prostate 70 Gene Call: Met-Like | 0.0119 | 2.05 | 1.18 to 3.59 |
| CAPRA Score | 0.3443 | 1.11 | 0.90 to 1.36 |
| b) Metastatic Disease | | | |
| Covariate | P-value | HR | 95% CI of HR |
| Prostate 70 Gene Call: Met-Like | 0.0054 | 3.39 | 1.44 to 7.97 |

(continued)

| b) Metastatic Disease | | | |
| --- | --- | --- | --- |
| Covariate | P-value | HR | 95% CI of HR |
| CAPRA Score | 0.7455 | 1.06 | 0.76 to 1.47 |

**Example 4 - Core and minimum gene analysis**

**Samples:**

- Internal training samples (Discovery cohort): This sample set comprised of 126 FFPE prostate resection FFPE tissue samples profiled on the Almac Prostate DSA™ microarray.

- FASTMAN Biopsy Validation Cohort: This sample set was comprised of 248 prostate biopsy FFPE tissue samples collected in collaboration with the FASTMAN Research Group under the Movember Programme.

- Internal Resection Validation Cohort: This sample set comprised of 322 prostate resection FFPE tissue samples collected internally by Almac Diagnostics. Samples were obtained from four clinical sites; University College Dublin (61 samples), Wales Cancer Bank (85 samples), University of Surrey (34 samples) and University Hospital of Oslo (142 samples).

**Methods:**

**Core gene analysis**

The purpose of evaluating the core gene set of the signature is to determine a ranking for the Entrez genes based upon their impact on performance when removed from the signature. This analysis involved 10,000 random samplings of 10 signature Entrez genes from the original 70 signature Entrez gene set. At each iteration, 10 randomly selected signature Entrez genes were removed and the performance of the remaining 65 genes was evaluated using the endpoint to determine the impact on HR (Hazard Ratio) performance when these 10 Entrez genes were removed in the following 2 datasets:

- FASTMAN Biopsy Validation Cohort - 248 samples

- Internal Resection Validation Cohort - 322 samples

FASTMAN Biopsy Validation was evaluated using the biochemical recurrence (BCR) endpoint and Internal Resection Validation was evaluated using the metastatic recurrence (MET) endpoint. Within each of the 2 datasets, the signature Entrez genes were weighted based upon the change in HR performance (Delta HR) based upon their inclusion or exclusion. Entrez genes ranked '1' have the most negative impact on performance when removed and those ranked '70' have the least impact on performance when removed.

**Minimum gene analysis**

The purpose of evaluating the minimum number of Entrez genes is to determine if significant performance can be achieved within smaller subsets of the original signature.

This analysis involved 10,000 random samplings of the 70 signature Entrez genes starting at 1 Entrez gene/feature, up to a maximum of 30 Entrez genes/features. For each randomly selected feature length, the signature was redeveloped using the PLS machine learning method under CV and model parameters derived. At each feature length, all randomly selected signatures were applied to calculate signature scores for the following 2 datasets:

- FASTMAN Biopsy Validation Cohort - 248 samples

- Internal Resection Validation Cohort - 322 samples

Continuous signature scores were evaluated with outcome to determine the HR effect; FASTMAN Biopsy Validation was evaluated with BCR and Internal Resection Validation was evaluated with MET. The HR for all random signatures at each feature length was summarized and figures generated to visualize the performance over CV.

## RESULTS

### Core gene analysis

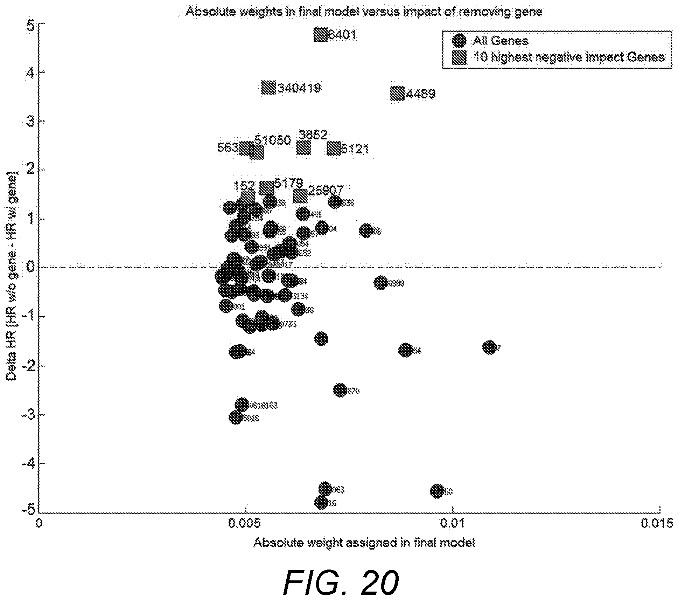

The results for the core gene analysis of the 70 gene signature in the 2 datasets is provided in this section.
• FASTMAN Biopsy Validation: Delta HR performance measured in this dataset for the 70 signature Entrez genes is shown in Figure 20. This figure highlights the top 10 ranked Entrez genes in the signature which are the most important in retaining a good HR performance within this dataset. This ranking can also been found in Table 35 below:

| Entrez Gene | Gene | Total Delta HR | Rank |
|---|---|---|---|
| 6401 | SELE | 4.761124889 | 1 |
| 340419 | RSPO2 | 3.687852175 | 2 |
| 4489 | MT1A | 3.565744532 | 3 |
| 3852 | KRT5 | 2.45747844 | 4 |
| 563 | AZGP1 | 2.446961746 | 5 |
| 5121 | PCP4 | 2.440528148 | 6 |
| 51050 | PI15 | 2.353758149 | 7 |
| 5179 | PENK | 1.642705501 | 8 |
| 25907 | TMEM158 | 1.476987515 | 9 |
| 152 | ADRA2C | 1.4186879 | 10 |
| 50636 | ANO7 | 1.34866117 | 11 |
| 2138 | EYA1 | 1.348354023 | 12 |
| 3832 | KIF11 | 1.291035934 | 13 |
| 23677 | SH3BP4 | 1.224986822 | 14 |
| 5166 | PDK4 | 1.188342205 | 15 |
| 57481 | KIAA1210 | 1.103651804 | 16 |
| 23784 | POTEH | 1.043547171 | 17 |
| 6614 | SIGLEC1 | 0.855535152 | 18 |
| 4604 | MYBPC1 | 0.819417585 | 19 |
| 2920 | CXCL2 | 0.813780936 | 20 |
| 6406 | SEMG1 | 0.768923782 | 21 |
| 9955 | HS3ST3A1 | 0.749239331 | 22 |
| 4057 | LTF | 0.71103352 | 23 |
| 7083 | TK1 | 0.677537934 | 24 |
| 57176 | VARS2 | 0.653632853 | 25 |
| 79054 | TRPM8 | 0.506824534 | 26 |
| 29951 | PDZRN4 | 0.420605146 | 27 |
| 9506 | PAGE4 | 0.340073483 | 28 |
| 50652 | PCA3 | 0.315775741 | 29 |
| 79689 | STEAP4 | 0.266189243 | 30 |

(continued)

| Entrez Gene | Gene | Total Delta HR | Rank |
|---|---|---|---|
| 1847 | DUSP5 | 0.178110535 | 31 |
| 50652 | SFRP1 | 0.138569985 | 32 |
| 79689 | MIR578 | 0.118486894 | 33 |
| 1847 | UBXN10-AS1 | 0.068688136 | 34 |
| 6422 | SORD | -0.004486521 | 35 |
| 5346 | PLIN1 | -0.086533897 | 36 |
| 56849 | TCEAL7 | -0.13067584 | 37 |
| 693163 | DPP4 | -0.144066233 | 38 |
| 5317 | PKP1 | -0.164994289 | 39 |
| 354 | KLK3 | -0.166136293 | 40 |
| 54097 | FAM3B | -0.209897076 | 41 |
| 6652 | MAFF | -0.214942264 | 42 |
| 9232 | PTTG1 | -0.256777275 | 43 |
| 5346 | FOSB | -0.264910805 | 44 |
| 406988 | MIR205HG | -0.303067689 | 45 |
| 56849 | ZNF765 | -0.423012094 | 46 |
| 284837 | #N/A | -0.449656588 | 47 |
| 1803 | SERPINE1 | -0.476929578 | 48 |
| 10257 | ABCC4 | -0.490520163 | 49 |
| 644844 | PHGR1 | -0.539343141 | 50 |
| 5317 | #N/A | -0.555242337 | 51 |
| 4588 | MUC6 | -0.574748909 | 52 |
| 51001 | MTERFD1 | -0.770988555 | 53 |
| 7538 | ZFP36 | -0.842688769 | 54 |
| 1672 | DEFB1 | -1.003111116 | 55 |
| 9076 | CLDN1 | -1.074445919 | 56 |
| 130733 | TMEM178A | -1.134351 | 57 |
| 84889 | SLC7A3 | -1.153855918 | 58 |
| 7739 | ZNF185 | -1.20365806 | 59 |
| 12 | SERPINA3 | -1.443334853 | 60 |
| 827 | CAPN6 | -1.618228454 | 61 |
| 5354 | PLP1 | -1.680375803 | 62 |
| 1164 | CKS2 | -1.700995591 | 63 |
| 8076 | MFAP5 | -1.724942849 | 64 |
| 84870 | RSPO3 | -2.50110156 | 65 |
| 100616163 | MIR4530 | -2.79787323 | 66 |
| 285016 | FAM150B | -3.055488057 | 67 |
| 27063 | ANKRD1 | -4.50925449 | 68 |

(continued)

| Entrez Gene | Gene | Total Delta HR | Rank |
|---|---|---|---|
| 7060 | THBS4 | -4.556568781 | 69 |
| 4316 | MMP7 | -4.78562355 | 70 |

• Internal Resection Validation: Delta HR performance measured in this dataset for the 70 signature Entrez genes is shown in Figure 2. This figure highlights the top 10 ranked Entrez genes in the signature which are the most important in retaining a good HR performance within this dataset. This ranking can also been found in Table 36 below:

| Entrez Gene | Gene | Total Delta HR | Rank |
|---|---|---|---|
| 3852 | KRT5 | 5.850910136 | 1 |
| 2354 | FOSB | 5.341991077 | 2 |
| 9232 | PTTG1 | 4.440300792 | 3 |
| 5179 | PENK | 4.359290179 | 4 |
| 340419 | RSPO2 | 3.715352525 | 5 |
| 563 | AZGP1 | 3.640373688 | 6 |
| 100616163 | MIR4530 | 3.034458226 | 7 |
| 7538 | ZFP36 | 2.900383458 | 8 |
| 4604 | MYBPC1 | 2.60456647 | 9 |
| 23764 | MAFF | 2.422195244 | 10 |
| 50652 | PCA3 | 2.343241624 | 11 |
| 50636 | ANO7 | 1.922305172 | 12 |
| 1803 | DPP4 | 1.747968953 | 13 |
| 693163 | MIR578 | 1.70934994 | 14 |
| 4057 | LTF | 1.457636816 | 15 |
| 1847 | DUSP5 | 1.441368066 | 16 |
| 7083 | TK1 | 1.432224235 | 17 |
| 101928017 | UBXN10-AS1 | 1.249812402 | 18 |
| 1164 | CKS2 | 1.152406332 | 19 |
| 23677 | SH3BP4 | 1.116227302 | 20 |
| 5121 | PCP4 | 1.047369238 | 21 |
| 152 | ADRA2C | 0.891075934 | 22 |
| 12 | SERPINA3 | 0.854606034 | 23 |
| 57481 | KIAA1210 | 0.762370469 | 24 |
| 3832 | KIF11 | 0.713624009 | 25 |
| 4489 | MT1A | 0.655338791 | 26 |
| 9506 | PAGE4 | 0.430978289 | 27 |
| 2138 | EYA1 | 0.384089193 | 28 |
| 91661 | ZNF765 | 0.309943842 | 29 |
| 284837 | #N/A | 0.303352744 | 30 |
| 25907 | TMEM158 | 0.247359339 | 31 |

(continued)

| Entrez Gene | Gene | Total Delta HR | Rank |
|---|---|---|---|
| 6614 | SIGLEC1 | 0.202684496 | 32 |
| 9076 | CLDN1 | 0.060049481 | 33 |
| 354 | KLK3 | -0.07704205 | 34 |
| 79054 | TRPM8 | -0.07716181 | 35 |
| 5054 | SERPINE1 | -0.083069191 | 36 |
| 84889 | SLC7A3 | -0.103594879 | 37 |
| 89689 | STEAP4 | -0.262219935 | 38 |
| 9955 | HS3ST3A1 | -0.310839602 | 39 |
| 130733 | TMEM178A | -0.328948061 | 40 |
| 10257 | ABCC4 | -0.420421537 | 41 |
| 51001 | MTERFD1 | -0.427114354 | 42 |
| 5346 | PLIN1 | -0.445607269 | 43 |
| 4588 | MUC6 | -0.452261632 | 44 |
| 644844 | PHGR1 | -0.527656877 | 45 |
| 283194 | #N/A | -0.623963891 | 46 |
| 29951 | PDZRN4 | -0.672143861 | 47 |
| 57176 | VARS2 | -0.673665413 | 48 |
| 6652 | SORD | -0.711615138 | 49 |
| 7739 | ZNF185 | -0.796601532 | 50 |
| 5317 | PKP1 | -0.91761911 | 51 |
| 6401 | SELE | -0.943930367 | 52 |
| 23784 | POTEH | -0.987487576 | 53 |
| 54097 | FAM3B | -1.064799882 | 54 |
| 5354 | PLP1 | -1.065316284 | 55 |
| 6422 | SFRP1 | -1.370192928 | 56 |
| 5166 | PDK4 | -1.863810081 | 57 |
| 84870 | RSPO3 | -2.4018171 | 58 |
| 56849 | TCEAL7 | -2.455318029 | 59 |
| 51050 | PI15 | -2.502066289 | 60 |
| 6406 | SEMG1 | -2.625125175 | 61 |
| 4316 | MMP7 | -3.015001652 | 62 |
| 2920 | CXCL2 | -3.051014073 | 63 |
| 406988 | MIR205HG | -3.231330366 | 64 |
| 285016 | FAM150B | -3.602511107 | 65 |
| 27063 | ANKRD1 | -3.836256996 | 66 |
| 1672 | DEFB1 | -4.174807907 | 67 |
| 8076 | MFAP5 | -4.187157544 | 68 |
| 827 | CAPN6 | -4.472033713 | 69 |

(continued)

| Entrez Gene | Gene | Total Delta HR | Rank |
|---|---|---|---|
| 7060 | THBS4 | -5.697080094 | 70 |

• Delta HR across these 2 datasets was evaluated to obtain a combined Entrez gene ranking for each of the signature Entrez genes. This is summarized in Table 37 below:

| Combined | | |
|---|---|---|
| Entrez Gene | Gene | Delta HR |
| 12 | SERPINA3 | 0.588728819 |
| 152 | ADRA2C | 2.309763834 |
| 354 | KLK3 | 0.243178342 |
| 563 | AZGP1 | 6.087335434 |
| 827 | CAPN6 | 6.090262167 |
| 1164 | CKS2 | 0.548589258 |
| 1672 | DEFB1 | 5.177919023 |
| 1803 | DPP4 | 1.60390272 |
| 1847 | DUSP5 | 1.6194786 |
| 2138 | EYA1 | 1.732443216 |
| 2354 | FOSB | 5.077080272 |
| 2920 | CXCL2 | 2.237233137 |
| 3832 | KIF11 | 2.004659943 |
| 3852 | KRT5 | 8.308388576 |
| 4057 | LTF | 2.168670336 |
| 4316 | MMP7 | 7.800625203 |
| 4489 | MT1A | 4.221083323 |
| 4588 | MUC6 | -1.02701054 |
| 4604 | MYBPC1 | 3.423984055 |
| 5054 | SERPINE1 | 0.559998768 |
| 5121 | PCP4 | 3.487897386 |
| 5166 | PDK4 | 0.675467876 |
| 5179 | PENK | 6.001995681 |
| 5317 | PKP1 | 1.082613399 |
| 5346 | PLIN1 | 0.532141166 |
| 5354 | PLP1 | 2.745692087 |
| 6401 | SELE | 3.817194522 |
| 6406 | SEMG1 | 1.856201393 |
| 6422 | SFRP1 | 1.231622942 |
| 6614 | SIGLEC1 | 1.058219648 |
| 6652 | SORD | 0.716101659 |
| 7060 | THBS4 | 10.25364888 |

(continued)

| Combined | | |
|---|---|---|
| **Entrez Gene** | **Gene** | **Delta HR** |
| 7083 | TK1 | 2.109762169 |
| 7538 | ZFP36 | 2.057694688 |
| 7739 | ZNF185 | 2.000259592 |
| 8076 | MFAP5 | 5.912100393 |
| 9076 | CLDN1 | 1.014396437 |
| 9232 | PTTG1 | 4.183523517 |
| 9506 | PAGE4 | 0.771051772 |
| 9955 | HS3ST3A1 | 0.438399729 |
| 10257 | ABCC4 | -0.9109417 |
| 23677 | SH3BP4 | 2.341214123 |
| 23764 | MAFF | 2.20725298 |
| 23784 | POTEH | 0.056059594 |
| 25907 | TMEM158 | 1.724346854 |
| 27063 | ANKRD1 | 8.345511486 |
| 29951 | PDZRN4 | 0.251538716 |
| 50636 | ANO7 | 3.270966342 |
| 50652 | PCA3 | 2.659017364 |
| 51001 | MTERFD1 | 1.198102909 |
| 51050 | PI15 | -0.14830814 |
| 54097 | FAM3B | - |
| | | 1.274696959 |
| 56849 | TCEAL7 | 2.585993869 |
| 57176 | VARS2 | -0.02003256 |
| 57481 | KIAA1210 | 1.866022273 |
| 79054 | TRPM8 | 0.429662725 |
| 79689 | STEAP4 | 0.003969308 |
| 84870 | RSPO3 | -4.90291866 |
| 84889 | SLC7A3 | 1.257450797 |
| 91661 | ZNF765 | 0.113068252 |
| 130733 | TMEM178A | 1.463299061 |
| 283194 | #N/A | 1.179206229 |
| 284837 | #N/A | 0.146303844 |
| 285016 | FAM150B | 6.657999164 |
| 340419 | RSPO2 | 7.4032047 |
| 406988 | MIR205HG | 3.534398055 |
| 644844 | PHGR1 | 1.067000018 |
| 693163 | MIR578 | 1.827836834 |

(continued)

| Combined | | |
|---|---|---|
| Entrez Gene | Gene | Delta HR |
| 100616163 | MIR4530 | 0.236584996 |
| 101928017 | UBXN10-AS1 | 1.318500539 |

The ranks assigned to the signature Entrez genes based on the combined core set analysis is summarized in Table 38 below:

| Entrez Gene | Gene | Total Delta HR | Rank |
|---|---|---|---|
| 3852 | KRT5 | 8.308388576 | 1 |
| 340419 | RSPO2 | 7.4032047 | 2 |
| 563 | AZGP1 | 6.087335434 | 3 |
| 5179 | PENK | 6.001995681 | 4 |
| 2354 | FOSB | 5.077080272 | 5 |
| 4489 | MT1A | 4.221083323 | 6 |
| 9232 | PTTG1 | 4.183523517 | 7 |
| 6401 | SELE | 3.817194522 | 8 |
| 5121 | PCP4 | 3.487897386 | 9 |
| 4604 | MYBPC1 | 3.423984055 | 10 |
| 50636 | ANO7 | 3.270966342 | 11 |
| 50652 | PCA3 | 2.659017364 | 12 |
| 23677 | SH3BP4 | 2.341214123 | 13 |
| 152 | ADRA2C | 2.309763834 | 14 |
| 23764 | MAFF | 2.20725298 | 15 |
| 4057 | LTF | 2.168670336 | 16 |
| 7083 | TK1 | 2.109762169 | 17 |
| 7538 | ZFP36 | 2.057694688 | 18 |
| 3832 | KIF11 | 2.004659943 | 19 |
| 57481 | KIAA1210 | 1.866022273 | 20 |
| 693163 | MIR578 | 1.827836834 | 21 |
| 2138 | EYA1 | 1.732443216 | 22 |
| 25907 | TMEM158 | 1.724346854 | 23 |
| 1847 | DUSP5 | 1.6194786 | 24 |
| 1803 | DPP4 | 1.60390272 | 25 |
| 101928017 | UBXN10-AS1 | 1.318500539 | 26 |
| 6614 | SIGLEC1 | 1.058219648 | 27 |
| 9506 | PAGE4 | 0.771051772 | 28 |
| 9955 | HS3ST3A1 | 0.438399729 | 29 |
| 79054 | TRPM8 | 0.429662725 | 30 |
| 100616163 | MIR4530 | 0.236584996 | 31 |

(continued)

| Entrez Gene | Gene | Total Delta HR | Rank |
|---|---|---|---|
| 23784 | POTEH | 0.056059594 | 32 |
| 79689 | STEAP4 | 0.003969308 | 33 |
| 57176 | VARS2 | -0.02003256 | 34 |
| 91661 | ZNF765 | -0.113068252 | 35 |
| 284837 | #N/A | -0.146303844 | 36 |
| 51050 | PI15 | -0.14830814 | 37 |
| 354 | KLK3 | -0.243178342 | 38 |
| 29951 | PDZRN4 | -0.251538716 | 39 |
| 5346 | PLIN1 | -0.532141166 | 40 |
| 1164 | CKS2 | -0.548589258 | 41 |
| 5054 | SERPINE1 | -0.559998768 | 42 |
| 12 | SERPINA3 | -0.588728819 | 43 |
| 5166 | PDK4 | -0.675467876 | 44 |
| 6652 | SORD | -0.716101659 | 45 |
| 10257 | ABCC4 | -0.9109417 | 46 |
| 9076 | CLDN1 | -1.014396437 | 47 |
| 4588 | MUC6 | -1.02701054 | 48 |
| 644844 | PHGR1 | -1.067000018 | 49 |
| 5317 | PKP1 | -1.082613399 | 50 |
| 283194 | #N/A | -1.179206229 | 51 |
| 51001 | MTERFD1 | -1.198102909 | 52 |
| 6422 | SFRP1 | -1.231622942 | 53 |
| 84889 | SLC7A3 | -1.257450797 | 54 |
| 54097 | FAM3B | -1.274696959 | 55 |
| 130733 | TMEM178A | -1.463299061 | 56 |
| 6406 | SEMG1 | -1.856201393 | 57 |
| 7739 | ZNF185 | -2.000259592 | 58 |
| 2920 | CXCL2 | -2.237233137 | 59 |
| 56849 | TCEAL7 | -2.585993869 | 60 |
| 5354 | PLP1 | -2.745692087 | 61 |
| 406988 | MIR205HG | -3.534398055 | 62 |
| 84870 | RSPO3 | -4.90291866 | 63 |
| 1672 | DEFB1 | -5.177919023 | 64 |
| 8076 | MFAP5 | -5.912100393 | 65 |
| 827 | CAPN6 | -6.090262167 | 66 |
| 285016 | FAM150B | -6.657999164 | 67 |
| 4316 | MMP7 | -7.800625203 | 68 |
| 27063 | ANKRD1 | -8.345511486 | 69 |

(continued)

| Entrez Gene | Gene | Total Delta HR | Rank |
|---|---|---|---|
| 7060 | THBS4 | -10.25364888 | 70 |

**Minimum gene analysis**

The results for the minimum gene analysis of the 70 gene signature in 2 datasets is provided in this section.

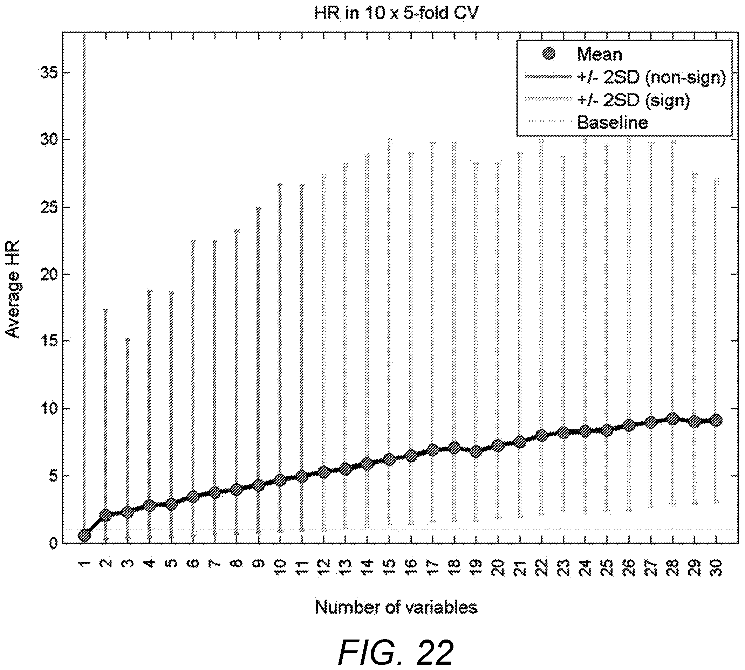

• FASTMAN Biopsy Validation: The average HR performance measured in this dataset using the random sampling of the signature Entrez genes from a feature length of 1 to 30 is shown in Figure 22. This figure shows that to retain a significant HR performance (i.e. lower CI of HR > 1) a minimum of 12 of the signature Entrez genes must be selected.

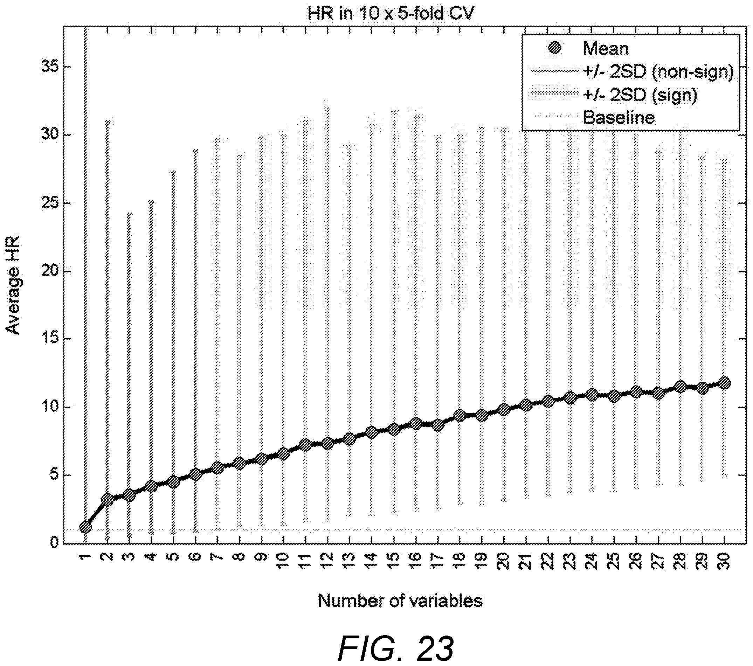

• Internal Resection Validation: The average HR performance measured in this dataset using the random sampling of the signature Entrez genes from a feature length of 1 to 30 is shown in Figure 23. This figure shows that to retain a significant HR performance (i.e. lower CI of HR > 1) a minimum of 7 of the signature Entrez genes must be selected.

**Claims**

1. A method for characterising and/or prognosing cancer in a subject with a primary cancer but no known metastases comprising:
determining the expression level of THBS4 and at least 11 genes selected from Table 1 in a sample comprising nucleic acids from the primary tumour from the subject wherein the determined expression level is used to provide a characterisation of and/or a prognosis for the cancer, wherein the characterisation of and/or prognosis for the cancer consists of predicting an increased likelihood of recurrence and/or predicting an increased likelihood of metastasis and/or a poor prognosis.

2. A method for selecting a treatment for cancer in a subject with a primary cancer but no known metastases comprising:

    (a) determining the expression level of THBS4 and at least 11 genes selected from Table 1 in a sample comprising nucleic acids from the primary tumour from the subject wherein the determined expression level is used to provide a characterisation of and/or a prognosis for the cancer and
    (b) selecting a treatment appropriate to the characterisation of and/or prognosis for the cancer,

    wherein the characterisation of and/or prognosis for the cancer consists of predicting an increased likelihood of recurrence and/or predicting an increased likelihood of metastasis and/or a poor prognosis.

3. The method of claim 2, wherein if the characterisation of and/or prognosis for the cancer is an increased likelihood of recurrence and/or metastasis and/or a poor prognosis the treatment selected is one or more of

    a) an anti-hormone treatment, preferably bicalutamide and/or abiraterone
    b) a cytotoxic agent;
    c) a biologic, preferably an antibody and/or a vaccine, more preferably Sipuleucel-T
    d) radiotherapy, optionally extended radiotherapy, preferably extended-field radiotherapy
    e) targeted therapy
    f) surgery.

4. The method of claim 2 wherein if the characterisation of and/or prognosis for the cancer is no increased likelihood of recurrence and/or metastasis and/or a poor prognosis no treatment, or no further treatment, is selected, optionally wherein the cancer is subsequently monitored to determine whether treatment, or further treatment, is required.

5. The method of any preceding claim wherein

    (a) the cancer comprises or is prostate cancer or ER positive breast cancer;
    (b) the method further comprises determining PSA levels and/or Gleason score in the subject and using the determined PSA levels and/or Gleason score in combination with the determined expression levels to provide a characterization and/or prognosis for the cancer (including diagnosing whether the cancer has increased metastatic potential); or

(c) the cancer comprises or is prostate cancer or ER positive breast cancer and the method further comprises determining PSA levels and/or Gleason score in the subject and using the determined PSA levels and/or Gleason score in combination with the determined expression levels to provide a characterization and/or prognosis for the cancer (including diagnosing whether the cancer has increased metastatic potential).

6. The method of any preceding claim wherein determining the expression levels employs:

(a) primers (primer pairs) and/or probes that hybridize with at least one of the full sequences or target sequences from Table 1; and/or
(b) at least one probe and/or probeset from Table 1/1A; and/or
(c) at least one primer and/or primer pair from Table 1B and/or of SEQ ID NOs 3151-3154.

7. The method of any preceding claim comprising comparing the expression level to a reference value or to the expression level in one or more control samples.

8. The method of any preceding claim wherein the expression level is

(a) compared to the expression level of the same gene in one or more control samples;
(b) determined by microarray, northern blotting, RNA-seq (RNA sequencing), in situ RNA detection or nucleic acid amplification; or
(c) compared to the expression level of the same gene in one or more control samples and determined by microarray, northern blotting, RNA-seq (RNA sequencing), in situ RNA detection or nucleic acid amplification.

9. The method of any preceding claim further comprising extracting total RNA from the sample.

10. The method of any preceding claim wherein a signature score is derived from the measured expression levels, optionally according to the formula:

$$SignatureScore = \sum_i w_i \times (ge_i - b_i) + k$$

Where $w_i$ is a weight for each gene, $b_i$ is a gene-specific bias, $ge_i$ is the gene expression after pre-processing, and $k$ is a constant offset.

11. The method of any preceding claim wherein the sample comprises, consists essentially of or consists of

(a) prostate cells and/or tissue or breast cells and/or tissue;
(b) a formalin-fixed paraffin-embedded biopsy sample or a resection sample; or
(c) a formalin-fixed paraffin-embedded biopsy sample or a resection sample comprising prostate cells and/or tissue or breast cells and/or tissue.

12. The method of any preceding claim wherein

(a) an increased expression level of at least one gene selected from Table 1 with a positive weight indicates an increased likelihood of recurrence and/or metastasis and/or a poor prognosis; or
(b) a decreased expression level of at least one gene selected from Table 1 with a negative weight indicates an increased likelihood of recurrence and/or metastasis and/or a poor prognosis; or
(c) an increased expression level of at least one gene selected from Table 1 with a positive weight and a decreased expression level of at least one gene selected from Table 1 with a negative weight indicates an increased likelihood of recurrence and/or metastasis and/or a poor prognosis.

13. The method of claim 10 or any claim dependent therefrom wherein

(a) a signature score above threshold indicates an increased likelihood of recurrence and/or metastasis and/or a poor prognosis;

(b) a signature score equal to or above threshold indicates an increased likelihood of recurrence and/or metastasis and/or a poor prognosis; and/or

(c) the signature score is calculated for measured gene expression levels of the genes in a signature selected from the signatures of Tables 1 and 11 to 24 which comprise THBS4.

14. A chemotherapeutic agent for use in treating cancer in a subject,
wherein the subject is selected for treatment on the basis of a method as claimed in claim 2 or 3 or any claim dependent thereon.

15. The chemotherapeutic agent for use of claim 14
wherein the chemotherapeutic agent comprises, consists essentially of or consists of

a) an anti-hormone treatment, preferably bicalutamide and/or abiraterone
b) a cytotoxic agent
c) a biologic, preferably an antibody and/or a vaccine, more preferably Sipuleucel-T and/or
d) a targeted therapeutic agent

16. The method of claim 3 or any claim dependent thereon, or chemotherapeutic agent for use of claim 15 wherein the cytotoxic agent is a platinum based agent and/or a taxane; optionally wherein

(a) the platinum based agent is selected from cisplatin, carboplatin and oxaliplatin; or
(b) the taxane is paclitaxel or docetaxel.

17. A system, device or test kit for characterising and/or prognosing cancer in a subject with a primary cancer but no known metastases, comprising:

a) one or more testing devices for determining the expression level of THBS4 and at least 11 genes selected from Table 1 in a sample comprising nucleic acids from the primary tumour from the subject
b) a processor; and
c) storage medium comprising a computer application that, when executed by the processor, is configured to:

(i) access and/or calculate the determined expression levels of the genes selected from Table 1 in the sample on the one or more testing devices
(ii) calculate whether there is an increased or decreased level of the genes selected from Table 1 in the sample; and
(iii) output from the processor the characterization of and/or prognosis for the cancer, wherein the characterisation of and/or prognosis for the cancer consists of predicting an increased likelihood of recurrence and/or predicting an increased likelihood of metastasis and/or a poor prognosis.

18. The system, device or test kit of claim 17 which is adapted to perform the method of any one of claims 1 to 16.

19. A computer application or storage medium comprising a computer application as defined in claim 17 or claim 18.

20. A computer program product for characterising and/or prognosing cancer in a subject with a primary cancer but no known metastases, comprising a non-transitory computer-readable storage device having computer-readable program instructions embodied thereon that cause the computer to:

(i) access and/or calculate the determined expression levels of THBS4 and at least 11 genes selected from Table 1 in a sample comprising nucleic acids from the primary tumour on one or more testing devices;
(ii) calculate whether there is an increased or decreased level of the genes selected from Table 1 in the sample; and,
(iii) provide an output regarding the characterization of and/or prognosis for the cancer, wherein the characterisation of and/or prognosis for the cancer consists of predicting an increased likelihood of recurrence and/or predicting an increased likelihood of metastasis and/or a poor prognosis.

**Patentansprüche**

1. Verfahren zur Charakterisierung und/oder Prognose von Krebs in einem Subjekt mit einem Primärkrebs, aber keinen bekannten Metastasen, umfassend:

   Bestimmen des Expressionsniveaus von THBS4 und wenigstens 11 Genen, die aus Tabelle 1 ausgewählt sind, in einer Probe, umfassend Nukleinsäuren aus dem Primärtumor von dem Subjekt, wobei das bestimmte Expressionsniveau verwendet wird, um eine Charakterisierung des Krebses und/oder eine Prognose für den Krebs bereitzustellen, wobei die Charakterisierung des Krebses und/oder die Prognose für den Krebs aus der Vorhersage einer erhöhten Wahrscheinlichkeit eines Wiederauftretens und/oder der Vorhersage einer erhöhten Wahrscheinlichkeit einer Metastasierung und/oder einer schlechten Prognose besteht.

2. Verfahren zur Auswahl einer Behandlung von Krebs in einem Subjekt mit einem Primärkrebs, aber keinen bekannten Metastasen, umfassend:

   (a) Bestimmen des Expressionsniveaus von THBS4 und wenigstens 11 Genen, die aus Tabelle 1 ausgewählt sind, in einer Probe, umfassend Nukleinsäuren aus dem Primärtumor von dem Subjekt, wobei das bestimmte Expressionsniveau verwendet wird, um eine Charakterisierung des Krebses und/oder eine Prognose für den Krebs bereitzustellen, und
   (b) Auswählen einer Behandlung, die für die Charakterisierung des Krebses und/oder der Prognose für den Krebs geeignet ist,

   wobei die Charakterisierung des Krebses und/oder die Prognose für den Krebs aus der Vorhersage einer erhöhten Wahrscheinlichkeit eines Wiederauftretens und/oder der Vorhersage einer erhöhten Wahrscheinlichkeit einer Metastasierung und/oder einer schlechten Prognose besteht.

3. Verfahren nach Anspruch 2, wobei die Behandlung, wenn die Charakterisierung des Krebses und/oder die Prognose für den Krebs eine erhöhte Wahrscheinlichkeit eines Wiederauftretens und/oder einer Metastasierung und/oder einer schlechten Prognose ist, ausgewählt ist aus einer oder mehreren von

   a) einer Antihormonbehandlung, vorzugsweise Bicalutamid und/oder Abirateron
   b) einem zytotoxischen Mittel
   c) einem Biologikum, vorzugsweise einem Antikörper und/oder einem Impfstoff, mehr bevorzugt Sipuleucel-T
   d) Strahlentherapie, gegebenenfalls erweiterte Strahlentherapie, vorzugsweise Extended-Field-Strahlentherapie
   e) gezielter Therapie
   f) Chirurgie.

4. Verfahren nach Anspruch 2, wobei keine Behandlung oder keine weitere Behandlung ausgewählt ist, wenn die Charakterisierung des Krebses und/oder die Prognose für den Krebs keine erhöhte Wahrscheinlichkeit eines Wiederauftretens und/oder Metastasierung und/oder einer schlechten Prognose ist, wobei der Krebs gegebenenfalls anschließend überwacht wird, um festzustellen, ob Behandlung oder eine weitere Behandlung erforderlich ist.

5. Verfahren nach einem der vorhergehenden Ansprüche, wobei

   (a) der Krebs Prostatakrebs oder ER-positiven Brustkrebs umfasst oder ist;
   (b) das Verfahren ferner Bestimmen von PSA-Niveaus und/oder Gleason-Score in dem Subjekt und Verwenden der bestimmten PSA-Niveaus und/oder dem Gleason-Score in Kombination mit den bestimmten Expressionsniveaus umfasst, um eine Charakterisierung des Krebses und/oder Prognose für den Krebs bereitzustellen (einschließlich der Diagnose, ob der Krebs erhöhtes metastatisches Potenzial aufweist); oder
   (c) der Krebs Prostatakrebs oder ER-positiven Brustkrebs umfasst oder ist und das Verfahren ferner Bestimmen von PSA-Niveaus und/oder Gleason-Score in dem Patienten und Verwenden der bestimmten PSA-Niveaus und/oder dem Gleason-Score in Kombination mit den bestimmten Expressionsniveaus umfasst, um eine Charakterisierung und/oder Prognose für den Krebs bereitzustellen (einschließlich der Diagnose, ob der Krebs erhöhtes metastatisches Potenzial aufweist).

6. Verfahren nach einem der vorhergehenden Ansprüche, wobei Bestimmen der Expressionsniveaus Folgendes einschließt:

(a) Primer (Primerpaare) und/oder Sonden, die mit wenigstens einer der vollständigen Sequenzen oder Zielsequenzen aus Tabelle 1 hybridisieren; und/oder

(b) wenigstens eine Sonde und/oder einen Sondensatz aus Tabelle 1/1A; und/oder

(c) wenigstens einen Primer und/oder ein Primerpaar aus Tabelle 1B und/oder von SEQ ID NOs 3151-3154.

7. Verfahren nach einem der vorhergehenden Ansprüche, umfassend Vergleichen des Expressionsniveaus mit einem Referenzwert oder mit dem Expressionsniveau in einer oder mehreren Kontrollproben.

8. Verfahren nach einem der vorhergehenden Ansprüche, wobei das Expressionsniveau

(a) mit dem Expressionsniveau desselben Gens in einer oder mehreren Kontrollproben verglichen wird;

(b) durch Microarray, Northern Blotting, RNA-seq (RNA-Sequenzierung), in situ RNA-Nachweis oder Nukleinsäureamplifikation bestimmt wird; oder

(c) mit dem Expressionsniveau desselben Gens in einer oder mehreren Kontrollproben verglichen wird und durch Microarray, Northern Blotting, RNA-seq (RNA-Sequenzierung), in situ RNA-Nachweis oder Nukleinsäureamplifikation bestimmt wird.

9. Verfahren nach einem der vorhergehenden Ansprüche, ferner umfassend Extrahieren von Gesamt-RNA aus der Probe.

10. Verfahren nach einem der vorhergehenden Ansprüche, wobei ein Signaturwert von den gemessenen Expressionsniveaus abgeleitet ist, gegebenenfalls gemäß der Formel:

$$\textbf{\textit{Signaturwert}} = \sum_i w_i \times (ge_i - b_i) + k$$

wobei $w_i$ ein Gewicht für jedes Gen ist, $b_i$ eine genspezifische Verzerrung ist, $ge_i$ die Genexpression nach Vorverarbeitung ist, und $k$ ein konstanter Offset ist.

11. Verfahren nach einem der vorhergehenden Ansprüche, wobei die Probe umfasst, im Wesentlichen besteht aus oder besteht aus:

(a) Prostatazellen und/oder -gewebe oder Brustzellen und/oder -gewebe;

(b) einer formalinfixierten, in Paraffin eingebetteten Biopsieprobe oder einer Resektionsprobe; oder

(c) einer formalinfixierten, in Paraffin eingebetteten Biopsieprobe oder einer Resektionsprobe, die Prostatazellen und/oder -gewebe oder Brustzellen und/oder -gewebe umfasst.

12. Verfahren nach einem der vorhergehenden Ansprüche, wobei

(a) ein erhöhtes Expressionsniveau wenigstens eines Gens, das aus Tabelle 1 ausgewählt ist, mit einem positiven Gewicht, eine erhöhte Wahrscheinlichkeit eines Wiederauftretens und/oder Metastasierung und/oder einer schlechten Prognose anzeigt; oder

(b) ein vermindertes Expressionsniveau wenigstens eines Gens, das aus Tabelle 1 ausgewählt ist, mit einem negativen Gewicht, eine erhöhte Wahrscheinlichkeit eines Wiederauftretens und/oder Metastasierung und/oder einer schlechten Prognose anzeigt; oder

(c) ein erhöhtes Expressionsniveau wenigstens eines Gens, das aus Tabelle 1 ausgewählt ist, mit einem positiven Gewicht, und ein vermindertes Expressionsniveau wenigstens eines Gens mit einem negativen Gewicht eine erhöhte Wahrscheinlichkeit eines Wiederauftretens und/oder Metastasierung und/oder einer schlechten Prognose anzeigt.

13. Verfahren nach Anspruch 10 oder einem beliebigen davon abhängigen Anspruch, wobei

(a) ein Signaturwert über dem Schwellenwert eine erhöhte Wahrscheinlichkeit eines Wiederauftretens und/oder Metastasierung und/oder einer schlechten Prognose anzeigt;

(b) ein Signaturwert gleich dem oder über dem Schwellenwert eine erhöhte Wahrscheinlichkeit eines Wiederauftretens und/oder Metastasierung und/oder einer schlechten Prognose anzeigt; und/oder

(c) der Signaturwert für gemessene Genexpressionsniveaus der Gene in einer Signatur berechnet wird, die aus den Signaturen von Tabellen 1 und 11 bis 24 ausgewählt ist, die THBS4 umfassen.

14. Chemotherapeutisches Mittel zur Verwendung bei der Behandlung von Krebs in einem Subjekt, wobei das Subjekt zur Behandlung auf der Grundlage eines Verfahrens nach Anspruch 2 oder 3 oder einem davon abhängigen Anspruch ausgewählt ist.

15. Chemotherapeutisches Mittel zur Verwendung nach Anspruch 14, wobei das chemotherapeutische Mittel umfasst, im Wesentlichen besteht aus oder besteht aus:

a) einer Antihormonbehandlung, vorzugsweise Bicalutamid und/oder Abirateron
b) einem zytotoxischen Mittel
c) einem Biologikum, vorzugsweise einem Antikörper und/oder einem Impfstoff, mehr bevorzugt Sipuleucel-T und/oder
d) einem zielgerichteten therapeutischen Mittel.

16. Verfahren nach Anspruch 3 oder einem beliebigen davon abhängigen Anspruch oder chemotherapeutisches Mittel zur Verwendung nach Anspruch 15, wobei das zytotoxische Mittel ein platinbasiertes Mittel und/oder ein Taxan ist; wobei gegebenenfalls

(a) das platinbasierte Mittel ausgewählt ist aus Cisplatin, Carboplatin und Oxaliplatin; oder
(b) das Taxan Paclitaxel oder Docetaxel ist.

17. System, Vorrichtung oder Testkit zur Charakterisierung und/oder Prognose von Krebs in einem Subjekt mit einem Primärkrebs, aber keinen bekannten Metastasen, umfassend:

a) eine oder mehrere Testvorrichtungen zur Bestimmung des Expressionsniveaus von THBS4 und wenigstens 11 Genen, die aus Tabelle 1 ausgewählt sind, in einer Probe, die Nukleinsäuren aus dem Primärtumor des Subjekts umfasst
b) einen Prozessor; und
c) ein Speichermedium, das eine Computeranwendung umfasst, die, wenn sie von dem Prozessor ausgeführt wird, konfiguriert ist, um:

(i) auf die bestimmten Expressionsniveaus der Gene, die aus Tabelle 1 ausgewählt sind, in der Probe auf der einen oder den mehreren Testvorrichtungen zuzugreifen und/oder diese zu berechnen
(ii) zu berechnen, ob es ein erhöhtes oder verringertes Niveau der Gene, die aus Tabelle 1 ausgewählt sind, in der Probe gibt; und
(iii) Ausgeben der Charakterisierung des Krebses und/oder Prognose für den Krebs durch den Prozessor, wobei die Charakterisierung des Krebses und/oder Prognose für den Krebs aus der Vorhersage einer erhöhten Wahrscheinlichkeit eines Wiederauftretens und/oder Vorhersage einer erhöhten Wahrscheinlichkeit einer Metastasierung und/oder einer schlechten Prognose besteht.

18. System, Vorrichtung oder Testkit nach Anspruch 17, das/die zur Durchführung des Verfahrens nach einem der Ansprüche 1 bis 16 geeignet ist.

19. Computeranwendung oder Speichermedium, das eine Computeranwendung umfasst, wie in Anspruch 17 oder Anspruch 18 definiert.

20. Computerprogrammprodukt für eine Charakterisierung des Krebses und/oder eine Prognose für den Krebs in einem Subjekt mit einem Primärkrebs, aber keinen bekannten Metastasen, umfassend eine nicht-transitorische computerlesbare Speichervorrichtung mit darauf enthaltenen computerlesbaren Programmanweisungen, die den Computer veranlassen,

(i) auf die bestimmten Expressionsniveaus von THBS4 und wenigstens 11 Genen, die aus Tabelle 1 ausgewählt sind, in einer Probe, die Nukleinsäuren aus dem Primärtumor umfasst, auf einer oder mehreren Testvorrichtungen zuzugreifen und/oder diese zu berechnen;
(ii) zu berechnen, ob ein erhöhtes oder vermindertes Niveau der aus Tabelle 1 ausgewählten Gene in der Probe vorhanden ist; und,
(iii) Bereitstellen einer Ausgabe bezüglich der Charakterisierung des Krebses und/oder Prognose für den Krebs, wobei die Charakterisierung des Krebses und/oder Prognose für den Krebs aus der Vorhersage einer erhöhten Wahrscheinlichkeit eines Wiederauftretens und/oder der Vorhersage einer erhöhten Wahrscheinlichkeit einer Metastasierung und/oder einer schlechten Prognose besteht.

**Revendications**

1. Procédé pour caractériser et/ou pronostiquer un cancer chez un sujet atteint d'un cancer primitif mais sans aucune métastase connue, comprenant :
la détermination du taux d'expression de THBS4 et d'au moins 11 gènes sélectionnés dans le Tableau 1 dans un échantillon comprenant des acides nucléiques de la tumeur primitive du sujet, dans lequel le taux d'expression déterminé est utilisé pour établir une caractérisation et/ou un pronostic du cancer, dans lequel la caractérisation et/ou le pronostic du cancer consiste en la prédiction d'une probabilité accrue de récidive et/ou la prédiction d'une probabilité accrue de métastase et/ou de mauvais pronostic.

2. Procédé pour sélectionner un traitement du cancer chez un sujet atteint d'un cancer primitif mais sans aucune métastase connue, comprenant :

   (a) la détermination du taux d'expression de THBS4 et d'au moins 11 gènes sélectionnés dans le Tableau 1 dans un échantillon comprenant des acides nucléiques de la tumeur primitive du sujet, dans lequel le taux d'expression déterminé est utilisé pour établir une caractérisation et/ou un pronostic du cancer et
   (b) la sélection d'un traitement approprié pour la caractérisation et/ou le pronostic du cancer,

   dans lequel la caractérisation et/ou le pronostic du cancer consiste en la prédiction d'une probabilité accrue de récidive et/ou la prédiction d'une probabilité accrue de métastase et/ou de mauvais pronostic.

3. Procédé selon la revendication 2, dans lequel si la caractérisation et/ou le pronostic du cancer est une probabilité accrue de récidive et/ou de métastase et/ou de mauvais pronostic, le traitement sélectionné est un ou plusieurs parmi :

   a) un traitement antihormonal, de préférence le bicalutamide et/ou l'abiratérone
   b) un agent cytotoxique ;
   c) un agent biologique, de préférence un anticorps et/ou un vaccin, de manière davantage préférée le Sipuleucel-T
   d) la radiothérapie, facultativement une radiothérapie étendue, de préférence une radiothérapie à champ étendu
   e) une thérapie ciblée
   f) une intervention chirurgicale.

4. Procédé selon la revendication 2, dans lequel si la caractérisation et/ou le pronostic du cancer est aucune probabilité accrue de récidive et/ou de métastase et/ou de mauvais pronostic, aucun traitement, ou aucun traitement supplémentaire, n'est sélectionné, facultativement dans lequel le cancer est ensuite surveillé pour déterminer si un traitement, ou un traitement supplémentaire, est requis.

5. Procédé selon une quelconque revendication précédente, dans lequel

   (a) le cancer comprend ou est le cancer de la prostate ou le cancer du sein ER-positif ;
   (b) le procédé comprend en outre la détermination des taux de PSA et/ou du score de Gleason chez le sujet et l'utilisation des taux de PSA déterminés et/ou du score de Gleason en combinaison avec les taux d'expression déterminés afin d'établir une caractérisation et/ou un pronostic du cancer (incluant le fait de diagnostiquer si le cancer présente un potentiel métastatique accru) ; ou
   (c) le cancer comprend ou est le cancer de la prostate ou le cancer du sein ER-positif et le procédé comprend en outre la détermination des taux de PSA et/ou du score de Gleason chez le sujet et l'utilisation des taux de PSA déterminés et/ou du score de Gleason en combinaison avec les taux d'expression déterminés afin d'établir une caractérisation et/ou un pronostic du cancer (incluant le fait de diagnostiquer si le cancer présente un potentiel métastatique accru) .

6. Procédé selon une quelconque revendication précédente, dans lequel la détermination des taux d'expression emploie :

(a) des amorces (paires d'amorces) et/ou des sondes qui s'hybrident avec au moins une des séquences entières ou séquences cibles du Tableau 1 ; et/ou
(b) au moins une sonde et/ou un jeu de sondes du Tableau 1/1A ; et/ou
(c) au moins une amorce et/ou paire d'amorces du Tableau 1B et/ou de SEQ ID NO: 3151-3154.

**7.** Procédé selon une quelconque revendication précédente, comprenant la comparaison du taux d'expression à une valeur de référence ou au taux d'expression dans un ou plusieurs échantillons de contrôle.

**8.** Procédé selon une quelconque revendication précédente, dans lequel le taux d'expression est

(a) comparé au taux d'expression du même gène dans un ou plusieurs échantillons de contrôle ;
(b) déterminé par un microréseau, un transfert de Northern, un RNA-seq (séquençage de l'ARN), une détection d'ARN *in situ* ou une amplification d'acide nucléique ; ou
(c) comparé au taux d'expression du même gène dans un ou plusieurs échantillons de contrôle et déterminé par un microréseau, un transfert de Northern, un RNA-seq (séquençage de l'ARN), une détection d'ARN *in situ* ou une amplification d'acide nucléique.

**9.** Procédé selon une quelconque revendication précédente, comprenant en outre l'extraction de l'ARN total de l'échantillon.

**10.** Procédé selon une quelconque revendication précédente, dans lequel un score de signature est dérivé des taux d'expression mesurés, facultativement selon la formule :

$$Score\ de\ signature = \sum_i w_i \times (ge_i - b_i) + k$$

dans laquelle $w_i$ est un poids pour chaque gène, $b_i$ est un biais spécifique au gène, $ge_i$ est l'expression du gène après un prétraitement, et $k$ est une constante de décalage.

**11.** Procédé selon une quelconque revendication précédente, dans lequel l'échantillon comprend, consiste essentiellement en ou consiste en

(a) des cellules et/ou un tissu de la prostate et/ou des cellules et/ou un tissu du sein ;
(b) un échantillon de biopsie inclus en paraffine et fixé au formol ou un échantillon de résection ; ou
(c) un échantillon de biopsie inclus en paraffine et fixé au formol ou un échantillon de résection comprenant des cellules et/ou un tissu de la prostate et/ou des cellules et/ou un tissu du sein.

**12.** Procédé selon une quelconque revendication précédente, dans lequel

(a) un taux d'expression augmenté d'au moins un gène sélectionné dans le Tableau 1 ayant un poids positif indique une probabilité accrue de récidive et/ou de métastase et/ou de mauvais pronostic ; ou
(b) un taux d'expression réduit d'au moins un gène sélectionné dans le Tableau 1 ayant un poids négatif indique une probabilité accrue de récidive et/ou de métastase et/ou de mauvais pronostic ; ou
(c) un taux d'expression augmenté d'au moins un gène sélectionné dans le Tableau 1 ayant un poids positif et un taux d'expression réduit d'au moins un gène sélectionné dans le Tableau 1 ayant un poids négatif indique une probabilité accrue de récidive et/ou de métastase et/ou de mauvais pronostic.

**13.** Procédé selon la revendication 10 ou une quelconque revendication dépendante de celle-ci, dans lequel

(a) un score de signature supérieur à une valeur seuil indique une probabilité accrue de récidive et/ou de métastase et/ou de mauvais pronostic ;
(b) un score de signature supérieur ou égal à une valeur seuil indique une probabilité accrue de récidive et/ou de métastase et/ou de mauvais pronostic ; et/ou
(c) le score de signature est calculé pour les taux d'expression génique mesurés des gènes dans une signature sélectionnée parmi les signatures des Tableaux 1 et 11 à 24 qui comprennent THBS4.

**14.** Agent de chimiothérapie destiné à être utilisé dans le traitement du cancer chez un sujet,

où le sujet est sélectionné pour un traitement sur la base d'un procédé selon la revendication 2 ou 3 ou une quelconque revendication dépendante de celles-ci.

15. Agent de chimiothérapie destiné à être utilisé selon la revendication 14
où l'agent de chimiothérapie comprend, consiste essentiellement en ou consiste en

a) un traitement antihormonal, de préférence le bicalutamide et/ou l'abiratérone
b) un agent cytotoxique
c) un agent biologique, de préférence un anticorps et/ou un vaccin, de manière davantage préférée le Sipuleucel-T et/ou
d) un agent thérapeutique ciblé.

16. Procédé selon la revendication 3 ou une quelconque revendication dépendante de celle-ci, ou agent de chimiothérapie destiné à être utilisé selon la revendication 15, dans lequel l'agent cytotoxique est un agent à base de platine et/ou un taxane ; facultativement dans lequel

(a) l'agent à base de platine est sélectionné parmi le cisplatine, le carboplatine et l'oxaliplatine ; ou
(b) le taxane est le paclitaxel ou le docétaxel.

17. Système, dispositif ou kit de test pour caractériser et/ou pronostiquer un cancer chez un sujet atteint d'un cancer primitif mais sans aucune métastase connue, comprenant :

a) un ou plusieurs dispositifs de test pour déterminer le taux d'expression de THBS4 et d'au moins 11 gènes sélectionnés dans le Tableau 1 dans un échantillon comprenant des acides nucléiques de la tumeur primitive du sujet
b) un processeur ; et
c) un support de stockage comprenant une application informatique qui, lorsqu'elle est exécutée par le processeur, est configurée pour :

(i) accéder à et/ou calculer les taux d'expression déterminés des gènes sélectionnés dans le Tableau 1 dans l'échantillon sur le un ou plusieurs dispositifs de test
(ii) calculer s'il existe un taux augmenté ou réduit des gènes sélectionnés dans le Tableau 1 dans l'échantillon ; et
(iii) générer, à partir du processeur, la caractérisation et/ou le pronostic du cancer, où la caractérisation et/ou le pronostic du cancer consiste en la prédiction d'une probabilité accrue de récidive et/ou la prédiction d'une probabilité accrue de métastase et/ou de mauvais pronostic.

18. Système, dispositif ou kit de test selon la revendication 17, qui est adapté pour réaliser le procédé selon l'une quelconque des revendications 1 à 16.

19. Application informatique ou support de stockage comprenant une application informatique tel que défini dans la revendication 17 ou la revendication 18.

20. Produit de programme informatique pour caractériser et/ou pronostiquer un cancer chez un sujet atteint d'un cancer primitif mais sans aucune métastase connue, comprenant un dispositif de stockage non transitoire lisible par ordinateur comportant des instructions de programme lisibles par ordinateur qui permettent à l'ordinateur de :

(i) accéder à et/ou calculer les taux d'expression déterminés de THBS4 et d'au moins 11 gènes sélectionnés dans le Tableau 1 dans un échantillon comprenant des acides nucléiques de la tumeur primitive sur un ou plusieurs dispositifs de test ;
(ii) calculer s'il existe un taux augmenté ou réduit des gènes sélectionnés dans le Tableau 1 dans l'échantillon ; et,
(iii) générer un résultat concernant la caractérisation et/ou le pronostic du cancer, où la caractérisation et/ou le pronostic du cancer consiste en la prédiction d'une probabilité accrue de récidive et/ou la prédiction d'une probabilité accrue de métastase et/ou de mauvais pronostic.

FIG. 1

| | Cluster 1 | Cluster 2 | Cluster 4 | Cluster 3 |
|---|---|---|---|---|
| Benign | 0 | 1 | 23 | 1 |
| Primary | 6 | 18 | 8 | 38 |
| Mets | 6 | 4 | 0 | 0 |
| Primary with Mets | 10 | 10 | 0 | 1 |

FIG. 2

*FIG. 3*

112

*FIG. 4*

FIG. 5

FIG. 6

| | Chi-Square | d.f. | P |
|---|---|---|---|
| Almac 70 gene | 7.3 | 2 | 0.0256 |
| Log2 Pre PSA | 6.9 | 2 | 0.0313 |
| PathStage2 | 1.8 | 2 | 0.3998 |
| Gleason | 24.5 | 2 | 0.0000 |
| almac.70.gene.call x log2 Pre PSA | 4.9 | 1 | 0.0271 |

*FIG. 7*

Sig Scores

FIG. 8A

FIG. 8B

*FIG. 9*

FIG. 10

FIG. 11

FIG. 12

*FIG. 13*

*FIG. 14*

FIG. 15

FIG. 16

EP 3 310 927 B1

FIG. 17

FIG. 18

FIG. 19

FIG. 20

*FIG. 21*

FIG. 22

FIG. 23

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20060134663 A
- US 20090082218 A
- US 6410276 B
- US 4437975 A
- WO 9006995 A
- WO 2004067726 A
- WO 9746705 A

### Non-patent literature cited in the description

- **GORLOV et al.** *BMC Med Genomics,* 04 August 2009, vol. 2, 48
- **HASTIE et al.** The Elements of Statistical Learning. Springer, 2001
- **BREIMAN.** Random Forests. *Machine Learning,* 2001, vol. 45 (5
- **BISHOP.** Neural Networks for Pattern Recognition. Clarendon Press, 1995
- **DUDA et al.** Pattern Classification. John Wiley, 2001
- **TIBSHIRANI et al.** *Proc. Natl. Acad. Sci. USA,* 2002, vol. 99, 6567-6572
- **WOLD.** *Pattern Recogn.,* 1976, vol. 8, 127-139
- **BREIMAN, LEO ; FRIEDMAN, J. H. ; OLSHEN, R. A. ; STONE, C. J.** Classification and regression trees. Wadsworth & Brooks/Cole Advanced Books & Software, 1984
- **BREIMAN ; LEO.** Random Forests. *Machine Learning,* 2001, vol. 45 (1), 5-32
- **NGUYEN et al.** *Bioinformatics,* 2002, vol. 18, 39-50
- **SCHÖLKOPF et al.** Learning with Kernels. MIT Press, 2002
- **AHDESMÄKI et al.** *Annals of applied statistics,* 2010, vol. 4, 503-519
- **L. STAHLE ; S. WOLD.** *J. Chemom.,* 1987, vol. 1, 185-196
- **D. V. NGUYEN ; D.M. ROCKE.** *Bioinformatics,* 2002, vol. 18, 39-50
- Gene Expression Profiling: Methods and Protocols. Humana Press, 2004
- *Nucleic acids research,* 1998, vol. 26 (10), 2255-2264
- *Nature Reviews,* 2003, vol. 3, 253-266
- *Oral Oncology,* 2006, vol. 42, 5-13
- **SAMBROOK, J. et al.** Molecular cloning: A laboratory Manual. Cold Spring Harbor, 2001
- Oligonucleotide Synthesis, A Practical Approach. IRL Press, 1984
- Nucleic Acid Hybridization, A Practical Approach. IRL Press, 1985
- Methods in Enzymology. Academic Press, Inc
- **JONES et al.** *Nature,* 29 May 1986, vol. 321 (6069), 522-5
- **ROGUSKA et al.** *Protein Engineering,* 1996, vol. 9 (10), 895-904
- **STUDNICKA et al.** Humanizing Mouse Antibody Frameworks While Preserving 3-D Structure. *Protein Engineering,* 1994, vol. 7, 805
- ImmunoAssay: A Practical Guide. Taylor & Francis, Ltd, 2005
- **IRIZARRY RA ; BOLSTAD BM ; COLLIN F ; COPE LM ; HOBBS B ; SPEED TP.** Summaries of Affymetrix GeneChip probe level data. *Nucleic acids research,* 2003, vol. 31, e15
- **TIBSHIRANI R ; WALTHER G ; HASTIE T.** Estimating the number of clusters in a data set via the gap statistic. *J Roy Stat Soc B,* 2001, vol. 63, 411-23
- **WARD JH.** Hierarchical Grouping to Optimize an Objective Function. *Journal of the American Statistical Association,* 1963, vol. 58, 236
- **ASHBURNER M ; BALL CA ; BLAKE JA et al.** Gene ontology: tool for the unification of biology. *The Gene Ontology Consortium. Nature genetics,* 2000, vol. 25, 25-9
- **CHO RJ ; HUANG MX ; CAMPBELL MJ et al.** Transcriptional regulation and function during the human cell cycle. *Nature genetics,* 2001, vol. 27, 48-54
- **BENJAMINI Y ; HOCHBERG Y.** Controlling the False Discovery Rate - a Practical and Powerful Approach to Multiple Testing. *J Roy Stat Soc B Met,* 1995, vol. 57, 289-300